(19)

Europäisches
Patentamt

European
Patent Office

Office européen
des brevets

(11) **EP 4 608 482 B1**

(12) **EUROPEAN PATENT SPECIFICATION**

(45) Date of publication and mention
of the grant of the patent:
**08.07.2026 Bulletin 2026/28**

(21) Application number: **24809036.7**

(22) Date of filing: **11.11.2024**

(51) International Patent Classification (IPC):
*A61M 11/00* (2006.01)   *A24F 40/05* (2020.01)
*A24F 40/30* (2020.01)   *A61M 15/00* (2006.01)

(52) Cooperative Patent Classification (CPC):
(C-Sets available)
**A61M 11/005; A24F 40/05; A24F 40/10;
A24F 40/30; A61M 15/0003; A61M 15/0085;
A61M 15/06; B05B 17/06; B05B 17/0646;
B05B 17/0684;** A61M 11/001; A61M 2016/0021;
A61M 2016/0027; A61M 2202/0468; A61M 2202/30;
(Cont.)

(86) International application number:
**PCT/GB2024/052867**

(87) International publication number:
**WO 2025/099459 (15.05.2025 Gazette 2025/20)**

(54) **MULTI-CHAMBER MIST GENERATOR FOR GENERATING MIST INCLUDING A THERAPEUTIC**

MEHRKAMMER-NEBELERZEUGER ZUR ERZEUGUNG VON NEBEL MIT EINEM
THERAPEUTIKUM

GÉNÉRATEUR DE BROUILLARD À CHAMBRES MULTIPLES POUR GÉNÉRER UN BROUILLARD
COMPRENANT UN AGENT THÉRAPEUTIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE MA MD TN**

(30) Priority: **09.11.2023 US 202363547976 P**

(43) Date of publication of application:
**03.09.2025 Bulletin 2025/36**

(60) Divisional application:
**26183014.5**

(73) Proprietor: **Shaheen Innovations Holding Limited
Abu Dhabi (AE)**

(72) Inventors:
• **ALSHAIBA SALEH GHANNAM ALMAZROUEI,,
Mohammed**
**Abu Dhabi Global Market Square Al Maryah
Island (AE)**
• **MOHAMED HUSSEIN ARBAB, Ahmed**
**Abu Dhabi Global Market Square Al Maryah
Island (AE)**
• **JAJURIE, Abdullah Fahad Jikiron**
**Abu Dhabi Global Market Square Al Maryah
Island (AE)**
• **BHATTI, Sajid**
**Abu Dhabi Global Market Square Al Maryah
Island (AE)**

(74) Representative: **Forresters IP LLP
Skygarden
Erika-Mann-Straße 11
80636 München (DE)**

(56) References cited:
**WO-A1-2015/015431   WO-A1-2020/176777
WO-A1-2020/227717   WO-A1-2021/074862**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 608 482 B1

WO-A1-2021/205158     US-A1- 2022 361 565

(52) Cooperative Patent Classification (CPC): (Cont.)
A61M 2205/3334; A61M 2205/3368;
A61M 2205/3592; A61M 2205/6018

C-Sets
A61M 2202/0468, A61M 2202/0007;
A61M 2202/30, A61M 2202/0007

**Description**

FIELD

The present invention relates to a mist generator. The present invention more particularly relates to a mist generator which atomises a liquid comprising a therapeutic by ultrasonic vibrations.

BACKGROUND

Mist inhaler devices are used for generating a mist or vapour for inhalation by a user. The mist may contain a therapeutic, drug or medicine which is inhaled by a user and absorbed into the user's blood stream.

Therapeutic aerosol delivery is the mainstay for the treatment of asthma, chronic obstructive pulmonary disease (COPD) and cystic fibrosis. Therapeutic aerosol also has applications for the treatment of influenza, osteoporosis as well as the delivery of vaccines.

Pulmonary delivery of therapeutics for the treatment of non-respiratory systemic disease is appealing because of high lung vascularity, a thin blood-alveolar barrier, large surface area, avoidance of gastric enzymes and first-pass hepatic metabolism. It is also appealing because of improved patient comfort and adherence. The pulmonary system can be leveraged to deliver antibodies, proteins, pain killers and nucleic acids. The treatment of central nervous system disorders, such as tobacco dependence, could be significantly enhanced through the efficient delivery of nicotine to the systematic circulation through the lungs.

The effectiveness of therapeutic aerosol relates to the amount of drug deposited beyond the oropharyngeal region. The region where the deposit occurs is a function of the inhaled particles size.

The devices currently used for the administration of inhaled drugs are divided into three categories: nebulizers, metered-dose inhalers, and dry powder inhalers. Nebulizers are typically divided into two types: jet and ultrasonic but in conventional devices both types have weaknesses and present issues.

Jet nebulizers are based on the Bernoulli principle and produce relatively large droplets that generally deposit in the oropharyngeal region and are therefore not particularly effective. Ultrasonic nebulizers use piezoelectric crystals that vibrate at frequencies, ranging between 1 MHz and 1.7 MHz, transmitting the vibratory energy to the liquid converting it to aerosol. It is acknowledged that ultrasonic nebulizers are not effective if viscous suspensions or solutions are used and tend to heat the medication, hence destroy the molecules and remove the benefits of inhalation.

Electronic vaporising inhalers and other vapour inhalers typically have similar designs. Most electronic vaporising inhalers feature a liquid reservoir with an interior membrane, such as a capillary element, typically cotton, that holds the liquid so as to prevent leaking from the reservoir. Nevertheless, these devices are still prone to leaking because there is no obstacle to prevent the liquid from flowing out of the membrane and into the mouthpiece. A leaking electronic vaporising inhaler is problematic for several reasons. As a first disadvantage, the liquid can leak into the electronic components, which can cause serious damage to the device. As a second disadvantage, the liquid can leak into the electronic vaporising inhaler mouthpiece, and the user may inhale the unvapourised liquid.

Electronic vaporising inhalers are also known for providing inconsistent doses between draws. The aforementioned leaking is one cause of inconsistent doses because the membrane may be oversaturated or undersaturated near the vaporiser. If the membrane is oversaturated, then the user may experience a stronger than desired dose of vapour, and if the membrane is undersaturated, then the user may experience a weaker than desired dose of vapour. Additionally, small changes in the strength of the user's draw may provide stronger or weaker doses. Inconsistent dosing, along with leaking, can lead to faster consumption of the vaping liquid.

WO2020/227717 discloses an ultrasonic droplet delivery device with a fluid cartridge comprising two fluid reservoirs located side by side, and with a mouthpiece including two ejector mechanisms also located generally side by side.

Additionally, conventional electronic vaporising inhalers tend to rely on inducing high temperatures of a metal heating component configured to heat a liquid, thus vaporising the liquid that can be breathed in. Problems with conventional electronic vaporising inhalers may include the possibility of burning metal and subsequent breathing in of the metal along with the burnt liquid. In addition, some may not prefer the burnt smell caused by the heated liquid.

It is now recognised that electronic vaporising inhalers can play a key role in a smoking cessation program by allowing a user to receive nicotine doses in a manner which is considered safer than conventional cigarettes. Users are generally more likely to adhere to a smoking cessation program using vaporising inhalers as compared with nicotine patches or chews. However, conventional vaporising inhalers are not able to deliver nicotine doses consistently with every puff taken by a user and conventional vaporising inhalers are typically poor at mimicking the Mouth-To-Lung (MTL) effect.

A further problem with conventional electronic vaporising inhalers is that conventional devices do not typically deliver a consistent dose of a therapeutic to the lungs of a user. This is because some nicotine remains in liquid droplets that collect in the user's mouth instead of being inhaled down into the lungs of the user. The inconsistent dosing is problematic if the device is being used as part of a medical prescription where precise and consistent dosing of a therapeutic is important.

Thus, a need exists in the art for an improved mist generator which seek to address at least some of the problems described herein.

## BRIEF DESCRIPTION OF THE INVENTION

The present invention provides a mist generator as claimed in claim 1.

The present invention also provides preferred embodiments as claimed in the dependent claims.

The various examples of this disclosure which are described below have multiple benefits and advantages over conventional mist delivery devices. These benefits and advantages are set out in the description below.

Since the mist generator of examples of this disclosure enables higher efficiency operation than conventional mist generator devices, the mist generator devices of examples of this disclosure have an environmental benefit due to the reduced power requirement.

It is noted that the expression "mist" used in the following disclosure means the liquid is not heated as usually in traditional inhalers known from the prior art. In fact, traditional inhalers use heating elements to heat the liquid above its boiling temperature to produce a vapour, which is different from a mist.

In fact, when sonicating liquids at high intensities, the sound waves that propagate into the liquid media result in alternating high-pressure (compression) and low-pressure (rarefaction) cycles, at different rates depending on the frequency. During the low-pressure cycle, high-intensity ultrasonic waves create small vacuum bubbles or voids in the liquid. When the bubbles attain a volume at which they can no longer absorb energy, they collapse violently during a high-pressure cycle. This phenomenon is termed cavitation. During the implosion, very high pressures are reached locally. At cavitation, broken capillary waves are generated, and tiny droplets break the surface tension of the liquid and are quickly released into the air, taking mist form.

The following will explain more precisely the cavitation phenomenon.

When the liquid is atomised by ultrasonic vibrations, micro water bubbles are produced in the liquid.

The bubble production is a process of formation of cavities created by the negative pressure generated by intense ultrasonic waves generated by the means of ultrasonic vibrations.

High intensity ultrasonic sound waves leading to rapid growth of cavities with relatively low and negligible reduction in cavity size during the positive pressure cycle.

Ultrasound waves, like all sound waves, consist of cycles of compression and expansion. When in contact with a liquid, Compression cycles exert a positive pressure on the liquid, pushing the molecules together. Expansion cycles exert a negative pressure, pulling the molecules away from another.

Intense ultrasound waves create regions of positive pressure and negative pressure. A cavity can form and grow during the episodes of negative pressure. When the cavity attains a critical size, the cavity implodes.

The amount of negative pressure needed depends on the type and purity of the liquid. For truly pure liquids, tensile strengths are so great that available ultrasound generators cannot produce enough negative pressure to make cavities. In pure water, for instance, more than 1,000 atmospheres of negative pressure would be required, yet the most powerful ultrasound generators produce only about 50 atmospheres of negative pressure. The tensile strength of liquids is reduced by the gas trapped within the crevices of the liquid particles. The effect is analogous to the reduction in strength that occurs from cracks in solid materials. When a crevice filled with gas is exposed to a negative-pressure cycle from a sound wave, the reduced pressure makes the gas in the crevice expand until a small bubble is released into solution.

However, a bubble irradiated with ultrasound continually absorbs energy from alternating compression and expansion cycles of the sound wave. These cause the bubbles to grow and contract, striking a dynamic balance between the void inside the bubble and the liquid outside. In some cases, ultrasonic waves will sustain a bubble that simply oscillates in size. In other cases, the average size of the bubble will increase.

Cavity growth depends on the intensity of sound. High-intensity ultrasound can expand the cavity so rapidly during the negative-pressure cycle that the cavity never has a chance to shrink during the positive-pressure cycle. In this process, cavities can grow rapidly in the course of a single cycle of sound.

For low-intensity ultrasound the size of the cavity oscillates in phase with the expansion and compression cycles. The surface of a cavity produced by low-intensity ultrasound is slightly greater during expansion cycles than during compression cycles. Since the amount of gas that diffuses in or out of the cavity depends on the surface area, diffusion into the cavity during expansion cycles will be slightly greater than diffusion out during compression cycles. For each cycle of sound, then, the cavity expands a little more than it shrinks. Over many cycles the cavities will grow slowly.

It has been noticed that the growing cavity can eventually reach a critical size where it will most efficiently absorb energy from the ultrasound. The critical size depends on the frequency of the ultrasound wave. Once a cavity has experienced a very rapid growth caused by high intensity ultrasound, it can no longer absorb energy as efficiently from the sound waves. Without this energy input the cavity can no longer sustain itself. The liquid rushes in and the cavity implodes due to a non-linear response.

The energy released from the implosion causes the liquid to be fragmented into microscopic particles which are dispersed into the air as mist.

The equation for description of the above non-linear response phenomenon may be described by the "Rayleigh-Plesset" equation. This equation can be derived from the "Navier-Stokes" equation used in fluid dynamics.

The inventors approach was to rewrite the "Rayleigh-Plesset" equation in which the bubble volume, V, is used as the dynamic parameter and where the physics describing the dissipation is identical to that used in the more classical form where the radius is the dynamic parameter.

The equation used derived as follows:

$$\frac{\left|\frac{1}{c^2}\frac{\delta^2\phi}{\delta t^2}\right|}{\nabla^2\phi} \sim \left(\frac{R}{\lambda}\right)^2 \ll 1$$

$$\frac{1}{4\pi}\left(\frac{4\pi}{3V}\right)^{\frac{1}{3}}\left(\ddot{V} - \frac{\dot{V}^2(t)}{6V}\right)$$

$$= \frac{1}{\rho_0}\left(\left(p_0 + 2\sigma\left(\frac{4\pi}{3V_0}\right)^{\frac{1}{3}} - p_V\right)\left(\frac{V_0}{V}\right)^\kappa + p_V - 2\sigma\left(\frac{4\pi}{3V}\right)^{\frac{1}{3}} - p_0 - P(t)\right)$$

wherein:

  $V$ is the bubble volume
  $V_0$ is the equilibrium bubble volume
  $\rho_0$ is the liquid density (assumed to be constant)
  $\sigma$ is the surface tension
  $p_V$ is the vapour pressure
  $p_0$ is the static pressure in the liquid just outside the bubble wall
  $\kappa$ is the polytropic index of the gas
  t is the time
  $R(t)$ is the bubble radius
  P(t) is the applied pressure $c$ is the speed sound of the liquid
  $\phi$ is the velocity potential
  $\lambda$ is the wavelength of the insonifying field

In the ultrasonic mist inhaler, the liquid has a kinematic viscosity between 1.05 Pa.sec and 1.412 Pa.sec.

By solving the above equation with the right parameters of viscosity, density and having a desired target bubble volume of liquid spray into the air, it has been found that the frequency range of 2.8MHz to 3.2MHz for liquid viscosity range of 1.05 Pa.s and 1.412 Pa.s produce a bubble volume of about 0.25 to 0.5 microns.

The process of ultrasonic cavitation has a significant impact on therapeutic concentration in the produced mist.

No heating elements are involved, thereby leading to no burnt elements and reducing second-hand smoke effects.

In the ultrasonic mist inhaler, a capillary element may extend between the sonication chamber and the liquid chamber.

In the ultrasonic mist inhaler, the capillary element is a material at least partly in bamboo fibres.

The capillary element allows a high absorption capacity, a high rate of absorption as well as a high fluid-retention ratio.

It was found that the inherent properties of the proposed material used for the capillarity have a significant impact on the efficient functioning of the ultrasonic mist inhaler.

Further, inherent properties of the proposed material include a good hygroscopicity while maintaining a good permeability. This allows the drawn liquid to efficiently permeate the capillary while the observed high absorption capacity allows the retention of a considerable amount of liquid thus allowing the ultrasonic mist inhaler to last for a longer time when compared with the other products available in the market.

Another significant advantage of using the bamboo fibres is the naturally occurring antimicrobial bio-agent namely "Kun" inherently present within the bamboo fibre making it antibacterial, anti-fungal and odour resistant, making it suitable for medical applications.

The inherent properties have been verified using numerical analysis regarding the benefits of the bamboo fibre for sonication.

The following formulae have been tested with bamboo fibres material and others material such cotton, paper, or other fibre strands for the use as capillary element and demonstrates that bamboo fibres have much better properties for the use in sonication:

$$C = A + \frac{T}{W_f} - \frac{1}{P_f} + (1 - \alpha)\frac{V_d}{W_f}$$

wherein:

$C$ (cc/gm of fluid/gm) is the volume per mass of the liquid absorbed divided by the dry mass of the capillary element,
$A$ ($cm^2$) is the total surface area of the capillary element
$T$ (cm) is the thickness of the capillary element,
$W_f$ (gm) is the mass of the dry capillary element,
$P_f$ (cc/g. sec) is the density of the dry capillary element,
$\alpha$ is the ratio of increase in volume of capillary element upon wetting to the volume of liquid diffused in the capillary element,
$V_d$ (cc) is the amount of liquid diffused in the capillary element,

$$Absorbent\ Rate, Q = \frac{\pi r \gamma 1 \cos\theta}{2\eta} \cdot \left(\frac{T}{W_f} - \frac{1}{AP_f}\right)$$

$Q$ (cc/sec) is the amount of liquid absorbed per unit time,
$r$ (cm) is the radius of the pores within the capillary element,
$\gamma$ (N/m) is the surface tension of the liquid,
$\theta$ (degrees) is the angle of contact of the fibre,
$\eta$ ($m^2$/sec) is the viscosity of the fluid.

In the ultrasonic mist inhaler, the capillary element may be a material at least partly in bamboo fibres.

In the ultrasonic mist inhaler, the capillary element material may be 100% bamboo fibre.

Extensive testing has concluded that a 100% pure bamboo fibre is the most optimal choice for sonication.

In the ultrasonic mist inhaler, the capillary element material may be at least 75% bamboo fibre and, optionally, 25% cotton.

Capillary element from 100% pure bamboo fibre or with a high percentage of bamboo fibres demonstrates a high absorption capacity as well as improved fluid transmission making it an optimal choice for the application of the ultrasonic mist inhaler.

REPRESENTATIVE FEATURES

Representative features are set out in the following clauses, which stand alone or may be combined, in any combination, with one or more features disclosed in the text and/or drawings of the specification.

1. A mist generator for generating a mist for inhalation by a user, the mist generator comprising:

an air inlet port;
a mouthpiece including a mist outlet port;
an air flow path from the air inlet port to the mist outlet port;
a first liquid chamber containing a first liquid including a therapeutic;
a second liquid chamber containing a second liquid;
a first ultrasonic transducer including a first atomisation surface;
a second ultrasonic transducer including a second atomisation surface;
a transducer holder including a holder surface that is provided with a first recess and a second recess, wherein the first recess retains the first ultrasonic transducer with the first atomisation surface generally in a plane with the holder surface and the second recess retains the second ultrasonic transducer with the second atomisation surface generally in the plane with the holder surface;

a first capillary which is absorbent, at least part of the first capillary being generally parallel with the plane and extending along the holder surface from the first liquid chamber to the first ultrasonic transducer, one part of the first capillary being superimposed on the holder surface and another part of the first capillary being in contact with at least a portion of the first atomisation surface, wherein the first capillary transports the first liquid by capillary action from the first liquid chamber to the first atomisation surface; and

a second capillary which is absorbent, at least part of the second capillary being generally parallel with the plane and extending along the holder surface from the second liquid chamber to the second ultrasonic transducer, one part of the second capillary being superimposed on the holder surface and another part of the second capillary being in contact with at least a portion of the second atomisation surface, wherein the second capillary transports the second liquid by capillary action from the second liquid chamber to the second atomisation surface,

the first ultrasonic transducer being configured to generate and transmit ultrasonic waves to generate a first portion of the mist from the first liquid at the interface between the first capillary and the first ultrasonic transducer, and

the second ultrasonic transducer being configured to generate and transmit ultrasonic waves to generate a second portion of the mist from the second liquid at the interface between the second capillary and the second ultrasonic transducer, wherein the first portion of the mist and the second portion of the mist combine and flow out through the mist outlet port to the user when the user draws on the mouthpiece.

2. The mist generator of clause 1, wherein the transducer holder comprises:

a first electrical contact channel which receives at least part of a first electrical contact, the first electrical contact extending from a first end of the transducer holder to the first transducer; and

a second electrical contact channel which receives at least part of a second electrical contact, the second electrical contact extending from the first end of the transducer holder to the second transducer.

3. The mist generator of clause 1 or clause 2, wherein the first recess of the transducer holder is provided with a first through hole and the second recess of the transducer holder is provided with a second through hole, where a side of the first ultrasonic transducer is contactable through the first through hole and a side of the second ultrasonic transducer is contactable through the second through hole.

4. The mist generator of clause 3, wherein the mist generator comprises:

a third electrical contact, part of the third electrical contact extending through the first through hole to contact the side of the first ultrasonic transducer; and

a fourth electrical contact, part of the fourth electrical contact extending through the second through hole to contact the side of the second ultrasonic transducer.

5. The mist generator of any one of the preceding clauses, wherein the mist generator comprises:
a plurality of assembly portions which are coupled to one another to form an air inlet port and an air flow path from the air inlet port to the mist outlet port, one assembly portion of the plurality of assembly portions comprising a mist outlet channel and at least a part of a mist outlet conduit.

6. The mist generator of clause 5, wherein the plurality of assembly portions include a first assembly portion, the first assembly portion including a first surface, a second surface, and two air inlet channels which extend along the first surface and to the second surface.

7. The mist generator of clause 6, wherein the first assembly portion includes the mist outlet channel and the at least a part of the mist outlet conduit.

8. The mist generator of clause 6 or clause 7, wherein the plurality of assembly portions include a second assembly portion, the second assembly portion including:

an upper section;
a lower section spaced from the upper section; and
a middle section extending from the upper section to the lower section,
the upper section including two sonication chamber recesses, two sonication chamber air inlets, two sonication chamber mist outlets; and

the lower section including two channels, such that then the first assembly portion is coupled to the second assembly portion:

the two air inlet channels of the first assembly portion and the two channels of the second assembly portion form an air flow path from the inlet port to the sonication chamber air inlets; and

a mist flow path from the sonication chamber mist outlets through the mist outlet conduit.

9. The mist generator of clause 6 or clause 7, wherein the plurality of assembly portions includes a second assembly portion, the second assembly portion including:

an upper section;

a lower section spaced from the upper section; and

a middle section extending from the upper section to the lower section,

the upper section including two sonication chamber recesses, two sonication chamber air inlets, two sonication chamber mist outlets, and a groove; and

the lower section including two channels,

wherein the two air inlet channels of the first assembly portion and the two channels of the second assembly portion form an air flow path from the inlet port to the sonication chamber air inlets; and

the at least a part of the mist outlet conduit of the first assembly portion and the groove of the upper section of the second assembly portion form a mist flow path from the sonication chamber mist outlets through the mist outlet conduit.

10. The mist generator of clause 8 or clause 9, wherein the upper section includes at least one biasing member within each recess for biasing the respective capillary against the portion of the respective atomisation surface.

11. The mist generator of any one of clauses 8 to 10, wherein the middle section of the second assembly portion includes two extrusions.

12. The mist generator of any one of clauses 8 to 11, wherein the plurality of assembly portions includes a third assembly portion, the third assembly portion including two through holes, the first liquid chamber, and second liquid chamber.

13. The mist generator of any one of the preceding clauses, further including two generally annular electrical connections, each of the two generally annular electrical connections coupled to a respective one of the first ultrasonic transducer and the second ultrasonic transducer.

14. The mist generator of any one of clauses 8 to 12, further including a fourth assembly portion which is at least partly superimposed on a portion of the transducer holder.

15. The mist generator of clause 12, wherein the third assembly portion includes two depressions, each depression overlapping a respective one of the two through holes in the third assembly portion.

16. The mist generator of any one of the preceding clauses, wherein the second liquid includes at least one further therapeutic.

17. The mist generator of any one of the preceding clauses, wherein the mist generator comprises:

at least one further liquid chamber each containing a respective further liquid;

at least one further ultrasonic transducer each including a respective further atomisation surface;

at least one further capillary, at least part of the further capillary being generally parallel with the plane and extending along the holder surface from the respective further liquid chamber to the respective further ultrasonic transducer, one part of the respective further capillary being superimposed on the holder surface and another part of the respective further capillary being in contact with at least a portion of the respective further atomisation surface, wherein the respective further capillary transports the respective further liquid by capillary action from the respective further liquid chamber to the respective further atomisation surface,

each further ultrasonic transducer being configured to generate and transmit ultrasonic waves to generate a respective further portion of the mist from the respective further liquid at the interface between the respective further capillary and the respective further ultrasonic transducer, wherein the respective further portion of the mist combines with the first portion of the mist and the second portion to form the mist which flows out through the mist outlet port to the user when the user draws on the mouthpiece.

18. The mist generator of clause 17, wherein each further liquid includes at least one further therapeutic.

19. A mist generator for generating a mist for inhalation by a user, the mist generator comprising:

an air inlet port;
a mouthpiece including a mist outlet port;
an air flow path from the air inlet port to the mist outlet port;
a liquid chamber containing a liquid including a therapeutic;
a first ultrasonic transducer including a first atomisation surface;
a second ultrasonic transducer including a second atomisation surface;
a transducer holder including a holder surface that is provided with a first recess and a second recess, wherein the first recess retains the first ultrasonic transducer with the first atomisation surface generally in a plane with the holder surface and the second recess retains the second ultrasonic transducer with the second atomisation surface generally in the plane with the holder surface; and
a capillary which is absorbent, at least part of the capillary being generally parallel with the plane and extending along the holder surface from the liquid chamber to the first ultrasonic transducer and to the second ultrasonic transducer, a first portion of the capillary being superimposed on the holder surface, a second portion of the capillary being in contact with at least a portion of the first atomisation surface and a third portion of the capillary being in contact with at least a portion of the second atomisation surface, wherein the capillary transports the liquid by capillary action from the liquid chamber to the first atomisation surface and the second atomisation surface, the first ultrasonic transducer being configured to generate and transmit ultrasonic waves to generate a first portion of the mist from the liquid at the interface between the second portion of the capillary and the first ultrasonic transducer, and
the second ultrasonic transducer being configured to generate and transmit ultrasonic waves to generate a second portion of the mist from the liquid at the interface between the third portion of the capillary and the second ultrasonic transducer, wherein the first portion of the mist and the second portion of the mist combine and flow out through the mist outlet port to the user when the user draws on the mouthpiece.

20. A mist delivery device for delivering a polydisperse mist including different liquid particle sizes for inhalation by a user, the mist delivery device comprising:

a mist generator including:

an air inlet port;
a mouthpiece including a mist outlet port;
a gas flow path from the air inlet port to the mist outlet port;
a first liquid chamber containing a first liquid including a therapeutic;
a second liquid chamber containing a second liquid;
a first ultrasonic transducer including a first atomisation surface;
a first capillary in contact with at least a portion of the first atomisation surface, the first capillary being absorbent and extending from the first liquid chamber to the first ultrasonic transducer;
a second ultrasonic transducer including a second atomisation surface; and
a second capillary in contact with at least a portion of the second atomisation surface, the second capillary being absorbent and extending from the

second liquid chamber to the second ultrasonic transducer,

wherein the device further comprises:

a driver configured to generate a first AC drive signal and a second AC drive signal concurrently, the driver being coupled electrically to the first ultrasonic transducer and the second ultrasonic transducer, wherein:

the first AC drive signal drives the first ultrasonic transducer to generate and transmit ultrasonic waves, the driver selecting a frequency of the first AC drive signal in a first frequency range of 2.9 MHz to 3.1 MHz to generate a first portion of the mist from the first liquid at the interface between the first capillary and the first ultrasonic transducer, the first portion of the mist having a first liquid particle size of less than 4 microns; and the second AC drive signal drives the second ultrasonic transducer to generate and transmit ultrasonic waves, the driver selecting a frequency of the second AC drive signal in a second frequency range of 0.5 MHz to 1.5 MHz to generate a second portion of the mist from the second liquid at the interface between the second capillary and the second ultrasonic transducer, the second portion of the mist having a second liquid particle size of 4 microns or greater,

wherein the first portion of the mist and the second portion of the mist combine to form the mist which flows through the mist outlet port to the user when the user draws on the mouthpiece.

21. The device of clause 20, wherein the second liquid includes at least one further therapeutic.

22. A mist delivery device for generating a mist for inhalation by a user, the device comprising:

a mist generator including:

an air inlet port;
a mouthpiece including a mist outlet port;
a gas flow path from the air inlet port to the mist outlet port;
a first liquid chamber containing a first liquid including a therapeutic;
a second liquid chamber containing a second liquid;
a first ultrasonic transducer including a first atomisation surface;
a first capillary in contact with at least a portion of the first atomisation surface, the first capillary being absorbent and extending from the first liquid chamber to the first ultrasonic transducer;
a second ultrasonic transducer including a second atomisation surface; and
a second capillary in contact with at least a portion of the second atomisation surface, the second capillary being absorbent and extending from the

second liquid chamber to the second ultrasonic transducer,

wherein the device further comprises:

a driver configured to generate a first AC drive signal and a second AC drive signal concurrently, the driver being coupled electrically to the first ultrasonic transducer and the second ultrasonic transducer, wherein:

the first AC drive signal drives the first ultrasonic transducer to generate and transmit ultrasonic waves, the driver selecting a frequency of the first AC drive signal in a first frequency range to generate a first portion of the mist from the first liquid at the interface between the first capillary and the first ultrasonic transducer, the first portion of the mist having a first liquid particle size; and
the second AC drive signal drives the second ultrasonic transducer to generate and transmit ultrasonic waves, the driver selecting a frequency of the second AC drive signal in a second frequency range to generate a second portion of the mist from the second liquid at the interface between the second capillary and the second ultrasonic transducer, the second portion of the mist having a second liquid particle size, and

wherein the first portion of the mist and the second portion of the mist combine to form the mist which flows through the mist outlet port to the user when the user draws on the mouthpiece.

23. The device of clause 22, wherein the first frequency range is 2.9 MHz to 3.1 MHz.

24. The device of clause 22 or clause 23, wherein the second frequency range is 0.5 MHz to 1.5 MHz.

25. The device of any one of clauses 22 to 24, wherein the first liquid particle size is less than 4 microns.

26. The device of any one of clauses 22 to 25, wherein the second liquid particle size is 4 microns or greater.

27. The device of any one of clauses 20 to 26, wherein:

the mist generator includes:

at least one further liquid chamber each containing a respective further liquid;
at least one further ultrasonic transducer each including a respective further atomisation surface;
at least one further capillary each in contact with a portion of a respective further atomisation surface, each further capillary being absorbent and each further capillary extending from a respective further liquid chamber to a respective further ultrasonic transducer;

wherein the driver is configured to generate at least one further AC drive signal concurrently with the first AC drive signal and the second AC drive signal, the driver being coupled electrically to each further ultrasonic transducer, wherein:

each further AC drive signal drives the respective further ultrasonic transducer to generate and transmit ultrasonic waves, the driver selecting a frequency of each further AC drive signal in at least one further frequency range to generate at least one further portion of the mist from each further liquid at the interface between each further capillary and a respective further ultrasonic transducer, each further portion of the mist having a respective further liquid particle size; and

wherein each further portion of the mist combines with the first portion of the mist and the second portion of the mist to form the mist.

28. The device of clause 27, wherein each further liquid includes at least one further therapeutic.

29. The device of any one of the clauses 20 to 28, wherein the device comprises:
a plurality of H-bridge circuits which are each connected to a respective one of the ultrasonic transducers, each H-bridge circuit being configured to generate the respective AC drive signal to drive the respective ultrasonic transducer.

30. The device of clause 29, wherein the plurality of H-bridge circuits are each subsystems of a bridge integrated circuit (IC).

31. The device of clause 29 or clause 30, wherein the device comprises:
a plurality of power management subsystems each configured to output a first phase output signal and a second phase output signal to a respective one of the H-bridge circuits, each power management subsystem including:
a first feedback input terminal which is configured to receive a feedback signal from the respective H-bridge circuit, the feedback signal being indicative of a parameter of the operation of the respective H-bridge circuit when the respective H-bridge circuit is driving the respective ultrasonic transducer with the respective AC drive signal, wherein the power management subsystem is configured to generate a respective voltage for modulation by the respective H-bridge circuit to drive the respective ultrasonic transducer in response to the feedback signal which is indicative of the operation of the respective ultrasonic transducer to optimise the generation of the respective portion of the mist.

32. The device of clause 31, wherein the plurality of power management subsystems are each subsystems of a power management integrated circuit (PMIC).

33. The device of clause 31 or clause 32, wherein the device comprises:
a first mist density sensor which is configured to sense a density of the first portion of the mist and generate a first mist density signal which is indicative of the density of the first portion of the mist, wherein the first mist density sensor provides the first mist density signal to a second feedback input terminal of a respective power management subsystem of the plurality of power management subsystems, and wherein the respective power management subsystem generates the respective voltage for modulation by the respective H-bridge circuit in response to the first mist density signal to optimise the generation of the first portion of the mist.

34. The device of clause 33, wherein the device comprises:
at least one further mist density sensor which is configured to sense a density of at least one further portion of the mist and generate at least one further mist density signal which is indicative of the density of the further portion of the mist, wherein each further mist density sensor provides the further mist density signal to a further feedback input terminal of a respective power management subsystem of the plurality of power management subsystems, and wherein the respective power management subsystem generates the respective voltage for modulation by the respective H-bridge circuit in response to the further mist density signal to optimise the generation of the further portion of the mist.

35. The device of any one of clauses 31 to 34, wherein each power management subsystem includes:

an oscillator which is configured to generate:

a main clock signal,
a first phase clock signal which is high for a first time during the positive half-period of the main clock signal and low during the negative half-period of the main clock signal, and
a second phase clock signal which is high for a second time during the negative half-period of the main clock signal and low during the positive half-period of the main clock signal, wherein the phases of the first phase clock signal and the second phase clock signal are centre aligned;

a pulse width modulation (PWM) signal generator subsystem comprising:

a delay locked loop which is configured to generate a double frequency clock signal using the first phase clock signal and the second phase clock signal, the double frequency clock signal being double the frequency of the main clock signal, wherein the delay locked loop is configured to control the rising edge of the first phase clock signal and the second phase clock signal to be synchronous with the rising edge of the double frequency clock signal, and wherein the delay locked loop is configured to adjust the frequency and the duty cycle of the first phase clock signal and the second phase clock signal in response to a driver control signal to produce the first phase output signal and the second phase output signal, wherein the first phase output signal and the second phase output signal are configured to drive the H-bridge circuit to generate an AC drive signal;
a first phase output signal terminal which is configured to output the first phase output signal; and
a second phase output signal terminal which is configured to output the second phase output signal.

36. The device of any one of clauses 31 to 35, wherein the device comprises:
a main controller coupled electrically to each power management subsystem, the main controller being configured to coordinate the operation of the power management subsystems in response to a configuration signal to control the generation of the polydisperse mist.

37. The device of clause 36, wherein the device comprises:
an input device for receiving an input from a user, wherein the driver is configured to generate the configuration signal in response to the input from the user.

38. The device of clause 37, wherein the input device includes a wireless communication device which is configured to communicate wirelessly with a computing device to enable a user to provide the input using the computing device.

39. A multi-chamber mist generator device for delivering a therapeutic to a user, the mist generator device for use with a driver device, wherein the mist generator device comprises:

a housing comprising a plurality of liquid chambers for containing liquids to be atomized, at least one of the liquids comprising a therapeutic;
a sonication assembly coupled to the housing, the sonication assembly comprising:

a plurality of ultrasonic transducers, each ultrasonic transducer comprising an atomization surface, a first electrical transducer contact and a second electrical transducer contact, the first and second electrical transducer contacts for receiving an AC drive signal from a driver device;
a first assembly portion comprising a plurality of recesses, each recess retains a respective one of the ultrasonic transducers;
a second assembly portion coupled to the first assembly portion, the second assembly portion comprising:

a plurality of sonication recesses, each sonication recess being superimposed on a respective one of the ultrasonic transducer to form a sonication chamber;
a sonication assembly air inlet port in fluid communication with the sonication recesses;
a sonication assembly mist outlet port in fluid communication with the sonication recesses; and
an airflow path extending from the sonication assembly air inlet port through the sonication recesses to the sonication assembly mist outlet port, wherein:
at least one of the first assembly portion and the second assembly portion comprises a resiliently deformable section that forms a seal between the first assembly portion and the second assembly portion which minimizes or prevents a fluid from leaking between the first assembly portion and the second assembly portion, wherein the mist generator device further comprises:

a barrier portion comprising a resiliently deformable seal that is positioned between the sonication assembly and the housing which minimizes or prevents fluid leakage, the barrier portion comprising a capillary aperture having a wall that is resiliently deformable; and

a plurality of capillaries, each capillary extending through the capillary aperture from the respective liquid chamber to the respective sonication chamber such that a first portion of the capillary is within the liquid chamber and a second portion of the capillary is within the sonication chamber, wherein the second portion of the capillary is superimposed on the atomization surface of a respective one of the ultrasonic transducers such that when the ultrasonic transducer is driven by an AC drive signal from the driver device, the atomization surface vibrates and atomizes the liquid carried by the second portion of the capillary to generate a mist comprising the atomized liquid and air within the sonication chamber, the mist flowing out through the sonication assembly mist outlet port for inhalation by a user.

40. The device of clause 39, wherein the first assembly portion is of a resiliently deformable material and part of the first assembly portion forms the seal between the first assembly portion and the second assembly portion.

41. The device of clause 40, wherein the resiliently deformable material is silicone.

42. The device of any one of clauses 39 to 41, wherein the second assembly portion is of a resiliently deformable material and part of the second assembly portion forms the seal between the first assembly portion and the second assembly portion.

43. The device of clause 42, wherein the resiliently deformable material is silicone.

44. The device of clause 42 or clause 43, wherein the second assembly portion comprises:

body portion which is provided with the sonication recess; and
a cover portion which is coupled to the body portion, the cover portion being provided with a channel defining at least part of the airflow path, wherein one end of the channel forms the air inlet port and the other end of the channel forms the mist outlet port.

45. The device of any one of clauses 39 to 44, wherein the second assembly portion comprises at least one biasing member that is provided within the sonication recess, each biasing member exerting a biasing force on the second portion of the capillary which biases the second portion of the capillary against the atomization surface of the ultrasonic transducer.

46. The device of clause 45, wherein the second assembly portion comprises a plurality of biasing members.

47. The device of clause 45 or clause 46, wherein each biasing member is formed integrally with the second assembly portion.

48. The device of any one of clauses 45 to 47, wherein each biasing member comprises an attachment end that is attached to the second assembly portion and a distal end which contacts the second portion of the capillary, the distal end being narrower than the attachment end.

49. The device of any one of clauses 45 to 48, wherein each biasing member is substantially conical in shape.

50. The device of any one of clauses 39 to 49, wherein the housing comprises an internal cavity that is defined by a base and side walls that project upwardly from the base, wherein the liquid chamber is formed in part of the internal cavity adjacent to the base and the sonication assembly is at least partly received in the internal cavity such that the liquid chamber is positioned between the sonication assembly and the base.

51. The device of clause 50, wherein sonication assembly contacts the side walls of the housing and forms a seal which minimises or prevents a fluid flowing between the sonication assembly and the side walls of the housing.

52. The device of clause 50 or clause 51, wherein the sonication assembly comprises a filling bore which extends from a filling aperture through part of the sonication assembly to the liquid chamber to allow a fluid to be injected into the liquid chamber through the filling bore.

53. The device of any one of clauses 50 to 52, wherein the device comprises a mouthpiece that is attached to the housing to substantially close the internal cavity, the mouthpiece comprising a mist outlet aperture that is in fluid communication with the sonication assembly mist outlet port to provide a mist flow path from the sonication assembly to the mist outlet aperture.

54. The device of clause 53, wherein the device comprises a foam layer which is positioned in the mist flow path to absorb droplets of liquid that are in excess of a predetermined size from the mist flowing along the mist flow path.

55. The device of clause 54, wherein the foam layer is positioned between the sonication assembly and a divider, the divider comprising a mist outlet aperture to allow mist to flow from the foam layer past the divider.

56. The device of clause 55 as dependent on clauses 52 to 54, wherein the divider comprises a plug which seals the filling aperture to minimise or prevent liquid leaking from the liquid chamber to the exterior of the device.

57. The device of any one of clauses 39 to 56, wherein the atomization surface of the ultrasonic transducer is substantially planar and the plane of the atomization surface is substantially parallel with a longitudinal length of the housing.

58. The device of any one of clauses 39 to 57, wherein the ultrasonic transducer is received within an ultrasonic transducer stack, the ultrasonic transducer stack comprising:

a generally cylindrical silicone base that is provided with a central aperture through which the first electrical transducer contact extends, wherein the silicone base comprises a generally cylindrical recess that receives the ultrasonic transducer such that a contact surface of the ultrasonic transducer is electrically connected to the first electrical transducer contact; and
a generally cylindrical metal shell that at least partly surrounds the silicone base, the metal shell comprising a lip which contacts at least part of the periphery of the ultrasonic transducer to retain the ultrasonic transducer in the cylindrical recess, the metal shell forming the second electrical transducer contact.

59. The device of clause 58, wherein part of the metal shell is cut away to provide access for a first elongate device terminal to connect electrically to the first electrical transducer contact, and wherein a second elongate device terminal is connected electrically to the metal shell, wherein the first and second elongate device terminals receive an AC drive signal from a driver device and transmit the AC drive signal to the ultrasonic transducer.

60. The device of any one of clauses 39 to 59, wherein the housing comprises a metal end cap that magnetically attracts to a magnet on a driver device to retain the mist generator device in engagement with the driver device.

61. The device of any one of clauses 39 to 60, wherein the airflow path comprises a plurality of turns that change the direction of the flow of air a plurality of times as the air flows from the air inlet port to the sonication recess.

62. The device of any one of clauses 39 to 61, wherein the capillary is of at least 75% bamboo fibre.

63. The device of any one of clauses 39 to 62, wherein the capillary is 100% bamboo fibre.

64. The device of any one of clauses 39 to 63, wherein the device comprises:
an identification arrangement which is carried by the housing to identify the mist generator device, the identification arrangement comprising:
a one-time programmable integrated circuit (OTP IC) having a memory which stores a unique identifier for the mist generator device, the OTP IC comprising a digital core which includes a cryptographic authenticator, and an electrical connection which provides an electronic interface for communication with the OTP IC and a driver device.

65. The device of clause 64, wherein the one-time-programmable (OTP) device includes an anti-counterfeiting feature to identify genuine mist generator devices and allow only the mist generating device identified as genuine to be authorized for use with the driver device.

66. The device of clause 64 or clause 65, wherein the OTP device controls the mist generator device to function to generate mist only when authorised.

67. The device of any one of clauses 64 to 66, wherein the OTP IC contains a unique information data that allows traceability of the mist generator device and a monitoring of the consumption by a user of the misting device.

68. The device of any one of clauses 64 to 67, wherein the memory of the OTP IC stores a record of the state of the mist generator device which is indicative of at least one of the historic use of the mist generator device and the volume of the liquid within the liquid chamber.

69. The device of any one of clauses 64 to 68, wherein the memory of the OTP IC stores a record of the seconds of aerosolization such that the mist generator is considered empty of liquid in the liquid chamber after a predetermined duration of use of approximately 1,000 seconds of aerosolization, and will not be able to be activated after the predetermined duration of use.

70. The device of any one of clauses 64 to 69, wherein whenever the mist generator device is considered empty, the mist generator device will not be activated if coupled to a driver device.

71. The device of any one of clauses 64 to 70, wherein the cryptographic authenticator uses an Elliptic Curve Digital Signature Algorithm (ECDSA) for encrypting/decrypting data stored within the OTP IC and data transmitted to and from the OTP IC.

72. The device of any one of clauses 39 to 71, wherein the liquid chamber contains the liquid having a liquid viscosity between 1.05 Pa•s and 1.412 Pa•s and a liquid density between 1.1 g/ml and 1.3 g/ml.

73. The device of clause 22, wherein the first frequency range is 2.9 MHz to 3.1 MHz and the second frequency range is 2.9 MHz to 3.1 MHz.

74. The device of clause 73, wherein the first liquid particle size is less than 4 microns and the second liquid particle size is less than 4 microns.

BRIEF DESCRIPTION OF THE FIGURES

In order that the present disclosure may be more readily understood, preferable embodiments thereof will now be described, by way of example only, with reference to the accompanying drawings, in which:

Figure 1 is a diagrammatic perspective view of a mist generator of an example of this disclosure.
Figure 2 is a diagrammatic perspective view of a mist generator of an example of this disclosure.
Figure 3 is a diagrammatic perspective view of a mist generator of an example of this disclosure.
Figure 4 is a diagrammatic perspective view of a mist generator of an example of this disclosure.
Figure 5 is a diagrammatic exploded perspective view of a mist generator of an example of this disclosure.
Figure 6 is a diagrammatic perspective view of a transducer holder of an example of this disclosure.
Figure 7 is a diagrammatic perspective view of a transducer holder of an example of this disclosure.
Figure 8 is a diagrammatic perspective view of a capillary element of an example of this disclosure.
Figure 9 is a diagrammatic perspective view of a capillary element of an example of this disclosure.
Figure 10 is a diagrammatic perspective view of a transducer holder of an example of this disclosure.
Figure 11 is a diagrammatic perspective view of a transducer holder of an example of this disclosure.
Figure 12 is a diagrammatic perspective view of a part of a housing of an example of this disclosure.
Figure 13 is a diagrammatic perspective view of an absorbent element of an example of this disclosure.
Figure 14 is a diagrammatic perspective view of a part of a housing of an example of this disclosure.
Figure 15 is a diagrammatic perspective view of a part of a housing of an example of this disclosure.
Figure 16 is a diagrammatic perspective view of an absorbent element of an example of this disclosure.
Figure 17 is a diagrammatic perspective view of a part of a housing of an example of this disclosure.
Figure 18 is a diagrammatic perspective view of a part of a housing of an example of this disclosure.
Figure 19 is a diagrammatic perspective view of a part of a housing of an example of this disclosure.
Figure 20 is a diagrammatic perspective view of a circuit board of an example of this disclosure.
Figure 21 is a diagrammatic perspective view of a circuit board of an example of this disclosure.
Figure 22 is a diagrammatic exploded perspective view of a mist generator of an example of this disclosure.

Figure 23 is a diagrammatic exploded perspective view of a mist generator of an example of this disclosure.

Figure 24 is a cross sectional view of a mist generator of an example of this disclosure.

Figure 25 is a cross sectional view of a mist generator of an example of this disclosure.

Figure 26 is a cross sectional view of a mist generator of an example of this disclosure.

Figure 27 is a diagrammatic perspective view of a mist generator of an example of this disclosure.

Figure 28 is a diagrammatic perspective view of a mist generator of an example of this disclosure.

Figure 29 is a diagrammatic exploded perspective view of a mist generator of an example of this disclosure.

Figure 30 is a diagrammatic perspective view of a transducer holder assembly of an example of this disclosure.

Figure 31 is a diagrammatic perspective view of a transducer holder assembly of an example of this disclosure.

Figure 32 is a diagrammatic exploded perspective view of a transducer holder assembly of an example of this disclosure.

Figure 33 is a diagrammatic perspective view of part of a mist generator of an example of this disclosure.

Figure 34 is a diagrammatic perspective view of part of a mist generator of an example of this disclosure.

Figure 35 is a diagrammatic perspective view of part of a mist generator of an example of this disclosure.

Figure 36 is a diagrammatic perspective view of part of a mist generator of an example of this disclosure.

Figure 37 is a diagrammatic perspective view of part of a mist generator of an example of this disclosure.

Figure 38 is a diagrammatic perspective view of part of a mist generator of an example of this disclosure.

Figure 39 is a diagrammatic perspective view of part of a mist generator of an example of this disclosure.

Figure 40 is a diagrammatic perspective view of part of a mist generator of an example of this disclosure.

Figure 41 is a diagrammatic perspective part cutaway view of part of a mist generator of an example of this disclosure.

Figure 42 is a diagrammatic perspective view of part of a mist generator of an example of this disclosure.

Figure 43 is a diagrammatic perspective view of part of a mist generator of an example of this disclosure.

Figure 44 is a diagrammatic perspective view of part of a mist generator of an example of this disclosure.

Figure 45 is a diagrammatic perspective cross-sectional view of part of a mist generator of an example of this disclosure.

Figure 46 is a diagrammatic perspective cross-sectional view of part of a mist generator of an example of this disclosure.

Figure 47 is a diagrammatic perspective view of part of a mist generator of an example of this disclosure.

Figure 48 is a diagrammatic perspective view of part of a mist generator of an example of this disclosure.

Figure 49 is a diagrammatic perspective view of part of a mist generator of an example of this disclosure.

Figure 50 is a diagrammatic perspective view of a mist generator of an example of this disclosure.

Figure 51 is a diagrammatic perspective view of a mist generator of an example of this disclosure.

Figure 52 is a diagrammatic perspective cross-sectional view of part of a mist generator of an example of this disclosure.

Figure 53 is a diagrammatic perspective exploded view of a mist generator of an example of this disclosure.

Figure 54 is a diagrammatic cross-sectional view of part of a mist generator of an example of this disclosure.

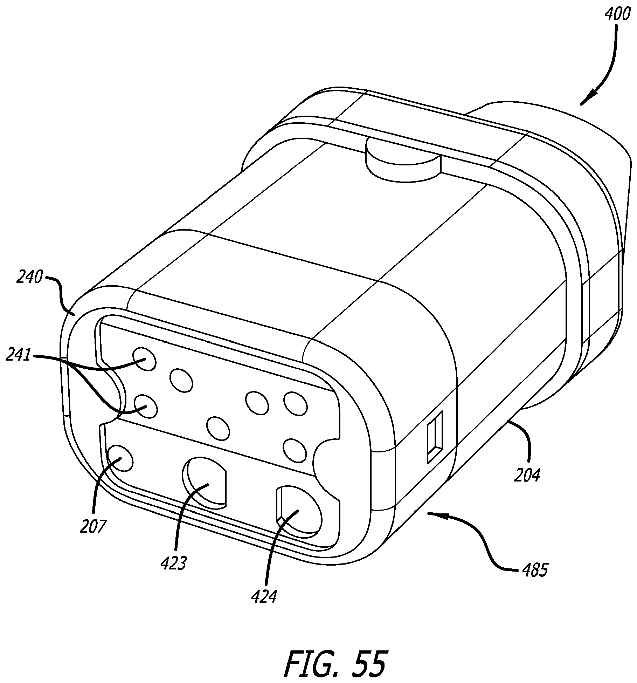

Figure 55 is a diagrammatic perspective view of a mist generator of an example of this disclosure.

Figure 56 is a diagrammatic perspective cross-sectional view of part of a mist generator of an example of this disclosure.

Figure 57 is a diagrammatic perspective cross-sectional view of part of a mist generator of an example of this disclosure showing an airflow path.

Figure 58 is a diagrammatic perspective cross-sectional view of part of a mist generator of an example of this disclosure showing an airflow path.

Figure 59 is a diagrammatic perspective cross-sectional view of part of a mist generator of an example of this disclosure showing an airflow path.

Figure 60 is a diagrammatic perspective view of a mist generator of this disclosure.

Figure 61 is a diagrammatic perspective view of a mist generator of this disclosure.

Figure 62 is a diagrammatic exploded perspective view of a mist generator of this disclosure.

Figure 63 is a diagrammatic exploded perspective view of a part of a mist generator of this disclosure.

Figure 64 is a diagrammatic exploded perspective view of a part of a mist generator of this disclosure.

Figure 65 is a diagrammatic exploded perspective view of a part of a mist generator of this disclosure.

Figure 66 is a diagrammatic perspective cross-sectional view of a part of a mist generator of this disclosure.

Figure 67 is a diagrammatic perspective view of a part of a mist generator of this disclosure.

Figure 68 is a diagrammatic perspective view of a part of a mist generator of this disclosure.

Figure 69 is a diagrammatic perspective cross-sectional view of a mist generator of this disclosure.

Figure 70 is a diagrammatic perspective cross-sectional view of a mist generator of this disclosure.

Figure 71 is a diagrammatic exploded perspective view of a mist generator of this disclosure.

Figure 72 is a diagrammatic perspective view of a mist generator of this disclosure.

Figure 73 is a diagrammatic perspective view of a mist generator of this disclosure.

Figure 74 is a diagrammatic exploded perspective view of a mist generator of this disclosure.

Figure 75 is a diagrammatic exploded perspective view of a driver of this disclosure.

Figure 76 is a diagrammatic perspective view of part of a driver of this disclosure.

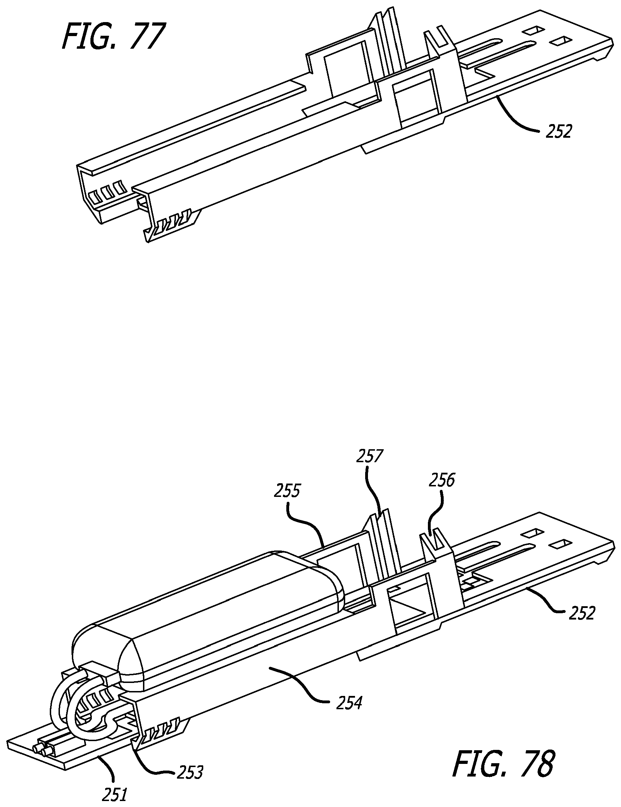

Figure 77 is a diagrammatic perspective view of part of a driver of this disclosure.

Figure 78 is a diagrammatic perspective view of part of a driver of this disclosure.

Figure 79 is a diagrammatic perspective view of part of a driver of this disclosure.

Figure 80 is a diagrammatic perspective view of part of a driver of this disclosure.

Figure 81 is a diagrammatic perspective view of part of a driver of this disclosure.

Figure 82 is a diagrammatic perspective view of part of a driver of this disclosure.

Figure 83 is a schematic diagram of an integrated circuit arrangement of this disclosure.

Figure 84 is a schematic diagram of an integrated circuit of this disclosure.

Figure 85 is a schematic diagram of a pulse width modulation generator of this disclosure.

Figure 86 is timing diagram of an example of this disclosure.

Figure 87 is timing diagram of an example of this disclosure.

Figure 88 is a table showing port functions of an example of this disclosure.

Figure 89 is a schematic diagram of an integrated circuit of this disclosure.

Figure 90 is a circuit diagram of an H-bridge of an example of this disclosure.

Figure 91 is a circuit diagram of a current sense arrangement of an example of this disclosure.

Figure 92 is a circuit diagram of an H-bridge of an example of this disclosure.

Figure 93 is a graph showing the voltages during the phases of operation of the H-bridge of Figure 90.

Figure 94 is a graph showing the voltages during the phases of operation of the H-bridge of Figure 90.

Figure 95 is a graph showing the voltage and current at a terminal of an ultrasonic transducer while the ultrasonic transducer is being driven by the H-bridge of Figure 90.

Figure 96 is a schematic diagram showing connections between integrated circuits of this disclosure.

Figure 97 is a schematic diagram of an integrated circuit of this disclosure.

Figure 98 is diagram illustrating the steps of an authentication method of an example of this disclosure.

Figure 99 is a diagrammatic perspective view of an end cap of a driver of this disclosure.

Figure 100 is a diagrammatic perspective view of the housing of a driver of this disclosure.

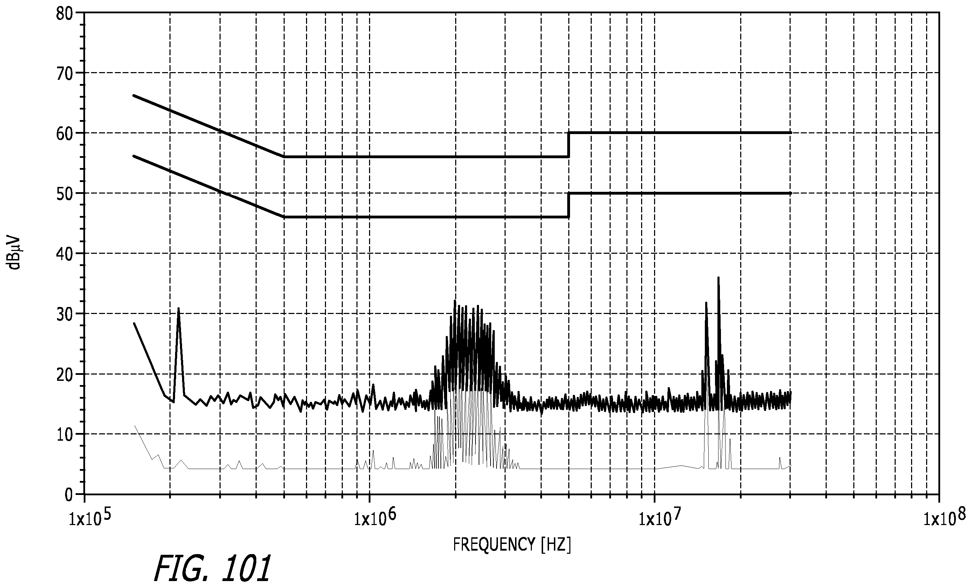

Figure 101 is a graph showing the result of an EMC test for a mist delivery device of this disclosure.

## DETAILED DESCRIPTION OF THE DISCLOSURE

Aspects of the present disclosure are best understood from the following detailed description when read with the accompanying figures. It is noted that, in accordance with the standard practice in the industry, various features are not drawn to scale. In fact, the dimensions of the various features may be arbitrarily increased or reduced for clarity of discussion.

The following disclosure provides many different embodiments, or examples, for implementing different features of the provided subject matter. Specific examples of components, concentrations, applications and arrangements are described below to simplify the present disclosure. These are, of course, merely examples and are not intended to be limiting. For example, the attachment of a first feature and a second feature in the description that follows may include embodiments in which the first feature and the second feature are attached in direct contact, and may also include embodiments in which additional features may be positioned between the first feature and the second feature, such that the first feature and the second feature may not be in direct contact. In addition, the present disclosure may repeat reference numerals and/or letters in the various examples. This repetition is for the purpose of simplicity and clarity and does not in itself dictate a relationship between the various embodiments and/or configurations discussed.

The following disclosure describes representative examples. Each example may be considered to be an embodiment and any reference to an "example" may be changed to "embodiment" in the present disclosure.

Some parts of the present disclosure are directed to an electronic vaporising inhaler. The specific example described below involves nicotine. However, other examples are envisioned, such as an inhaler for therapeutics, medicine, and herbal supplements. Additionally, the device can be packaged to look like a medical device which does not resemble a cigarette.

Ultrasonic mist inhalers are either disposable or reusable. The term "reusable" as used herein implies that the energy storage device is rechargeable or replaceable or that the liquid is able to be replenished either through refilling or through replacement of the liquid reservoir structure. Alternatively, in some examples reusable electronic device is both rechargeable and the liquid can be replenished.

Conventional electronic vaporising inhaler tend to rely on inducing high temperatures of a metal component configured to heat a liquid in the inhaler, thus vaporising the liquid that can be breathed in. The liquid typically contains components blended into a solution of propylene glycol (PG) and vegetable glycerin (VG), which is vaporised via a heating component at high temperatures. Problems with conventional inhaler may include the possibility of burning metal and subsequent breathing in of the metal along with the burnt liquid. In addition, some may not prefer the burnt smell or taste caused by the heated liquid.

For each different application for a mist generation system, there is an optimum frequency or frequency range for driving the means of ultrasonic vibrations 5 in order to optimize the generation of mist. In examples where the means of ultrasonic vibrations 5 is a piezoelectric transducer, the optimum frequency or frequency range will depend on at least the following four parameters:

## 1. Transducer Manufacturing Processes

In some examples, the means of ultrasonic vibrations 5 comprises a piezoelectric ceramic. The piezoelectric ceramic is manufactured by mixing compounds to make a ceramic dough and this mixing process may not be consistent throughout production. This inconsistency can give rise to a range of different resonant frequencies of the cured piezoelectric ceramic.

If the resonant frequency of the piezoelectric ceramic does not correspond to the required frequency of operation of the device then no mist is produced during the operation of the device. In the case of a therapeutic mist inhaler, even a slight offset in the resonant frequency of the piezoelectric ceramic is enough to impact the production of mist, meaning that the device will not deliver adequate therapeutic levels to the user.

2. Load on transducer

During operation, any changes in the load on the piezoelectric transducer will inhibit the overall displacement of the oscillation of the piezoelectric transducer. To achieve optimal displacement of the oscillation of the piezoelectric transducer, the drive frequency must be adjusted to enable the circuit to provide adequate power for maximum displacement.

The types of loads that can affect the oscillator's efficiency can include the amount of liquid on the transducer (dampness of the wicking material), and the spring force applied to the wicking material to keep permanent contact with the transducer. It may also include the means of electrical connection.

3. Temperature

Ultrasonic oscillations of the piezoelectric transducer are partially damped by its assembly in a device. This may include the transducer being placed in a silicone/rubber ring, and the spring exerting pressure onto the wicking material that is above the transducer. This dampening of the oscillations causes rise in local temperatures on and around the transducer.

An increase in temperature affects the oscillation due to changes in the molecular behaviour of the transducer. An increase in the temperature means more energy to the molecules of the ceramic, which temporarily affects its crystalline structure. Although the effect is reversed as the temperature reduces, a modulation in supplied frequency is required to maintain optimal oscillation. This modulation of frequency cannot be achieved with a conventional fixed frequency device.

An increase in temperature also reduces the viscosity of the solution (e-liquid) which is being vaporized, which may require an alteration to the drive frequency to induce cavitation and maintain continuous mist production. In the case of a conventional fixed frequency device, a reduction in the viscosity of the liquid without any change in the drive frequency will reduce or completely stop mist production, rendering the device inoperable.

## 4. Distance to Power Source

The oscillation frequency of the electronic circuit can change depending on the wire-lengths between the transducer and the oscillator-driver. The frequency of the electronic circuit is inversely proportional to the distance between the transducer and the remaining circuit.

Although the distance parameter is primarily fixed in a device, it can vary during the manufacturing process of the device, reducing the overall efficiency of the device. Therefore, it is desirable to modify the drive frequency of the device to compensate for the variations and optimise the efficiency of the device.

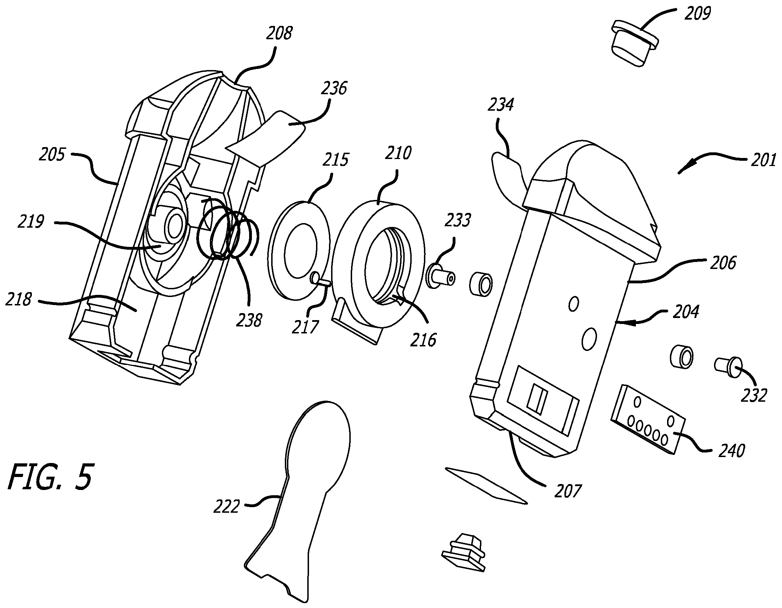

A piezoelectric transducer can be modelled as an RLC circuit in an electronic circuit, as shown in Figure 5. The four parameters described above may be modelled as alterations to the overall inductance, capacitance, and/or resistance of the RLC circuit, changing the resonance frequency range supplied to the transducer. As the frequency of the circuit increases to around the resonance point of the transducer, the log Impedance of the overall circuit dips to a minimum and then rises to a maximum before settling to a median range.

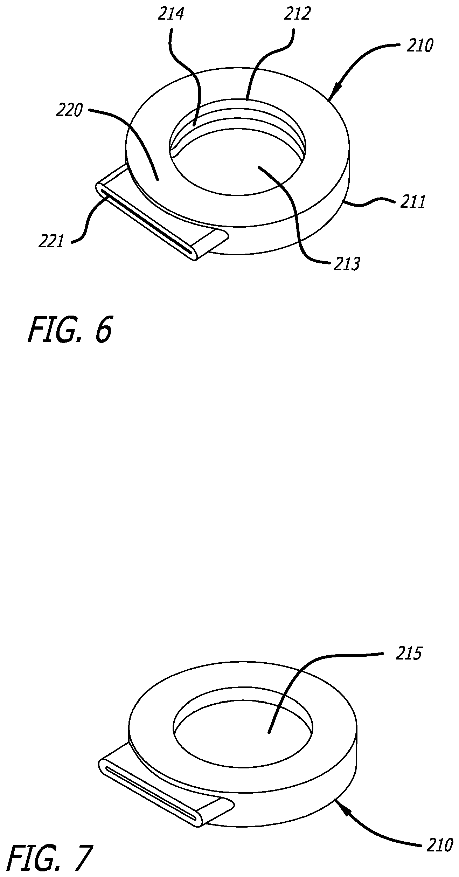

Figure 6 shows a generic graph explaining the change in overall impedance with increase in frequency in an RLC circuit. Figure 7 shows how a piezoelectric transducer acts as a capacitor in a first capacitive region at frequencies below a first predetermined frequency $f_s$ and in a second capacitive region at frequencies above a second predetermined frequency $f_p$. The piezoelectric transducer acts as an inductor in an inductive region at frequencies between the first and second predetermined frequencies $f_s$, $f_p$. In order to maintain optimal oscillation of the transducer and hence maximum efficiency, the current flowing through the transducer must be maintained at a frequency within the inductive region.

The frequency controller of the device of some examples is configured to maintain the frequency of oscillation of the piezoelectric transducer (the means of ultrasonic vibrations 5) within the inductive region, in order to maximise the efficiency of the device.

The frequency controller is configured to perform a sweep operation in which the frequency controller drives the transducer at frequencies which track progressively across a predetermined sweep frequency range. As the frequency controller performs the sweep, the frequency controller monitors an Analog-to-Digital Conversion (ADC) value of an Analog-to-Digital converter which is coupled to the transducer. In some examples the ADC value is a parameter of the ADC which is proportional to the voltage across the transducer. In other examples, the ADC value is a parameter of the ADC which is proportional to the current flowing through the transducer.

As will be described in more detail below, the frequency controller of some examples determines the active power being used by the ultrasonic transducer by monitoring the current flowing through the transducer.

During the sweep operation, the frequency controller locates the inductive region of the frequency for the transducer. Once the frequency controller has identified the inductive region, the frequency controller records the ADC value and locks the drive frequency of the transducer at a frequency within the inductive region (i.e. between the first and second predetermined frequencies $f_s$, $f_p$) in order to optimise the ultrasonic cavitation by the transducer. When the drive frequency is locked within the inductive region, the electromechanical coupling factor of the transducer is maximised, thereby maximising the efficiency of the device.

In some examples, the frequency controller is configured to perform the sweep operation to locate the inductive region each time the oscillation is started or re-started. In the examples, the frequency controller is configured to lock the drive frequency at a new frequency within the inductive region each time the oscillation is started and thereby compensate for any changes in the parameters that affect the efficiency of operation of the device.

In some examples, the frequency controller ensures optimal mist production and maximises efficiency of therapeutic delivery to the user. In some examples, the frequency controller optimises the device and improves the efficiency and maximises therapeutic delivery to the user.

In other examples, the frequency controller optimises the device and improves the efficiency of any other device which uses ultrasound. In some examples, the frequency controller is configured for use with ultrasound technology for therapeutic applications in order to extend the enhancement of drug release from an ultrasound-responsive drug delivery system. Having precise, optimal frequency during operation, ensures that the microbubbles, nanobubbles, nanodroplets, liposome, emulsions, micelles or any other delivery systems are highly effective.

In some examples, in order to ensure optimal mist generation and optimal delivery of compounds as described above, the frequency controller is configured to operate in a recursive mode. When the frequency controller operates in the recursive mode, the frequency controller runs the sweep of frequencies periodically during the operation of the device and monitors the ADC value to determine if the ADC value is above a predetermined threshold which is indicative of optimal oscillation of the transducer.

In some examples, the frequency controller runs the sweep operation while the device is in the process of aerosolising liquid in case the frequency controller is able to identify a possible better frequency for the transducer. If the frequency controller identifies a better frequency, the frequency controller locks the drive frequency at the newly identified better frequency in order to maintain optimal operation of the device.

In some examples, the frequency controller runs the sweep of frequencies for a predetermined duration periodically during the operation of the device. In the case of the device of the examples described above, the predetermined duration of the sweep and the time period between sweeps are selected to optimise the functionality of the device. When implemented in an ultrasonic mist delivery device, this will ensure an optimum delivery to a user throughout the user's inhalation.

To ensure adequate aerosol production, in this example the mist delivery device comprises an ultrasonic/piezoelectric transducer of exactly or substantially 16mm diameter. This transducer is manufactured to specific capacitance and impedance values to control the frequency and power required for desired aerosol volume production.

A horizontally placed disc-shaped 16mm diameter ultrasonic transducer would result in a large device that may not be ergonomic as handheld. To mitigate this concern, the ultrasonic transducer of this example is held vertically in the sonication chamber (the planar surface of the ultrasonic transducer is generally parallel with the flow of aerosol mist to the mouthpiece and/or generally parallel to the longitudinal length of the mist delivery device). Put another way, the ultrasonic transducer is generally perpendicular to a base of the mist delivery device.

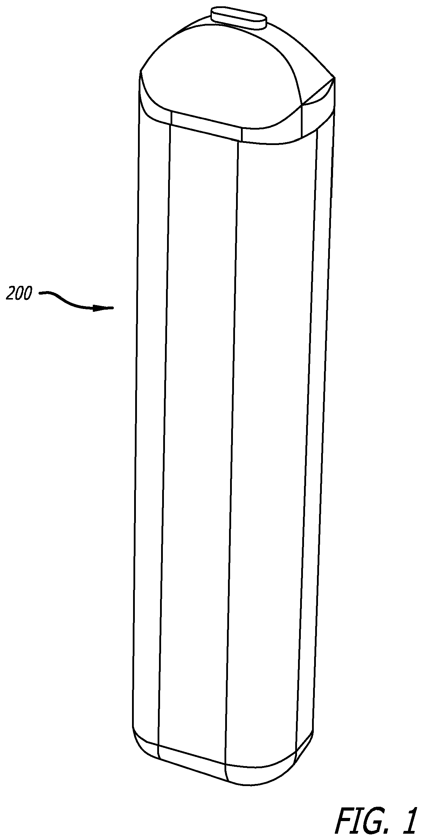

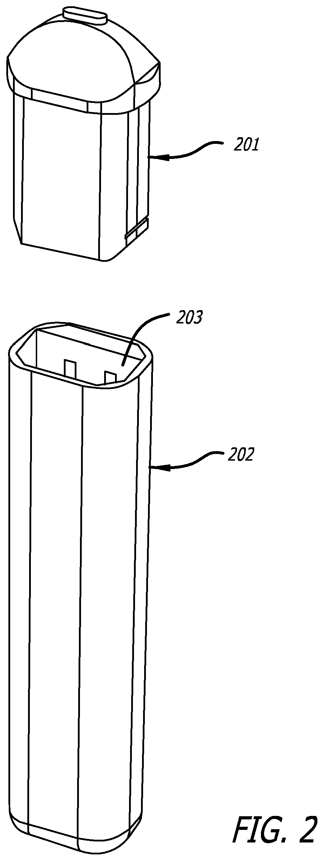

Referring now to Figures 1 and 2 of the accompanying drawings, a mist delivery device 200 of some examples comprises a mist generator 201 and a driver 202. The driver 202 is, in this example, provided with a recess 203 which receives and retains part of the mist generator 201. The mist generator 201 can therefore be coupled with the driver 202 to form a compact and portable mist delivery device 200, as shown in Figure 1.

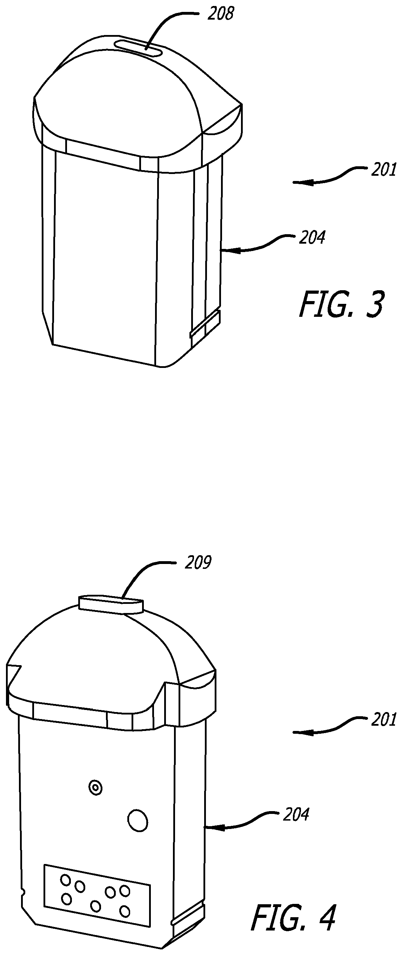

Referring now to Figures 3 to 5 of the accompanying drawings, the mist generator 201 comprises a mist generator housing 204 which is elongate and optionally formed from two housing portions 205, 206 which are attached to one another. The mist generator housing 204 comprises an air inlet port 207 and a mist outlet port 208.

In this example, the mist generator housing 204 is of injection moulded plastic, specifically polypropylene that is typically used for medical applications. In this example, the mist generator housing 204 is of a heterophasic copolymer. More particularly a BF970MO heterophasic copolymer, which has an optimum combination of very high stiffness and high impact strength. The mist generator housing parts moulded with this material exhibit good anti-static performance.

A heterophasic copolymer such as polypropylene is particularly suitable for the mist generator housing 204 since this material does not cause condensation of the aerosol as it flows from the sonication chamber 219 through the mouthpiece to the user. This plastic material can also be directly recycled easily using industrial shredding and cleaning processes.

In Figures 1, 2 and 4, the mist outlet port 208 is closed by a closure element 209. However, it is to be appreciated that when the mist inhaler 200 is in use, the closure element 209 is removed from the mist outlet port 208, as shown in Figure 3.

Referring now to Figures 6 and 7, the mist generator 200 comprises a transducer holder 210 which is held within the mist generator housing 204. The transducer holder 210 comprises a body portion 211 which, in this example, is cylindrical or generally cylindrical in shape with circular upper and lower openings 212, 213. The transducer holder 210 is provided with an internal recess 214 for receiving an edge of an ultrasonic transducer 215, as shown in Figure 7.

The transducer holder 210 incorporates a cutaway section 216 through which an electrode 217 extends from the ultrasonic transducer 215 so that the electrode 217 may be connected electrically to an AC driver of the drive device, as described in more detail below.

Referring again to Figure 5, the mist generator 201 comprises a liquid chamber 218 which is provided within the mist generator housing 204. The liquid chamber 218 is for containing a liquid, such as a therapeutic liquid, to be atomised. In some examples, a liquid is contained in the liquid chamber 218. In other examples, the liquid chamber 218 is empty initially and the liquid chamber is filled with a liquid subsequently.

The liquid preferably includes at least one therapeutic suitable for the aerosol delivery to the lungs through inhalation by a patient for providing a desired treatment to the patient. Some examples of therapeutics include, but are not limited to, aerosol delivery of pharmacological agents to the lungs to promote a systemic or direct clinical effect while producing a minimal adverse-effect. A therapeutic may also include for example, but not limited to, natural drugs, cannabinoid derivatives such as CBD for pain relief and other treatments; botanicals; opioids; RNA; DNA; chemotherapies; subcellular components including for example ribosomes, endoplasmic reticulum, cytoskeleton, and mitochondria; supplements for performance enhancement; albuterol/salbutamol for asthma patients; agents such as beta-lactams, polymyxins, and aminoglycosides bactericidal antibiotics; amphotericin B; morphine; fentanyl; prostacyclin, amiloride, and interferon-g; and cyclosporine as rescue therapy rejection in lung-transplantation patients and a treatment for asthma.

The following description refers to nicotine, but in other examples of this disclosure the nicotine is replaced with a therapeutic, such as, but not limited to one or more of the therapeutics described herein.

A liquid (also referred to herein as an e-liquid) composition suitable for use in an ultrasonic device that is powered at a frequency of 3.0 MHz ($\pm$0.2 MHz) by a 3.7V lithium polymer (LiPo) battery consisting of a nicotine salt consisting of nicotine levulinate wherein:

The relative amount of vegetable glycerin in the composition is: from 55 to 80% (w/w), or from 60 to 80% (w/w), or from 65 to 75% (w/w), or 70% (w/w); and/or,
The relative amount of propylene glycol in the composition is: from 5 to 30% (w/w), or from 10 to 30% (w/w), or from 15 to 25% (w/w), or 20% (w/w); and/or,
The relative amount of water in the composition is: from 5 to 15% (w/w), or from 7 to 12% (w/w), or 10% (w/w); and/or,
The amount of nicotine and/or nicotine salt in the composition is: from 0.1 to 80 mg/ml, or from 0.1 to 50 mg/ml, or from 1 to 25 mg/ml, or from 10 to 20 mg/ml, or 17 mg/ml.

In some examples, the mist generator 201 contains an e-liquid having a kinematic viscosity between 1.05 Pa•s and 1.412 Pa•s.

In some examples, the liquid chamber 218 contains a liquid comprising a nicotine levulinate salt at a 1:1 molar ratio.

In some examples, the liquid chamber 218 contains an e-liquid or liquid comprising at least one therapeutic.

In some examples, the liquid chamber 218 contains an e-liquid or liquid comprising at least one cannabinoid or phytocannabinoid derived from a cannabis plant. In some examples, the at least one cannabinoid or phytocannabinoid comprises one or more of tetrahydrocannabinol (THC), cannabidiol (CBD) and/or cannabinol (CBN).

In some examples, the liquid chamber 218 contains an e-liquid or liquid comprising a psychedelic compound or compounds for use as a therapeutic. In some examples, the psychedelic compound or compounds is one or more of lysergic acid diethylamide (LSD), 3,4-methylenedioxymethamphetamine (MDMA), ketamine, esketamine, ibogaine, mescaline, a tryptamine, a substituted tryptamine, O-acetylpsilocin (4-AcO-DMT), psilocybin (4-PO-DMT), psilocin (4-HO-DMT), O-methylbufotenin (5-MeO-DMT), - 46 -ufotenine (5-HO-DMT) and/or N,N-Dimethyltryptamine (DMT or N,N-DMT).

In some examples, the liquid chamber 218 contains an e-liquid or liquid comprising at least one cannabinoid or phytocannabinoid derived from a cannabis plant. In some examples, the at least one cannabinoid or phytocannabinoid comprises one or more of tetrahydrocannabinol (THC), cannabidiol (CBD) and/or cannabinol (CBN).

In some examples, the liquid chamber 218 contains a liquid having a kinematic viscosity between 1.05 Pa•s and 1.412 Pa•s and a liquid density between 1.1 g/ml and 1.3 g/ml.

By using an e-liquid with the correct parameters of viscosity, density and having a desired target bubble volume of liquid spray into the air, it has been found that the frequency range of 2.8MHz to 3.2MHz for liquid viscosity range of 1.05 Pa•s and 1.412 Pa•s and density of approximately 1.1-1.3 g/mL (get density ranges from Hertz) produce a droplet volume where 90% of droplets are below 1 micron and 50% of those are less than 0.5 microns.

The mist generator 201 comprises a sonication chamber 219 which is provided within the mist generator housing 204.

Returning to Figures 6 and 7, the transducer holder 210 comprises a divider portion 220 which provides a barrier between the liquid chamber 218 and the sonication chamber 219. The barrier provided by the divider portion 220 minimises the risk of the sonication chamber 219 being is flooded with liquid from the liquid chamber 218 or for a capillary element over the ultrasonic transducer 215 becoming oversaturated, either of which would overload and reduce the efficiency of the ultrasonic transducer 215. Moreover, flooding the sonication chamber 219 or over saturating the capillary element could also cause an unpleasant experience with the liquid being sucked in by the user during inhalation. To mitigate this risk, the divider portion 220 of the transducer holder 210 sits as a wall between the sonication chamber 219 and the liquid chamber 218.

The divider portion 220 comprises a capillary aperture 221 which is the only means by which liquid can flow from the liquid chamber 218 to the sonication chamber 219, via a capillary element. In this example, the capillary aperture 221 is an elongate slot having a width of 0.2mm to 0.4mm. The dimensions of the capillary aperture 221 are such that the edges of the capillary aperture 221 provide a biasing force which acts on a capillary element extending through the capillary aperture 221 for added control of liquid flow to the sonication chamber 219.

In this example, the transducer holder 210 is of liquid silicone rubber (LSR). In this example, the liquid silicone rubber has a Shore A 60 hardness. This LSR material ensures that the ultrasonic transducer 215 vibrates without the transducer holder 210 dampening the vibrations. In this example, the vibratory displacement of the ultrasonic transducer 215 is 2-5 nanometres and any dampening effect may reduce the efficiency of the ultrasonic transducer 215. Hence, this LSR material and hardness is selected for optimal performance with minimal compromise.

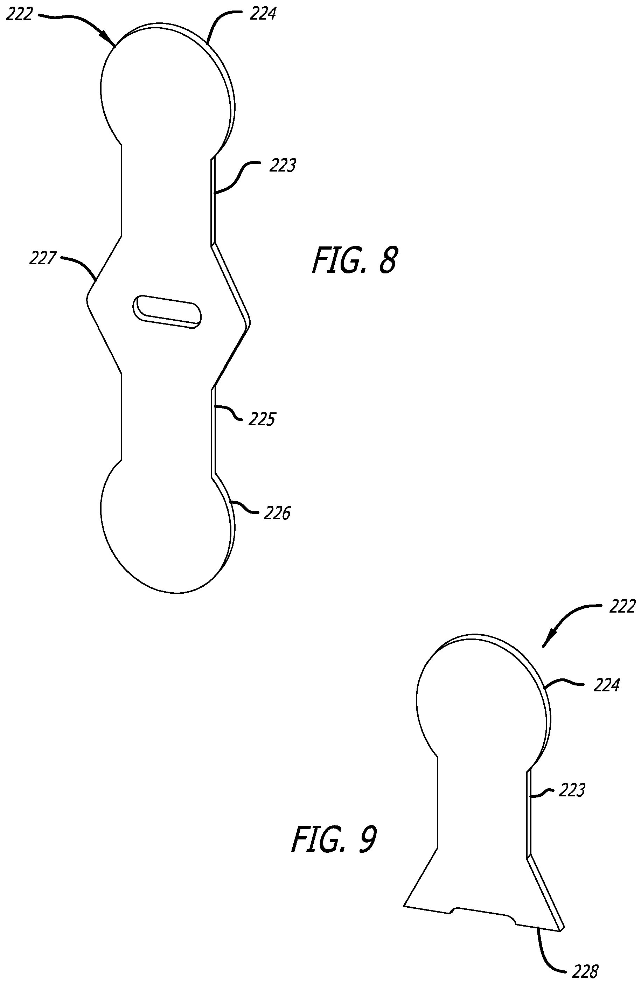

Referring now to Figures 8 and 9, the mist generator 201 comprises a capillary or capillary element 222 for transferring a liquid (containing a drug, therapeutic or other substance) from the liquid chamber 218 to the sonication chamber 219. The capillary element 222 is planar or generally planar with a first portion 223 and a second portion 224. In this example, the first portion 223 has a rectangular or generally rectangular shape and the second portion 224 has a partly circular shape.

In this example, the capillary element 222 comprises a third portion 225 and a fourth portion 226 which are respectively identical in shape to the first and second portions 223, 224. The capillary element 222 of this example is folded about a fold line 227 such that the first and second portions 223, 224 and the third and fourth portions 225, 226 are superimposed on one another, as shown in Figure 9.

In this example, the capillary element has a thickness of approximately 0.28mm. When the capillary element 222 is folded to have two layers, as shown in Figure 9, the overall thickness of the capillary element is approximately 0.56mm. This double layer also ensures that there is always sufficient liquid on the ultrasonic transducer 215 for optimal aerosol production.

In this example, when the capillary element 222 is folded, the lower end of the first and third parts 223, 225 defines an enlarged lower end 228 which increases the surface area of the capillary element 222 in the portion of the capillary element 222 which sits in liquid within the liquid chamber 218 to maximise the rate at which the capillary element 222 absorbs liquid.

In this example, the capillary element 222 is 100% bamboo fibre. In other examples, the capillary element is of at least 75% bamboo fibre. The benefits of using bamboo fibre as the capillary element are as described above.

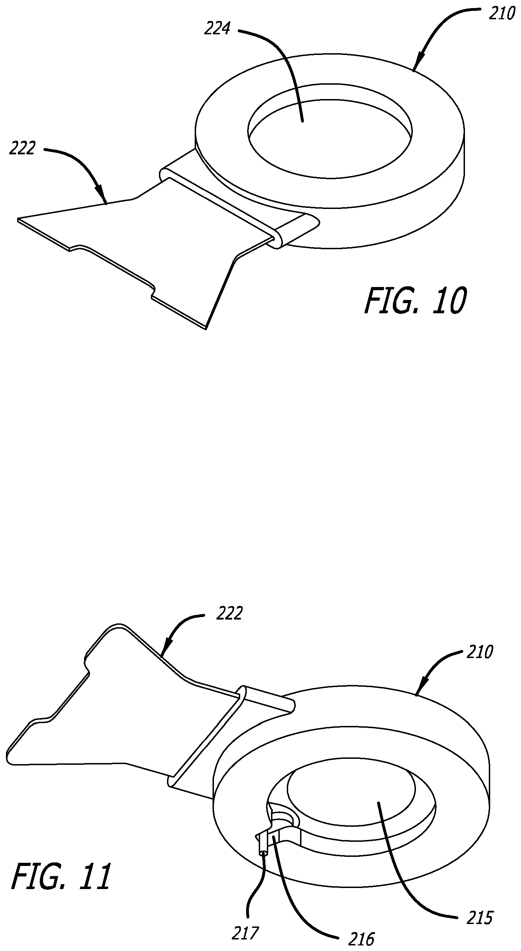

Referring now to Figures 10 and 11, the capillary element 222 is retained by the transducer holder 210 such that the transducer holder 210 retains the second portion 224 of the capillary element 222 superimposed on part of an atomisation surface of the ultrasonic transducer 215. In this example, the circular second portion 224 sits within the internal recess 214 of the transducer holder 210.

The first portion 223 of the capillary element 222 extends through the capillary aperture 221 in the transducer holder 210.

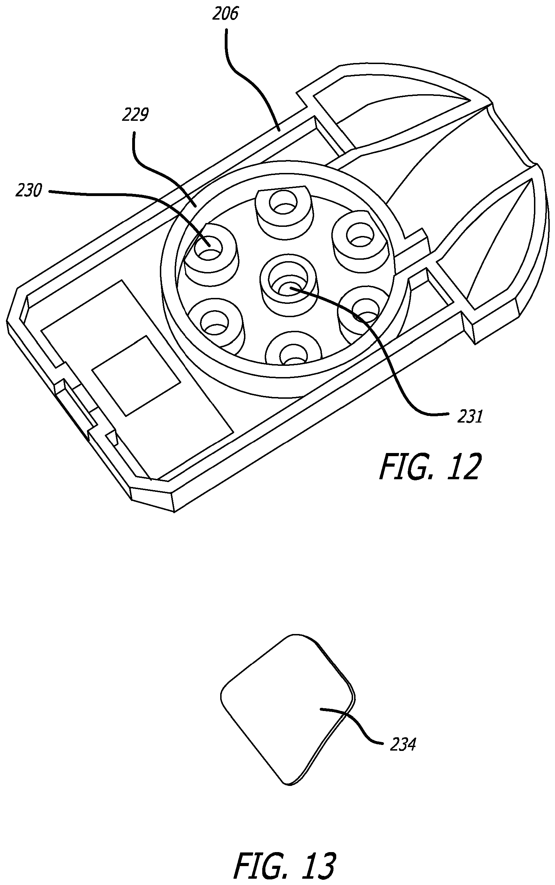

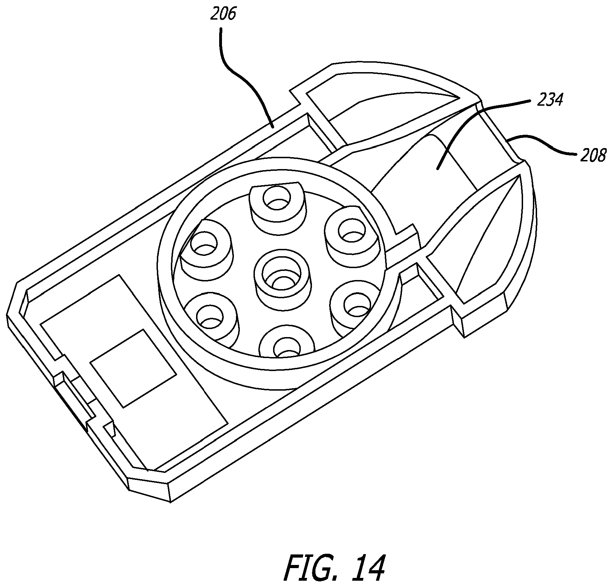

Referring now to Figures 12 to 14, the second portion 206 of the mist generator housing 204 comprises a generally circular wall 229 which receives the transducer holder 210 and forms part of the wall of the sonication chamber 219.

Contact apertures 230 and 231 are provided in a side wall of the second portion 206 for receiving electrical contacts 232 and 233 which form electrical connections with the electrodes of the ultrasonic transducer 215.

In this example, an absorbent tip or absorbent element 234 is provided adjacent the mist outlet port 208 to absorb liquid at the mist outlet port 208. In this example, the absorbent element 234 is of bamboo fibre.

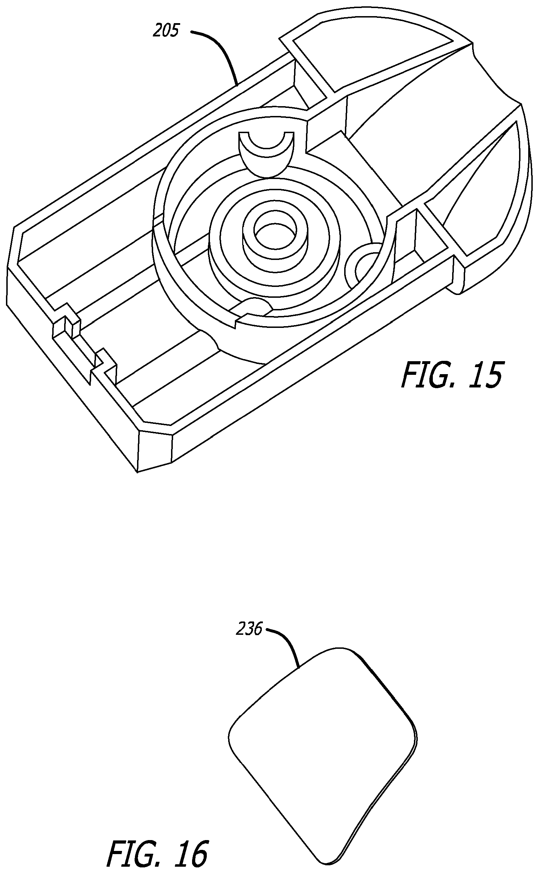

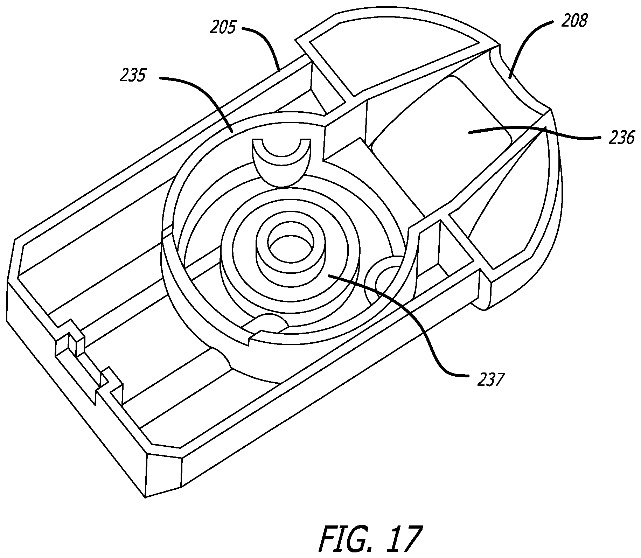

Referring now to Figures 15 to 17, the first portion 205 of the mist generator housing 204 is of a similar shape to the second portion 206 and comprises a further generally circular wall portion 235 which forms a further portion of the wall of the sonication chamber 219 and retains the transducer holder 210.

In this example, a further absorbent element 236 is provided adjacent the mist outlet port 208 to absorb liquid at the mist outlet port 208.

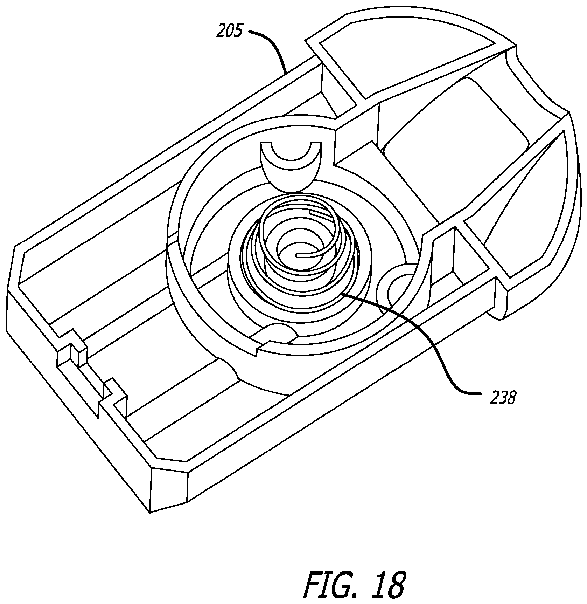

In this example, the first portion 205 of the mist generator housing 204 comprises a spring support arrangement 237 which supports the lower end of a retainer spring 238, as shown in Figure 18.

An upper end of the retainer spring 238 contacts the second portion 224 of the capillary element 222 such that the retainer spring 238 provides a biasing force which biases the capillary element 222 against the atomisation surface of the ultrasonic transducer 215.

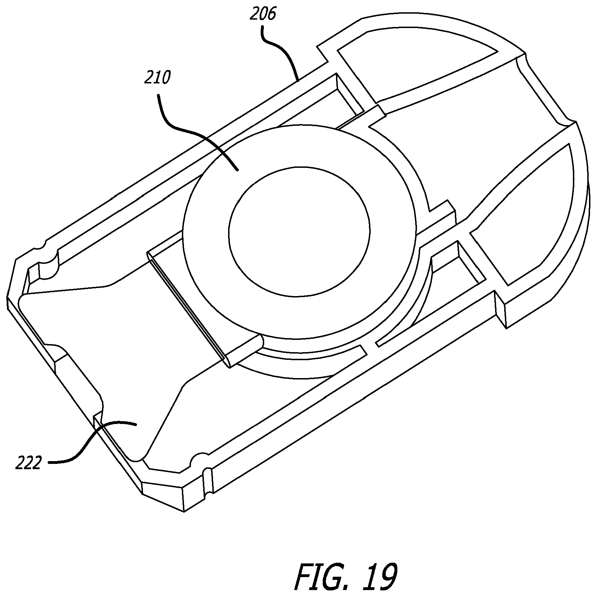

Referring to Figure 19, the transducer holder 210 is shown in position and being retained by the second portion 206 of the mist generator housing 204, prior to the two portions 205, 206 of the mist generator housing 204 being attached to one another.

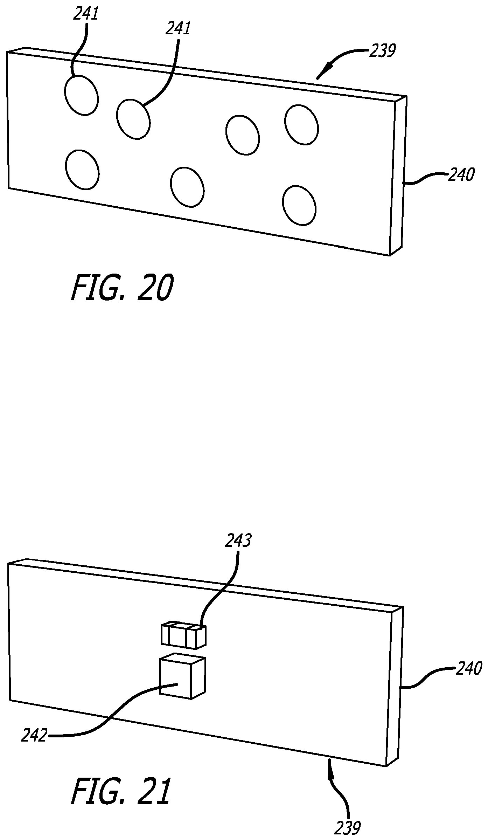

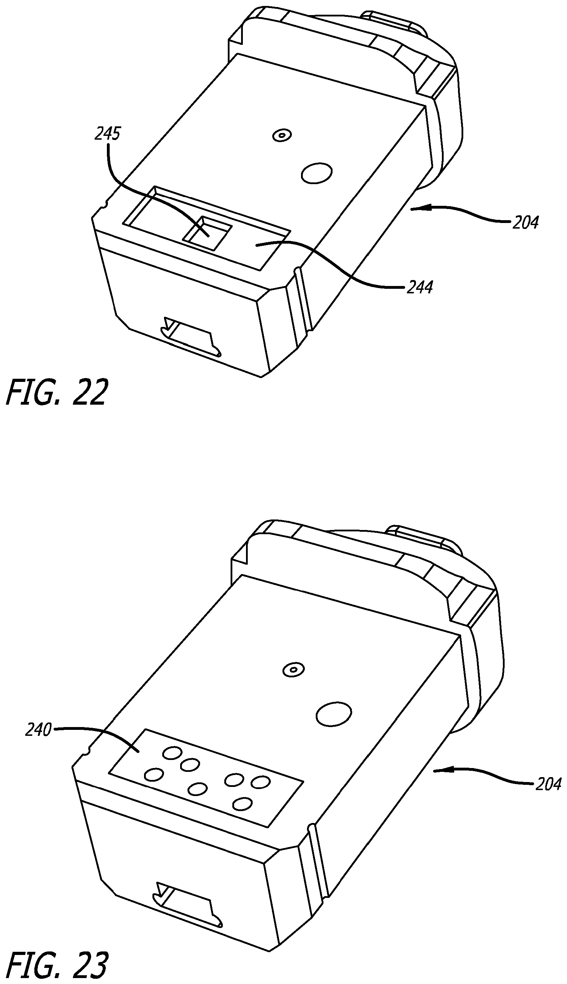

Referring to Figures 20 to 23, in this example, the mist generator 201 comprises an identification arrangement 239. The identification arrangement 239 comprises a printed circuit board 240 having electrical contacts 241 provided on one side and an integrated circuit 242 and another optional component 243 provided on the other side.

The integrated circuit 242 has a memory which stores a unique identifier for the mist generator 201. The electrical contacts 241 provide an electronic interface for communication with the integrated circuit 242.

The printed circuit board 240 is, in this example, mounted within a recess 244 on one side of the mist generator housing 204. The integrated circuit 242 and optional other electronic components 243 sit within a further recess 245 so that the printed circuit board 240 is generally flush with the side of the mist generator housing 204.

In this example, the integrated circuit 242 is a one-time-programmable (OTP) device which is an anti-counterfeiting feature that allows only genuine mist generators from the manufacturer to be used with the device. This anti-counterfeiting feature is implemented in the mist generator 201 as a specific custom integrated circuit (IC) that is bonded (with the printed circuit board 240) to the mist generator 201. The OTP as IC contains a truly unique information that allows a complete traceability of the mist generator 201 (and its content) over its lifetime as well as a precise monitoring of the consumption by the user. The OTP IC allows the mist generator 201 to function to generate mist only when authorised.

An implementation of the OTP IC of an example of this disclosure is described in detail below.

The OTP, as a feature, dictates the authorised status of a specific mist generator 201. Indeed, in order to prevent emissions of carbonyls and keep the aerosol at safe standards, experiments have shown that the mist generator 201 is considered empty of liquid in the liquid chamber 218 after approximately 1,000 seconds of aerosolisation. In that way a mist generator 201 that is not genuine or empty will not be able to be activated after this predetermined duration of use.

The OTP, as a feature, may be part of a complete chain with the conjunction of the digital sale point, the mobile companion application and the mist generator 201. Only a genuine mist generator 201 manufactured by a trusted party and sold on the digital sale point can be used in the device. A mobile companion digital app, being a link between the user account on a manufacturer's digital platform and the mist generator 201, ensures safe usage of a known safe content for a safe amount of puff duration.

The OTP, as a feature, also enables high access control and monitoring required as per medical drug administration in the case of business to business (B2B) use with trusted health establishments. The OTP IC is read by the driver 202 which can recognise the mist generator 201 inserted and the prescription associated with it. The driver 202 cannot be used with this mist generator 201 more than nor outside of the time frame specified by the prescription. In addition a reminder on the mobile companion app can be provided to minimise a user missing a dose.

In some examples, the OTP IC is disposable in the same way as the mist generator 201. Whenever the mist generator 201 is considered empty, it will not be activated if inserted into a driver 202. Similarly, a counterfeit generator device 201 would not be functional in the driver 202.

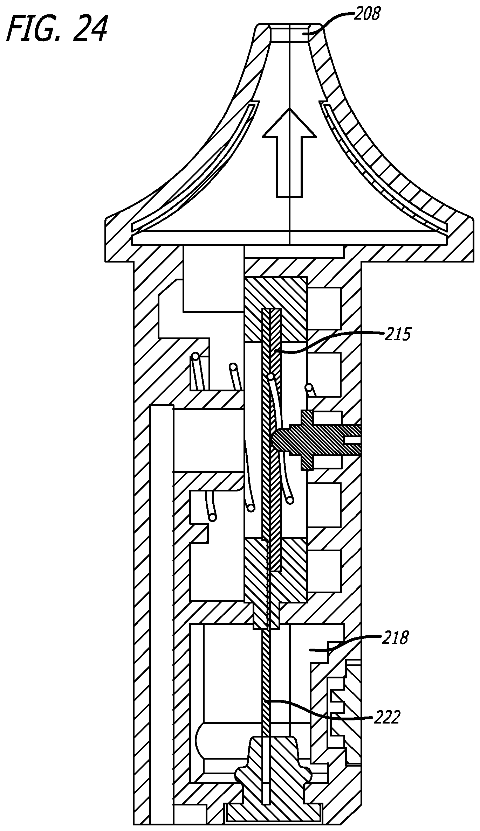

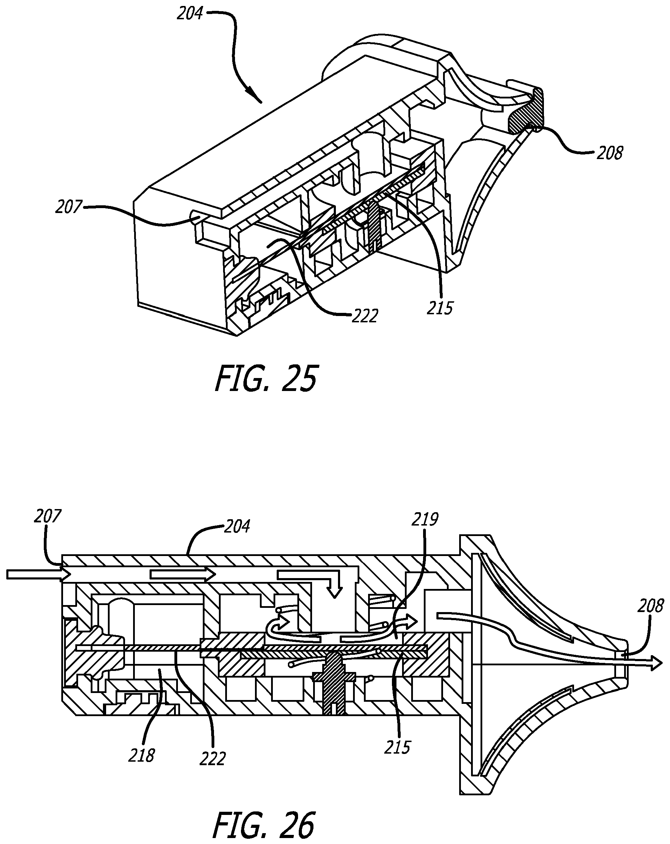

Figures 24 to 26 illustrate how air flows through the mist generator 201 during operation.

The sonication of the liquid therapeutic (medical solutions, medical suspensions, protein solutions, supplements, etc.) transforms it into mist (aerosolisation). However, this mist would settle over the ultrasonic transducer 215 unless enough ambient air is available to replace the rising aerosol. In the sonication chamber 219, there is a requirement for a continuous supply of air as mist (aerosol) is generated and pulled out through the mouthpiece to the user. To cater to this requirement, an airflow channel is provided. In this example the airflow channel has an average cross-sectional area of 11.5mm$^2$, which is calculated and designed into the sonication chamber 219 based on the negative air pressure from an average user. This also controls the mist-to-air ratio of the inhaled aerosol, controlling the amount of drug delivered to the user.

Based on design requirements, the air flow channel is routed such that it initiates from the bottom of the sonication chamber 219. The opening at the bottom of the aerosol chamber aligns with and is tightly adjacent to the opening to an airflow bridge in the device. The air flow channel runs vertically upwards along the reservoir and continues until the centre of the sonication chamber (concentric with the ultrasonic transducer 215). Here, it turns 90° inwards. The flow path then continues on until approximately 1.5mm from the ultrasonic transducer 215. This routing ensures maximised ambient air supplied directly in the direction of the atomisation surface of the ultrasonic transducer 215. The air flows through the channel, towards the transducer, collects the generated mist as it travels out through the mouthpiece and to the user.

Air flows into the mist generator 201 via the air inlet port 207 which, as described below, is in fluid communication with an airflow bridge within the driver 202. The air flows along a flow path which changes the direction of the air flow by approximately 90° to direct the flow of air towards the ultrasonic transducer 215.

In some examples, the airflow arrangement is configured to change the direction of a flow of air along the air flow path such that the flow of air is substantially perpendicular to the atomisation surface of the ultrasonic transducer as the flow of air passes into the sonication chamber.

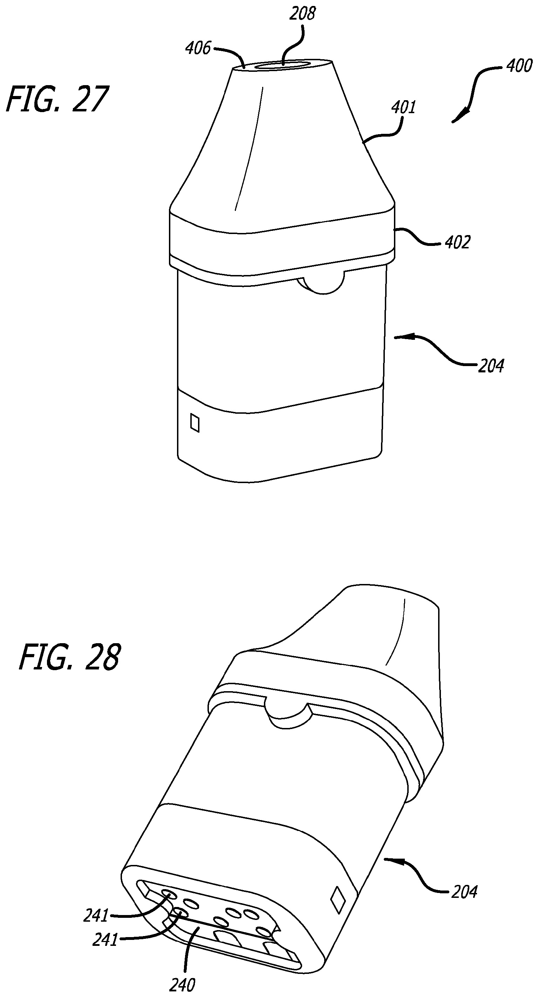

Referring now to Figures 27-29 of the accompanying drawings, a mist generator 400 of some examples of this disclosure comprises many of the same elements as the mist generator 201 described herein. The same reference numbers are used for equivalent elements of the mist generator 400 as the mist generator 201. Each mist generator 201, 401 described herein may alternatively be known as a pod or a cartridge.

The mist generator 400 is configured to be releasably attached to the driver 202 to form a mist delivery device for operation in substantially the same way as the mist delivery device 201 described herein. In other examples, the mist generator 400 may be fixed to, formed integrally with or otherwise non-releasably attached to the driver 202.

The mist generator 400 comprises a housing 204 comprising a liquid chamber 218 for containing a liquid to be atomised. The liquid chamber 218 may contain any e-liquid or liquid described in the present disclosure or any other liquid which is to be atomised.

The mist generator 400 comprises a mouthpiece 401 that is coupled to the housing 204. The mouthpiece 401 comprises a base 402 having an opening 403 which receives a connector portion 404 of the housing 204. The connector portion 404 comprises at least one latch element 405 that engages a latch recess (not shown) to retain the mouthpiece 401 in connection with the housing 204.

The mouthpiece 401 narrows progressively from the base 402 to a distal end 406. The distal end 406 comprises a mist outlet port 208 to enable mist to be output from the mist generator 400 for inhalation by a user.

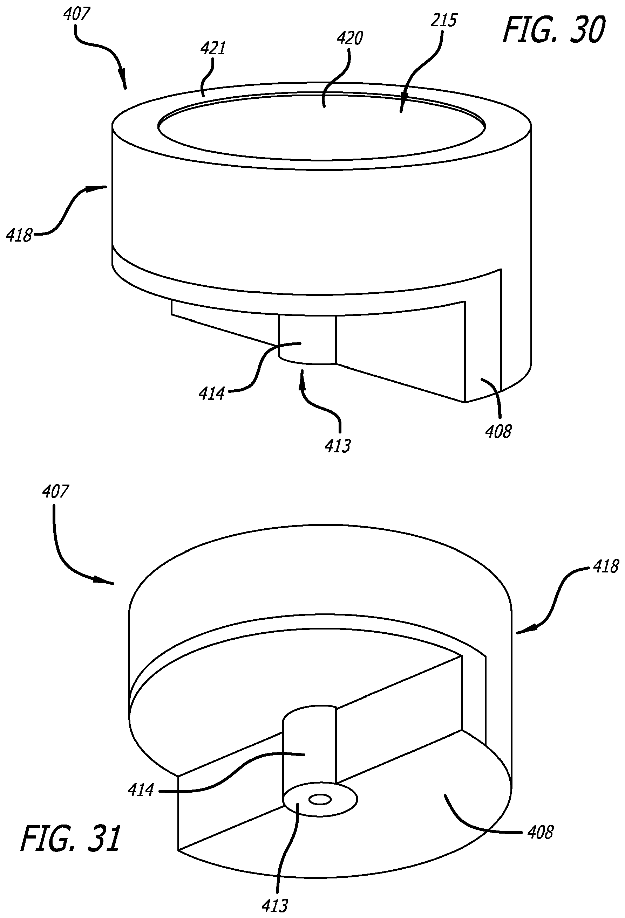

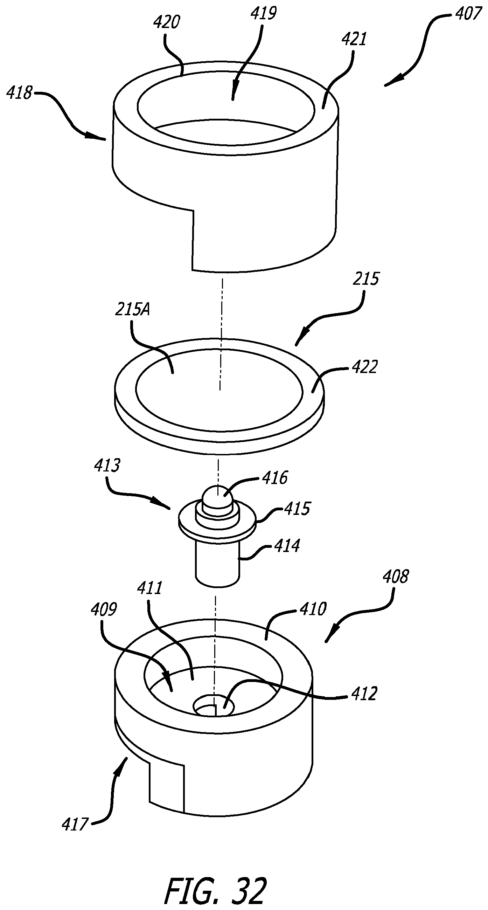

Referring now to figures 30-32 of the accompanying drawings, the mist generator 400 comprises an ultrasonic transducer 215. In this example, the ultrasonic transducer 215 is received within an ultrasonic transducer stack 407 which may alternatively be known as an ultrasonic transducer assembly. The ultrasonic transducer stack 407 comprises a generally cylindrical base 408 comprising a generally cylindrical recess 409. A generally cylindrical rim 410 surrounds an open upper end of the generally cylindrical recess 409. The rim 410 present a surface which, in this example, is a generally planar ring-shaped surface that contacts and supports the ultrasonic transducer 215.

The base 408 is of a resiliently deformable material. In this example, the base 408 is of silicone, but in other examples the base 408 is of a different resiliently deformable plastic.

In this example, the base 408 comprises a divider wall 411 at a lower part of the generally cylindrical recess 409. The divider wall 411 is provided with a central aperture 412.

The ultrasonic transducer stack 407 comprises a first electrical transducer contact 413. In this example, the first electrical transducer contact 413 comprises a cylindrical lower portion 414 which is coupled to a radial flange 415. An electrical connection 416 is coupled to the upper side of the flange 415 on an opposite side of the flange 415 to the cylindrical lower portion 414. In this example, the electrical connection 416 is springloaded and configured to move relative to the flange 415. In other examples, the electrical connection 416 is fixed relative to the flange 415.

When the first electrical transducer contact 413 is received within the central aperture 412, the flange 415 sits against the divider wall 414 around the periphery of the central aperture 412. The electrical connection 416 extends upwardly above the level of the rim 410. When the ultrasonic transducer 215 is in contact with the rim 410, the electrical connection 416 contacts the first electrical connection on the underside of the ultrasonic transducer 215 and is biased against the first electrical connection of the transducer 215.

In this example, the base 408 comprises a cutaway portion 417 in which a portion of the base 408 beneath the divider 411 on one side of the base 408 is cut away to allow access to the side of the cylindrical lower portion 414 of the first electrical transducer contact 413.

The ultrasonic transducer stack 407 comprises a generally cylindrical metal shell 418 that at least partly surrounds the base 408 when the ultrasonic transducer stack 407 is assembled. The shell 418 comprises a generally cylindrical inner recess 419 that is provided with an opening 420 at one end. The ultrasonic transducer 215 comprises an atomisation surface 215a that is positioned at the opening 420 of the shell 418. In this example, the atomisation surface 215a is planar or generally planar. An inwardly directed generally circular lip 421 surrounds the opening 420. A lower portion of the shell 418 is cut away to correspond with the cutaway portion 417 of the base 408.

When the ultrasonic transducer stack 407 is assembled, the shell 418 sits over the ultrasonic transducer 215 with the lip 421 contacting at least part of the periphery of the ultrasonic transducer 215 to retain the ultrasonic transducer 215 in a position relative to the base and retain the ultrasonic transducer 215 in contact with the rim 410 of the base 408. The rim 421 connects electrically to a second electrical connection 422 that is provided around the periphery of the ultrasonic transducer 215. The shell 418 thus forms a second electrical transducer contact.

Similar to the transducer holder 210 described herein, the resiliently deformable base 408 ensures that the ultrasonic transducer 215 vibrates without the base 408 dampening the vibrations. The height of the base 408 is selected to ensure that the ultrasonic transducer 215 is held tightly between the lip 421 of the shell 418 and the rim 410. This minimises movement of the ultrasonic transducer 215 relative to the electrical connection 416 and the shell 418 that might otherwise disrupt the electrical connection between the electrical connections on the ultrasonic transducer 215 and the electrical connection 416 and the lip 421 of the shell 418. The ultrasonic transducer stack 407 therefore enables optimum vibration of the ultrasonic transducer 215 while maintaining a reliable electrical connection to the ultrasonic transducer 215.

As described below, a first elongate device terminal 423 extends through the mist generator 400 to connect electrically to the cylindrical lower portion 414 of the first electrical transducer contact 413. A second elongate device terminal 424 extends through part of the mist generator 400 to connect electrically to the shell 418. The first and second elongate device terminals 423, 424 receive an AC drive signal from the driver 202 and transmit the AC drive signal to the ultrasonic transducer 215.

The stacked configuration of the transducer stack 407 is such that the ultrasonic transducer stack 407 may be assembled easily, either by hand or using automated machinery.

While in this example, the ultrasonic transducer stack 407 is generally cylindrical, in other examples, the ultrasonic transducer stack 407 may be a different shape, such as oblong or cuboid.

While the ultrasonic transducer stack 407 is described and shown herein in use with the mist generator 400, it is to be appreciated that the ultrasonic transducer stack 407 may be provided in isolation and/or integrated for use with any other type of device that comprises an ultrasonic transducer. Consequently, any device that uses an ultrasonic may benefit from the secure retention of the ultrasonic transducer and reliable electrical connection to the ultrasonic transducer that is provided by the ultrasonic transducer stack 407.

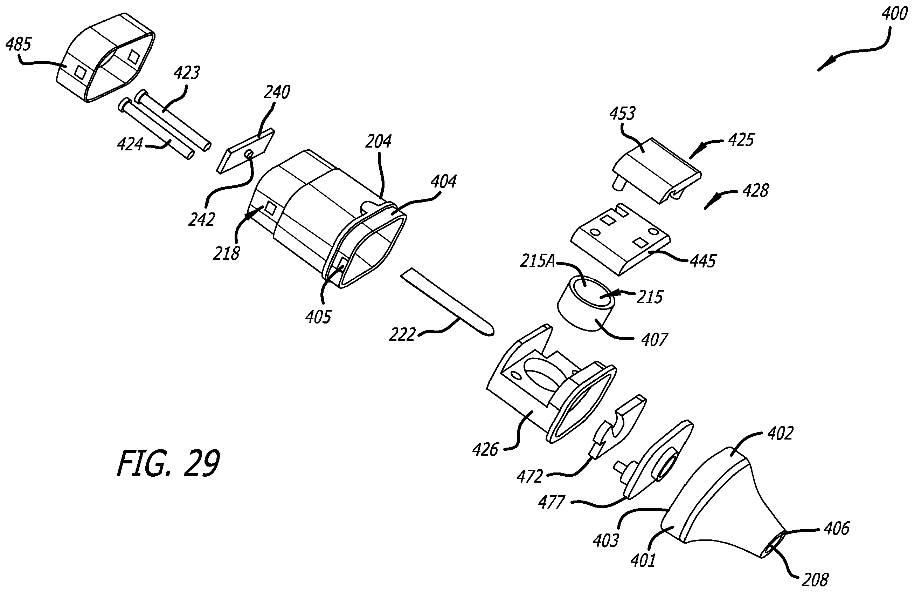

Referring again to figure 29 of the accompanying drawings, the mist generator 400 comprises a sonication assembly 425 which receives the ultrasonic transducer stack 407. The sonication assembly 425 comprises a first assembly portion 426 comprising a recess 427 that receives and retains the ultrasonic transducer 215 within the ultrasonic transducer stack 407.

The sonication assembly 425 comprises a second assembly portion 428 which, when assembled, is coupled to the first assembly portion 426. As described below, the configuration of the first and second assembly portions 426, 428 enables the first and second assembly portions 426, 428 to fit together easily, which facilitates assembly of the mist generator 400. The parts of the sonication assembly 425 may be assembled using automated robots on a production line with minimal human intervention. The mist generator 400 is therefore configured to be mass produced on a production line relatively easily and at low cost compared with conventional mist generators.

At least one of the first assembly portion 426 and the second assembly portion 428 comprises a resiliently deformable section that forms a seal between the first assembly portion 426 and the second assembly portion 428. The seal minimises or prevents a fluid from leaking between the first assembly portion 426 and the second assembly portion 428. The resiliently deformable configuration of the first and second assembly portions 426, 428 avoids the need for a sealant or adhesive to be applied, which reduces the complexity and cost of manufacturing the mist generator 400.

In this example, both the first assembly portion 426 and the second assembly portion 428 are of a resiliently deformable material. In other examples, only one of the first assembly portion 426 and the second assembly portion 428 is of a resiliently deformable material.

In this example, the resiliently deformable material is silicone. In other examples, the resiliently deformable material is a different resiliently deformable plastic material.

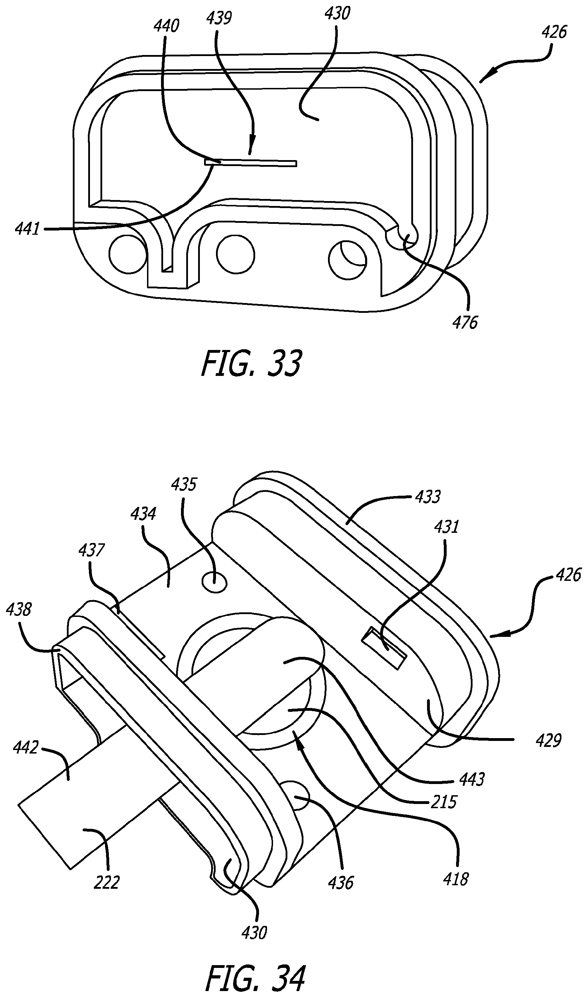

Referring now to Figures 33 and 34 of the accompanying drawings, the first assembly portion 426 comprises an end wall 429 that is spaced apart from a barrier portion 430. The end wall 429 is provided with a mist outlet aperture 431 to enable mist to flow from one side of the upper wall 429 to the other. A recess 432 (visible in figure 29) is provided on the other side of the end wall 429. An outwardly directed flange 433 extends around the periphery of the end wall 429 to contact the housing 204.

The first assembly portion 426 comprises a central portion of the end wall 429 and the barrier portion 430. The central portion 434 is provided with the recess 427 that receives the ultrasonic transducer 215. In this example, the central portion 434 is provided with first alignment retainer apertures 435,436. The central portion 434 comprises an inlet aperture 437 which allows the passage of air from the exterior of the mist generator 400 within the sonication assembly 425.

The first assembly portion 426 retains the ultrasonic transducer 215 in an orientation such that the plane of the atomisation surface 215a of the ultrasonic transducer 215 is aligned with and substantially parallel to a plane extending along a longitudinal length of the housing 204.

The barrier portion 430 comprises a resiliently deformable seal 438 which, in this example, is a protrusion that extends from the barrier portion 430 that is positioned between the sonication assembly 428 and the inner walls of the internal cavity of the housing 204. The barrier portion minimises or prevents fluid leakage from the liquid chamber 218.

The barrier portion 430 comprises a capillary aperture 439. In this example, the capillary aperture 439 is positioned centrally in the barrier portion 430 and is in the form of a generally elongate slot. The capillary aperture 439 is formed between adjacent capillary aperture walls 440, 441. In this example, both the first and second capillary aperture walls 440, 441 are resiliently deformable, but in other examples only one of the capillary aperture walls 440, 441 is resiliently deformable.

A capillary 222 extends through the capillary aperture 439 from the liquid chamber 218 positioned within a lower portion of the housing 204 such that a first portion 442 of the capillary 222 is within the liquid chamber 218. The capillary 222 is of a wicking material, such as bamboo, of the type described herein. A second portion 443 of the capillary 222 is superimposed on the atomisation surface of the ultrasonic transducer 215.

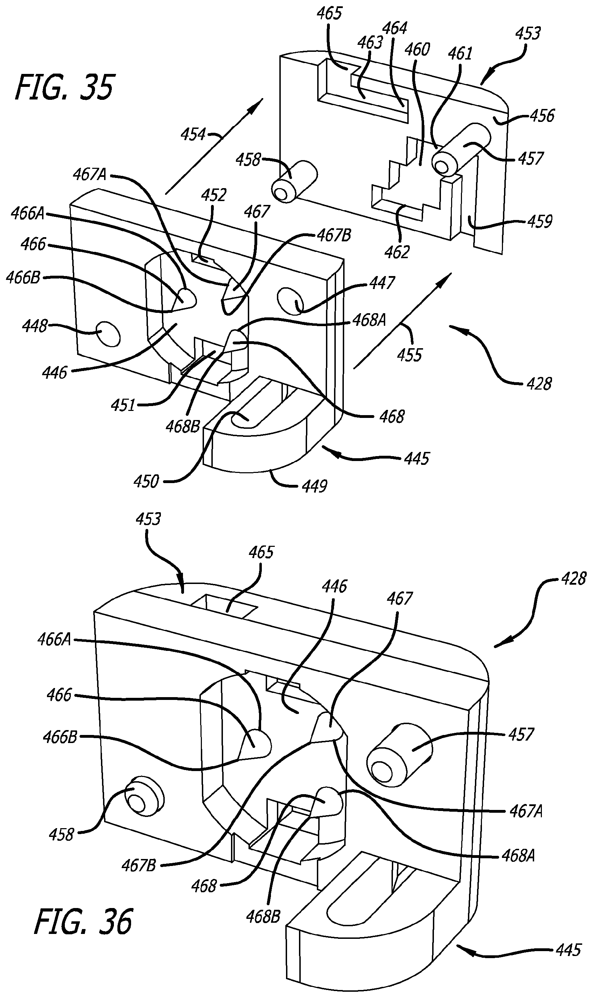

Referring now to Figures 35 and 36 of the accompanying drawings, the second assembly portion 428 comprises a body portion 445 which is provided with a sonication recess 446. When the sonication assembly 425 is assembled, the sonication recess 446 is superimposed on the ultrasonic transducer 215 to form a sonication chamber 219.

The body portion 445 is, in this example, generally rectangular and is provided with apertures 447, 448 that align respectively with the recesses 435, 436 in the first assembly portion 426. The sonication recess 446 is formed centrally in part of the body portion 445.

The body portion 445 comprises a protrusion 449 that extends outwardly from the body portion 445 adjacent sonication recess 446. The protrusion 449 is provided with an elongate channel 450. The channel 450 provides an air inlet port which, when the sonication assembly 425 is assembled enables air to flow along a flow path through a sonication assembly air inlet port 451 to the sonication recess 446.

The body portion 445 comprises a sonication assembly mist outlet port 452 that is in fluid communication with the sonication recess 446. An airflow path extends from the sonication assembly air in that port 451 through the sonication recess 446 to the sonication assembly mist outlet port 452.

The second assembly portion 428 comprises a cover or cover portion 453 which is coupled to the body portion 445 by moving the body portion 445 and the cover portion 453 together as indicated generally by arrows 454 and 455 in Figure 35.

The cover portion 453 is generally rectangular with a cover side 456 that contacts the body portion 445. The cover side 456 is provided with generally cylindrical pegs 457, 458 that protrude outwardly from the cover side 456. The pegs 457, 458 extend through the apertures 447, 448 in the body portion 445 and into the recesses 435, 436 in the first assembly portion 426. The pegs 457, 458 form an interference fit with the apertures 447, 448 and the recesses 435, 436 to maintain the first assembly portion 426 and the second assembly portion 428 in alignment and in connection with one another. The interference fit enables the body portion 445 and the cover portion 453 to be coupled securely to one another without the need for an adhesive or other attachment arrangement. However, in other examples, the pegs 458, 457, apertures 447,448 and recesses 435,436 may be omitted.

The cover portion 453 is provided with an air inlet channel 459 that couples fluidly to the air inlet 450 of the body portion 445. In this example, the air inlet channel 459 is straight or generally straight and extends from the lower edge of the cover portion 453 upwardly. The air inlet channel 459 undergoes a turn, which in this example is a 90 degree turn, towards the middle of the cover portion 453. The air inlet channel 459 couples fluidly with a stepped channel 460 that is formed in the cover portion 453.

In this example, the stepped channel 460 comprises a plurality of turns, which in this example are each a 90° turn. In other examples, there may be a greater or fewer number of turns and the angle of each turn may be an oblique angle. The stepped channel 460 has a first end 416 that is coupled fluidly to the inlet channel 459 and a second end 462 that is coupled fluidly to the sonication assembly air inlet port 451. As will be described below, the stepped channel 460 defines a portion of an airflow path through the device that may be configured to control the distance over which air travels through the mist generator 400. This configuration may be selected to finely tune the flow of air through the mist generator 400 and the volume and tightness of the draw experienced by a user of the device 400. Consequently, the mist generator 400 may be configured to operate in Mouth-To-Lung (MTL) mode. The mist generator 400 therefore mimics a traditional cigarette to improve the experience of the mist generator 400 for a user who is used to smoking traditional cigarettes.

The cover portion 453 is provided with a mist outlet channel 463 that is spaced apart from the stepped channel 460. The mist outlet channel 463 has a first end 464 that is coupled fluidly to the sonication assembly mist outlet port 452 and a second end 465 that is coupled fluidly to the mist outlet port 208 via the mouthpiece 401.

In this example, the second assembly portion 428 comprises three biasing members 466-468 that are provided within the sonication recess 446. In other examples, the second assembly portion 446 comprises only one biasing member. In further examples, the second assembly portion 428 comprises a plurality of biasing members. Each biasing member 466-468 exerts a biasing force on the second portion 443 of the capillary 222 which biases the second portion 443 of the capillary 222 against the atomisation surface 215a of the ultrasonic transducer 215. The biasing force exerted by each biasing member 466-468 helps to optimise the operation of the mist generator 400 by ensuring a tight contact between the capillary 222 and the atomisation surface 215a. Consequently, liquid carried by the capillary 222 is delivered directly on to or proximate to the atomisation surface 215a for atomisation.

In this example, each biasing member 466-468 is formed integrally with the second assembly portion 428. This simplifies the process for manufacturing the mist generator 400 compared with conventional devices that require a separate biasing component to be attached within the mist generator in an additional manufacturing step. A further benefit is that each biasing member 466-468 may be positioned precisely on the second assembly portion 428 to ensure that the capillary 222 is biased at optimal positions against the atomisation surface 215a.

In this example, each biasing member 466-468 comprises an attachment end 466a-468a that is attached to the second assembly portion 428 within the sonication recess 446. Each biasing member 466-468 comprises a distal end 466b-468b which contacts the second portion 443 of the capillary 222. In this example, each distal end 466b-468b is narrower than the attachment end 466a-468a of each respective biasing member 466-468.

In this example, each biasing member 466-468 is conical or substantially conical in shape. In other examples, each biasing member 466-468 may be a different shape, with each distal end 466b-468b preferably being narrower than each respective attachment end 466a-468a.

The narrow distal end 466a-468a minimise the point of contact between the biasing member 466-468 and the capillary 222. This optimises the operation of the mist generator 400 by ensuring that the capillary 222 is held tightly against the atomisation surface 215a without the biasing members 466-468 dampening the vibration of the ultrasonic transducer 215. Nevertheless, while not optimal, other examples comprise biasing members that may have a wider distal end, such as at least one cylindrical biasing member.

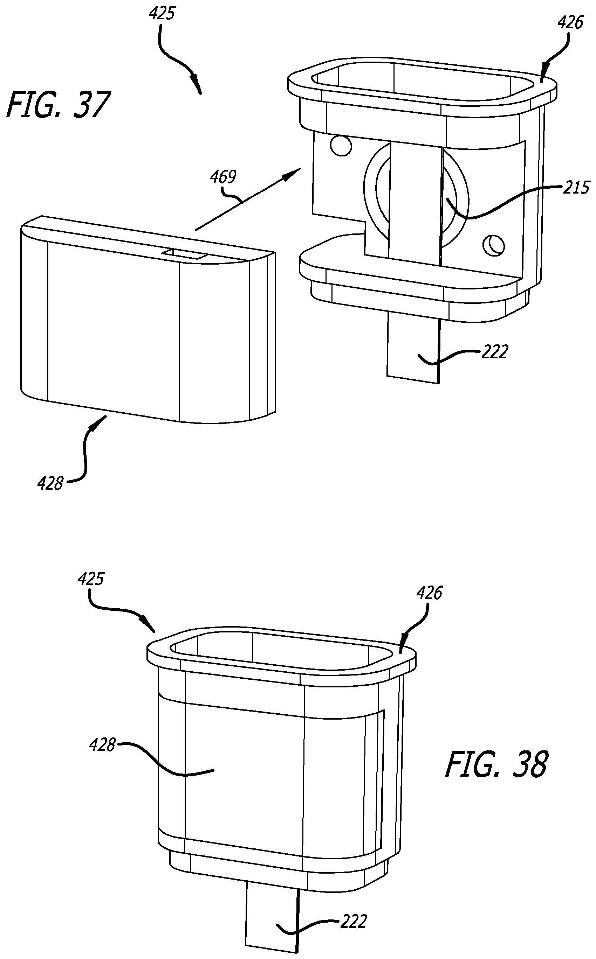

Referring now to Figures 37 and 38 of the accompanying drawings, once the second assembly portion 428 has been assembled, the second assembly portion 428 is coupled with the first assembly portion 426 by moving the first assembly portion 426 and the second assembly portion 428 towards one another in the direction generally indicated by arrow 469 in Figure 37. The body portion 445 of the second assembly portion 428 sits against the central portion 434 of the first assembly portion 426. The pegs 457-458 extend into the recesses 435, 436 in the first assembly portion 426. The first and second assembly portions 426, 428 are held together with an interference fit, as shown in Figure 38. Again, the interference fit simplifies the manufacturing process by avoiding the need to apply an adhesive or sealant, although the present disclosure does not preclude the use of an adhesive or sealant.

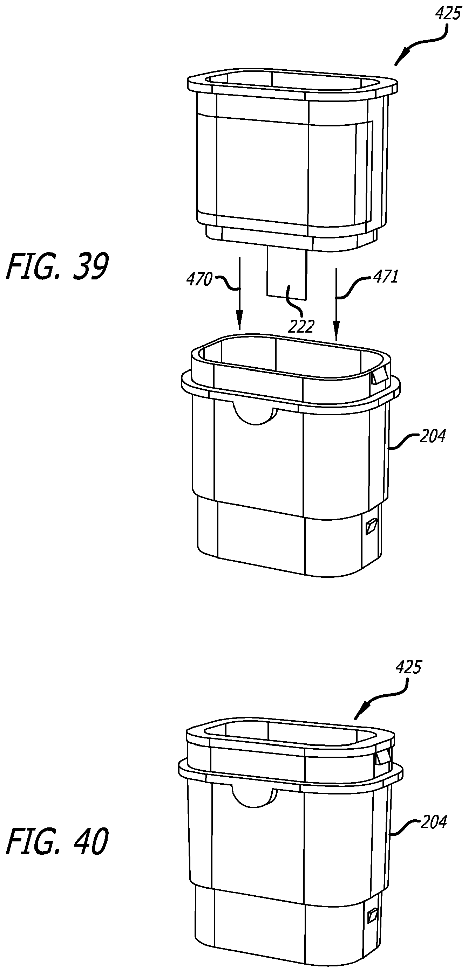

Referring now to Figures 39 and 40 of the accompanying drawings, once the sonication assembly 425 has been assembled, the sonication assembly 425 is inserted at least partly into the internal cavity of the housing 204 in the direction generally indicated by arrows 470, 471 in Figure 39.

The sonication assembly 425 contacts the side walls of the housing 204 and forms a seal which minimises or prevents a fluid flowing between the sonication assembly 425 and the side walls of the housing 204. In this example, the seal is achieved by deformation of the resilient material of the sonication assembly 425 against the walls of the housing 204. In this example, since the seal is achieved by the deformation of the resilient material, no additional sealant or adhesive is required between the sonication assembly 425 and the housing 204. However, the present disclosure does not preclude the use of an adhesive or sealant.

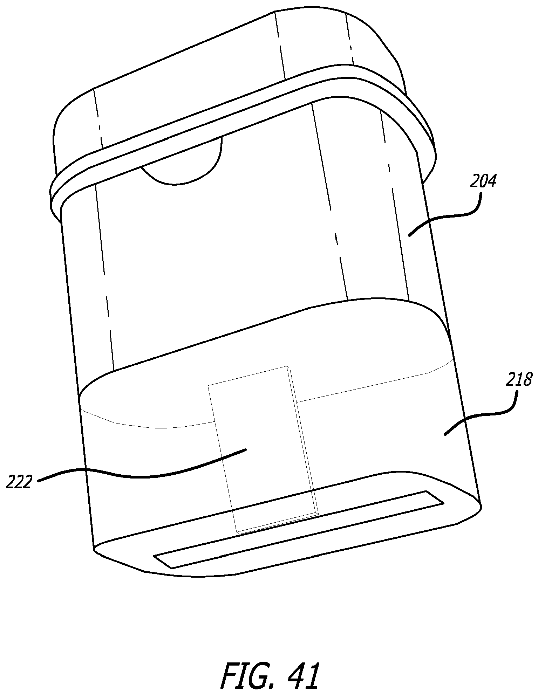

Referring now Figure 41 of the accompanying drawings, the liquid chamber 218 is formed in part of the internal cavity of the housing 204 adjacent to the base of the housing 204. The liquid chamber 218 is positioned between the sonication assembly 425 and the base of the housing 204. The capillary 222 protrudes into the liquid chamber 214 to enable liquid to be carried by capillary action from the liquid chamber 218 via the capillary 222 for aerosolisation by the ultrasonic transducer 215.

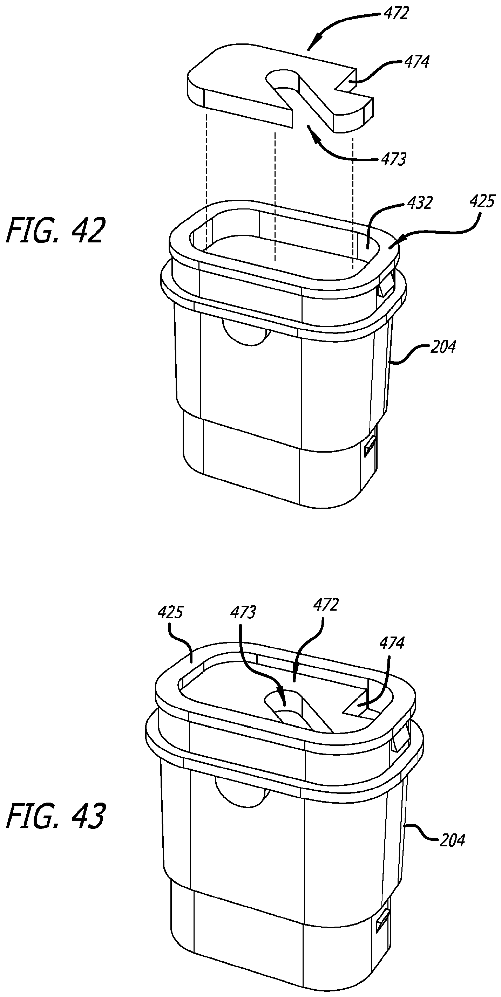

Referring now to Figures 42 and 43 of the accompanying drawings, a foam layer 472 is inserted into the recess 432 of the sonication assembly 425. The foam layer 472 comprises an airflow channel 473 that is cut into one side of the foam layer 472 and is angled from one corner of the foam layer 472 to the centre of the foam layer 472. The foam layer 472 comprises a cutaway portion 474. One end of the airflow channel 473 at the edge foam layer 472 is aligned with the mist outlet aperture 465 of the sonication assembly 425. The airflow channel 473 provides a channel for mist to flow from the mist outlet aperture 465 to the centre of the device 400. In other examples, the airflow channel 473 is omitted and the mist instead passes through the foam layer 472 as it is drawn out from the mist generator 400 by a user.

The foam layer 472 absorbs droplets of liquid that are in excess of a predetermined size from mist flowing out from the mist outlet aperture 465. The foam layer 472 also absorbs any condensed liquid and helps to minimise liquid leaking from the mist outlet port 208 of the mist generator 400. While the foam layer 472 optimises the operation of the mist generator 400 by minimising liquid leaking from the device, in other examples, the foam layer 472 may be omitted.

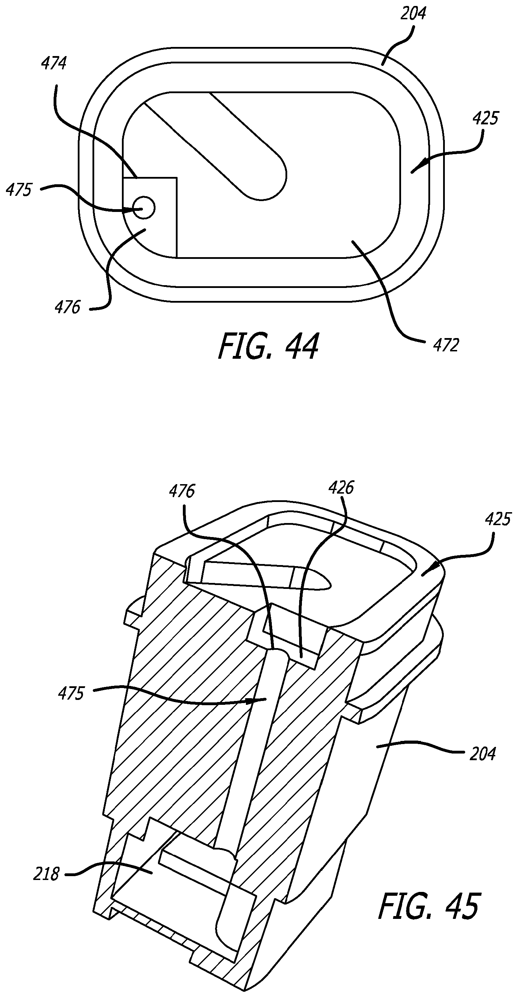

Referring now to Figures 44 and 45 of the accompanying drawings, the sonication assembly 425 comprises a filling bore which extends from a filling aperture 476 through the first assembly portion 426 to the liquid chamber 218 to allow a liquid to be injected (e.g. using a needle) into the liquid chamber 218 through the filling bore 475. The cutaway portion 474 of the foam layer 472 is aligned with the aperture 476 to allow access to the aperture 476 without needing to remove the foam layer 472.

The liquid chamber 218 may alternatively be pre-filled with a liquid before the sonication assembly 425 is inserted within the recess of the housing 204. In this case, the filling bore 475 and the filling aperture 476 allow air to be vented as the sonication assembly 425 is inserted within the recess of the housing 204.

In other examples, the filling bore 475 and the filling aperture 476 may be omitted.

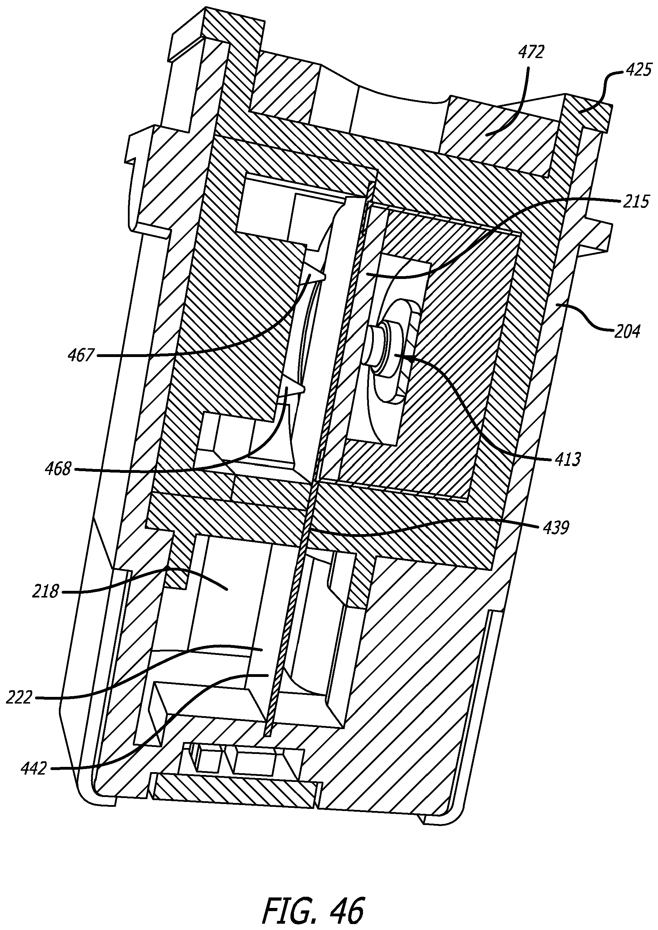

Referring now to Figure 46 of the accompanying drawings, when the sonication assembly 425 is inserted within the recess of the housing 204, the first portion 442 of the capillary 222 is located within the liquid chamber 218. The capillary 222 extends from the first portion 442, through the capillary aperture 439 such that the second end 443 of the capillary 222 is superimposed on the ultrasonic transducer 215. The biasing members 466-468 bias the capillary 222 against the atomisation surface 215a of the ultrasonic transducer 215.

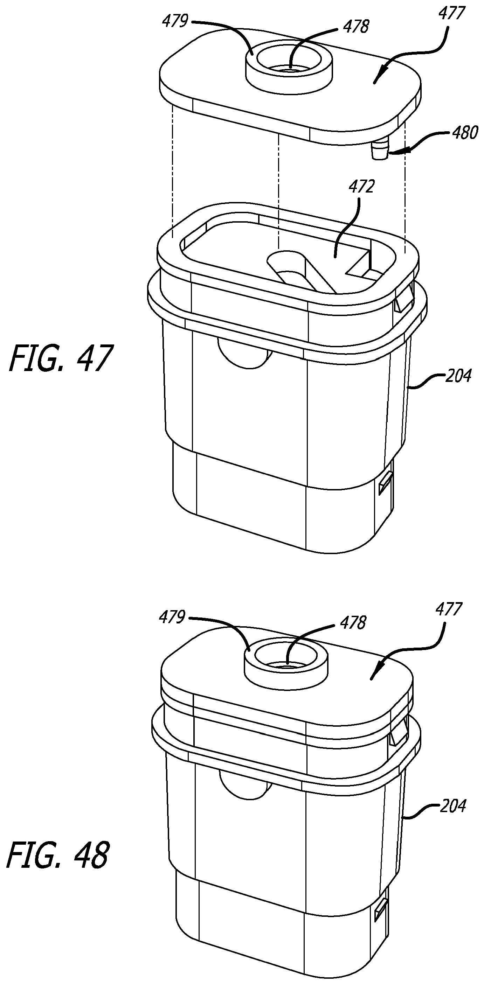

Referring now to Figures 47 and 48 of the accompanying drawings, once the sonication assembly 425 has been inserted into the recess in the housing 204 and the foam layer 472 has been inserted into the recess 32 of the sonication assembly 425, a divider 477 is superimposed over the foam layer 472. In this example, the divider 477 is generally planar and of a resilient deformable material, such as silicone. The divider 477 comprises a central aperture 478 that is surrounded by an upwardly protruding cylindrical wall 479.

On the opposite side of the divider 477 to the cylindrical wall 479, there is a plug 480 that is elongate and generally cylindrical. The plug 480 at least partly fits within the aperture 476 to seal the aperture 476 and prevent liquid from leaking from the liquid chamber 418 via the filling bore 475.

Since the divider 477 is resiliently deformable, the divider 477 forms a seal around the upper periphery of the sonication assembly 475 to minimise or prevent liquid from leaking between the divider 477 and the sonication assembly 425.

The mist generator 400 minimises or eliminates the possibility of any liquid leaking out from within the mist generator 400. This is critical for consistency of dosage delivery. A leaking device may have less liquid (containing therapeutic, drug, etc.) in the liquid chamber 218 than what would be prescribed for treatment of a condition.

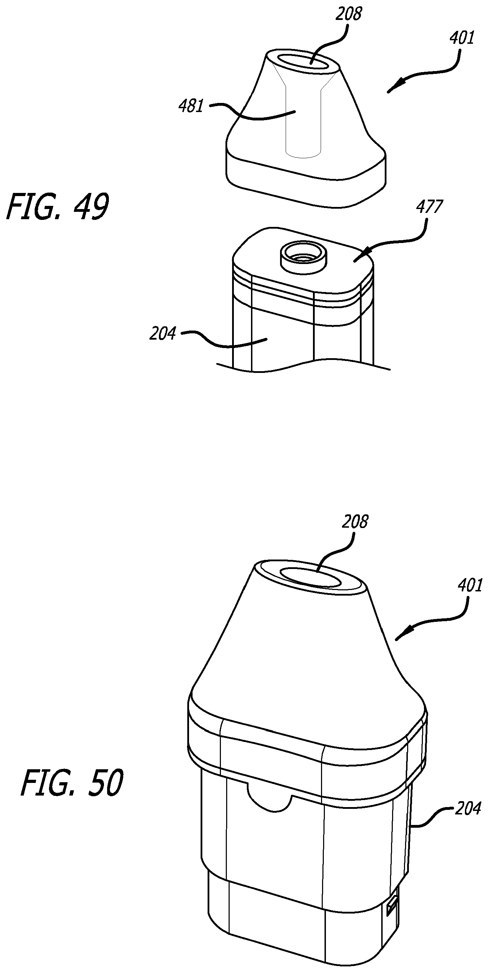

Referring now to Figures 49 and 50 of the accompanying drawings, once the divider 477 is in place, the mouthpiece 401 is attached to the housing 204 to substantially close the internal cavity of the housing 204. The mouthpiece 401 comprises an inner tube 481. One end of the inner tube 481 couples fluidly with the central aperture 478 of the divider 477 and the other end of the inner tube 481 couples fluidly with the mist outlet aperture 208. The mouthpiece 401 provides a mist flow path from the sonication assembly 425 to the mist outlet aperture 208.

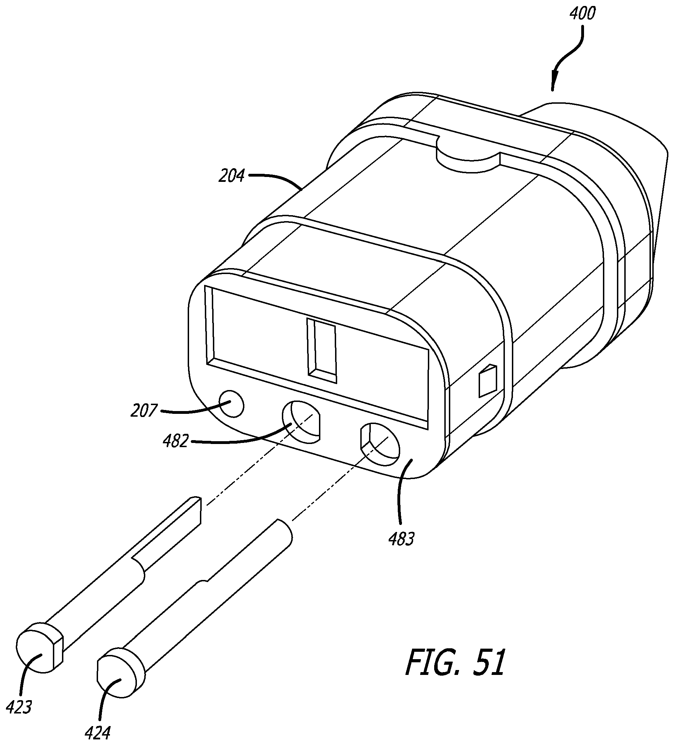

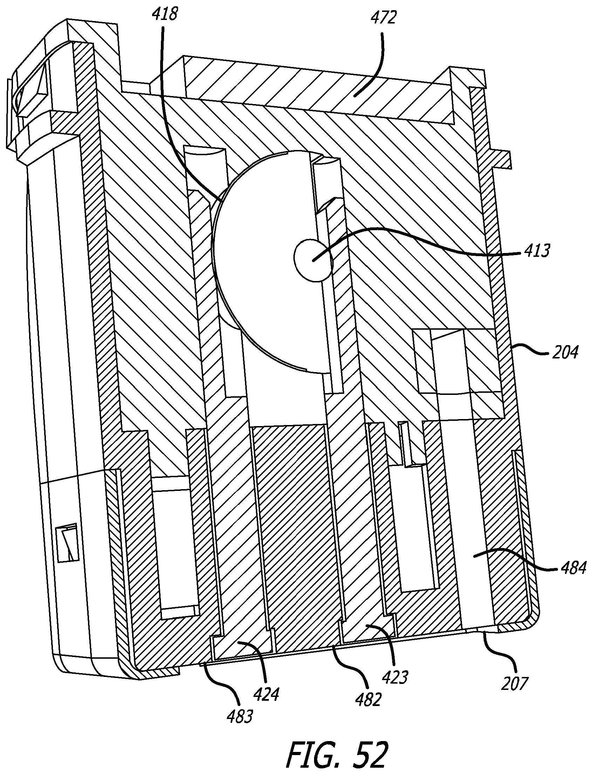

Referring now to Figures 51 and 52 of the accompanying drawings, the first and second elongate device terminals 423, 424 are inserted through respective terminal apertures 482, 483 in the base of the housing 204. The first elongate device terminal 423 extends through the mist generator 400 to connect electrically to the first electrical transducer contact 413. The second elongate device terminal 424 extends through part of the mist generator 400 to connect electrically to the shell 418. The configuration of the ultrasonic transducer stack 407 and the resiliently deformable material of the sonication assembly 425 ensures that no liquid can seep between the shell 418 and the sonication assembly 425. The first and second elongate device terminals 423, 424 are therefore isolated from the liquid within the mist generator 400 that might otherwise disrupt the AC drive signal delivered to the ultrasonic transducer 215 and cause the mist generator 400 to malfunction.

The first and second elongate device terminals 423, 424 provide electrical connections from the ends of the elongate device terminals 423, 424 that are accessible at the base of the housing 204 to the terminals of the ultrasonic transducer 215. An AC drive signal generated by the driver 202 may therefore be transmitted to the ultrasonic transducer 215 via the elongate device terminals 423, 424.

Also visible in Figures 51 and 52 is an air inlet aperture 207 that is provided in the base of the housing 204. The air inlet aperture 207 is coupled fluidly to an air channel 484 that extends through the mist generator 400 to the sonication assembly air inlet port 451. As described above, air flows into the mist generator 400 via the air inlet port 207 which, as described below, is in fluid communication with the air flow bridge within the driver 202.

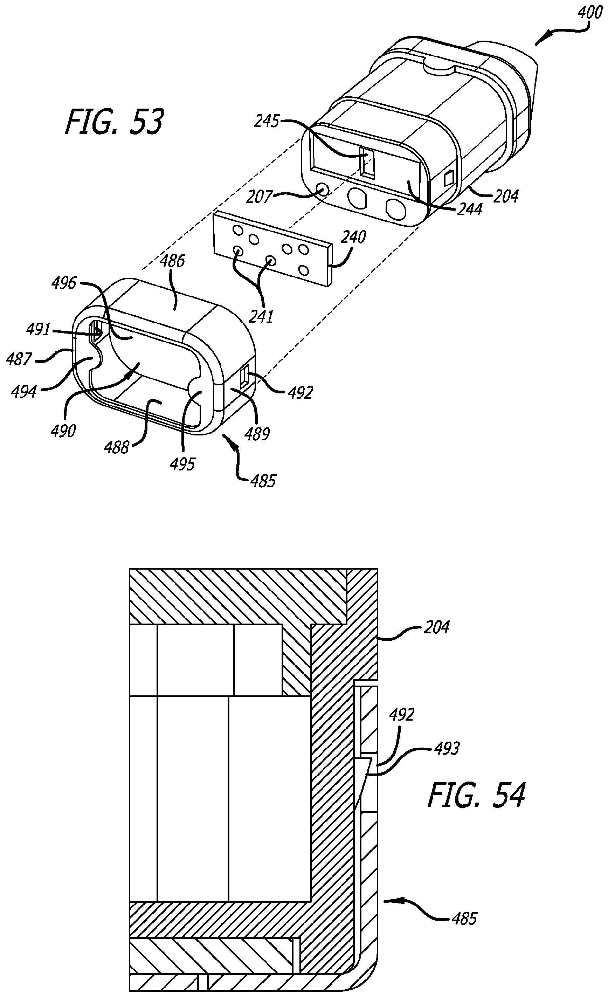

Referring now to Figures 53 and 54 of the accompanying drawings, the mist generator 400 comprises an end cap 485 that attaches to the lower end of the housing 204. The end cap 485 comprises four walls that define a recess 490 that receives an end portion of the housing 204. Opposing side walls 487, 489 each comprise a respective retainer slot 491, 492. Each retainer slot 491, 491 receives a respective outwardly directed chamfer 493 (only one of which is visible in Figures 53 and 54). Each chamfer 493 retains the end cap 485 attached to the housing 204.

The end cap 485 comprises two inwardly directed tabs 494, 495 at the base of the end cap 485. The tabs 494, 495 are superimposed on respective ends of the printed circuit board 240 when the end cap 485 is attached to the housing 204.

The printed circuit board 240 carries the OTP IC 242. The tabs 494, 495 retain the printed circuit board 240 in position within the recess 244 on the housing 204.

The end cap 484 comprises an aperture 496 to allow access to the electrical contact 241 on the printed circuit board 240 and access to the elongate device terminals 423, 424. the aperture 496 also allows air to flow into the air inlet aperture 207 on the housing 204.

In this example, the end cap 485 is of metal and the end 485 enables the mist generator 400 to be retained in the recess 203 of the driver 202 by a magnetic force through attraction to a magnet provided on the driver 202. However, in other examples, the end cap 485 may be of a different material or may be omitted entirely.

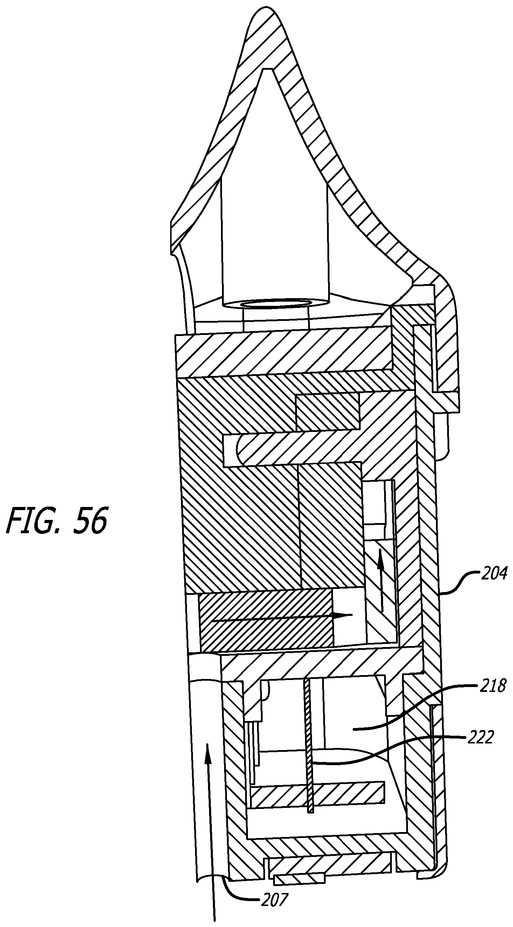

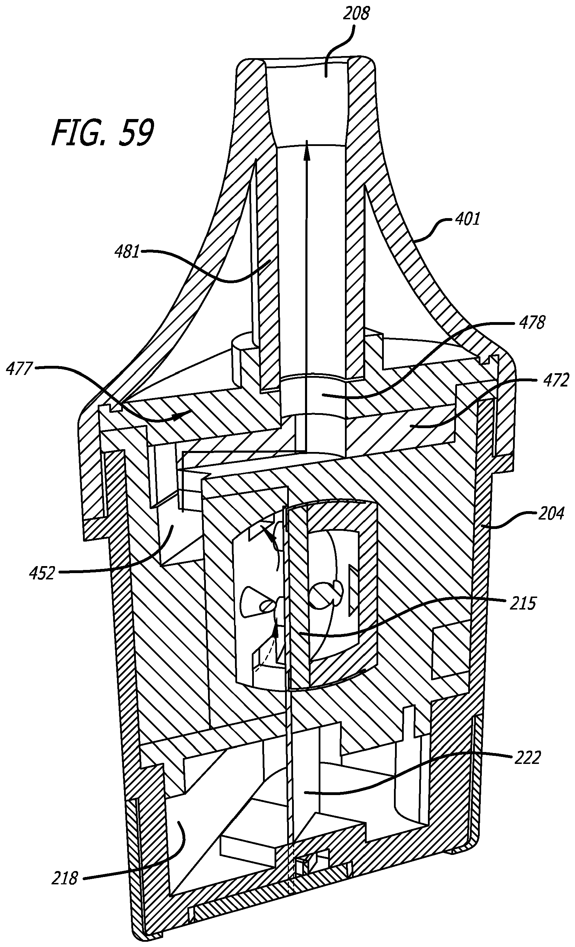

Referring now to Figures 56-59 of the accompanying drawings, when the mist generator 400 is in use and is connected to the driver 202, an airflow path exists from the air inlet port 207 at the base of the mist generator 400, through the mist generator 400 to the mist outlet port 208. Air flows through the air inlet port 207, through the air channel 484, through the elongate channel 450 to the sonication assembly air inlet port 451.

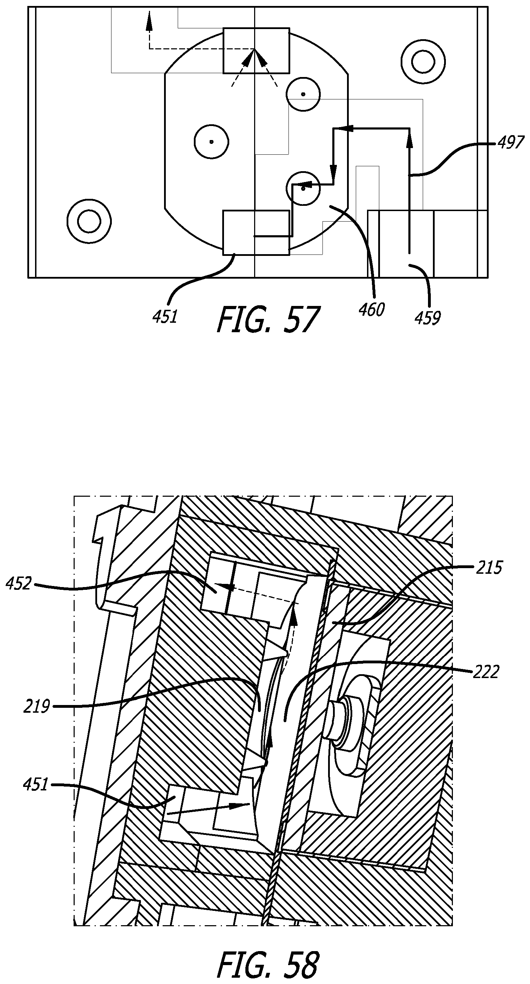

As shown in Figure 57, an air flow path 497 comprises a plurality of turns as the air flows from the air inlet channel 459, through the stepped channel 460 to the sonication assembly air inlet 451. The air then enters the sonication chamber via the sonication assembly air inlet port 451, as shown in Figure 58. The air is directed against the capillary 222 and flows along part of the length of the capillary 222 as the air flows through the sonication chamber 219. This optimises the operation of the mist generator 400 by ensuring that the airflow is directed against part of the capillary 222.

The ultrasonic transducer 215 vibrates and atomises an aerosolises liquid from the capillary 222 within the sonication chamber 219. This creates a mist within the sonication chamber 219 which is drawn out from the sonication chamber 219 via the sonication assembly mist outlet port 452. The operation of the mist generator 400 is optimised by ensuring that all or substantially all of the generated mist is evacuated from the sonication chamber 219 as the airflow path travels along part of the length of the capillary 222 before being drawn out from the sonication chamber 219.

The mist flows out from the mist outlet aperture 465, through the air flow channel 473 provided in the foam layer 472 to the central aperture 478 in the divider 477. The mist then passes through the inner tube 481 of the mouthpiece 401 to the mist outlet port 208 for inhalation by a user.

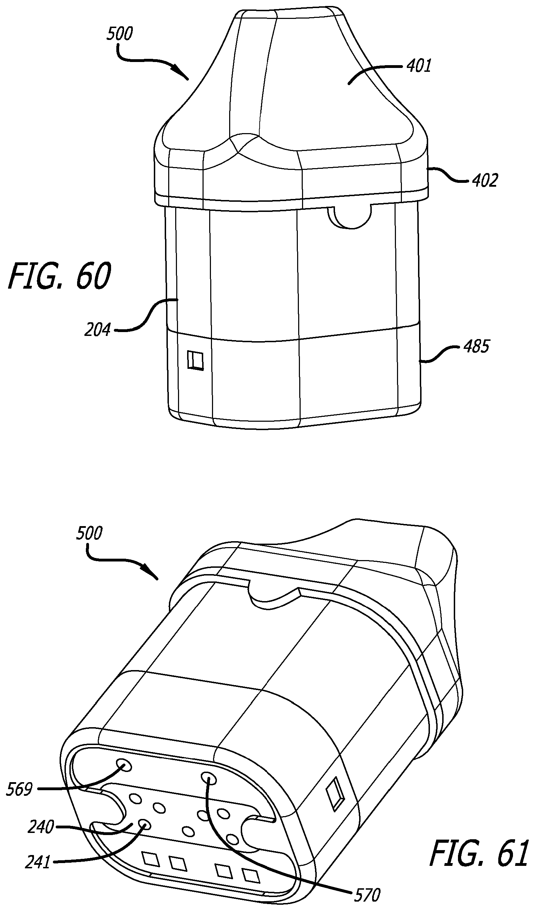

Referring now to Figures 60 and 61 of the accompanying drawings, a mist generator 500 of some examples of this disclosure comprises many of the same elements as the mist generator 400 described herein, and so these same components may not be described again. The same reference numbers may be used for equivalent elements of the mist generator 500 as the mist generator 400.

The mist generator 500 is configured to be releasably attached to the driver 202 for operation in substantially the same way as the mist generator 400 described herein. In other examples, the mist generator 500 may be fixed to, formed integrally with or otherwise non-releasably attached to the driver 202.

The mist generator 500 comprises a housing 204 comprising two liquid chambers 546, 547 for containing a liquid to be atomised. The liquid chambers 546, 547 may contain any e-liquid or liquid described in the present disclosure or any other liquid which is to be atomised. The liquid in the two liquid chambers 546, 547 may be the same. Alternatively, the liquid in the first liquid chamber 546 may be different to that in the second liquid chamber 547. The liquid in the first liquid chamber 546 preferably includes a therapeutic.

The mist generator 500 comprises a mouthpiece 401 that is coupled to the housing 204. The mouthpiece 401 comprises a base 402 having an opening which receives a connector portion of the housing 204 in the same way as the mist generator 400 described herein.

The mouthpiece 401 narrows progressively, and the narrow end comprises the mist outlet port 208 to enable the mist output from the mist generator 500 for inhalation by a user.

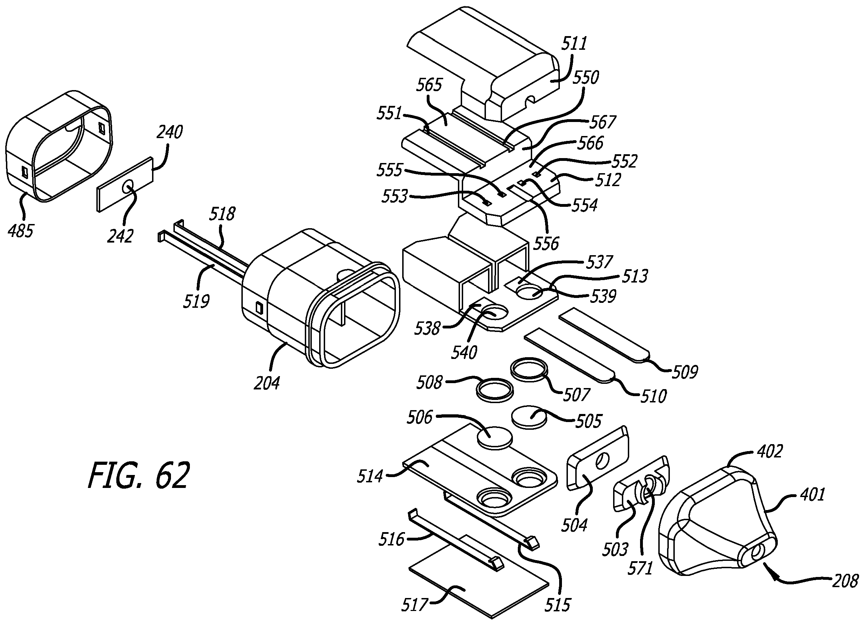

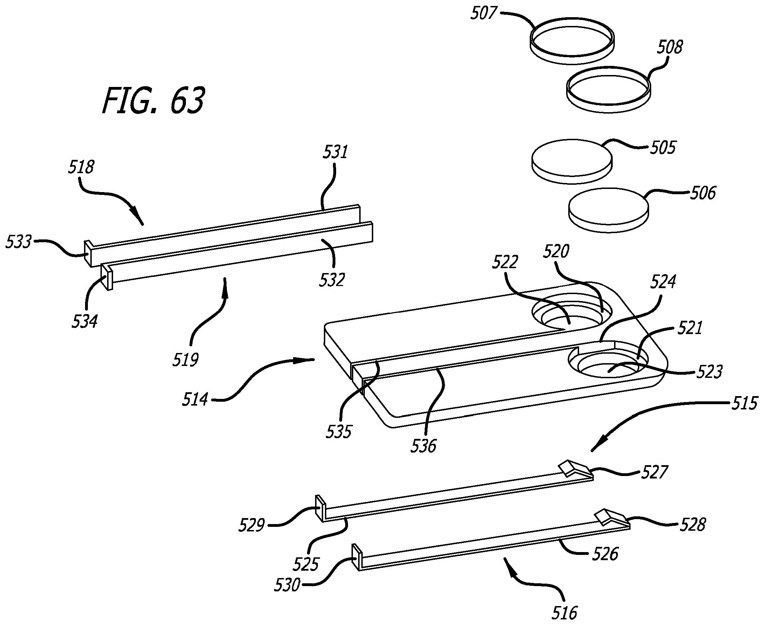

Referring now to figures 62 and 63 of the accompanying drawings, the mist generator 500 comprises two ultrasonic transducers 505, 506. In some examples, the ultrasonic transducers 505, 506 are solid (non-porous) disc ultrasonic transducers. In this example, the ultrasonic transducers 505, 506 are received within a transducer holder 514, which may alternatively be known as an ultrasonic transducer assembly. The transducer holder 514 is preferably planar or generally planar, and comprises two recesses 520, 521 that are provided in a holder surface for accepting the ultrasonic transducers 505, 506. The recesses 520, 521 of this example are of a slightly larger diameter than the diameter of the ultrasonic transducers 505, 506 to facilitate the fitting of annular electrical connections 507, 508. The annular electrical connections 507, 508 are thus configured to be positioned radially between each ultrasonic transducer 505, 506 and its respective recess 520, 521. While the transducer holder 514 of this example is planar, in other examples the transducer holder 514 is not planar but still comprising a planar or generally planar holder surface.

The first recess 520 retains the first ultrasonic transducer 505 with the first atomisation surface generally in a plane with the holder surface. The second recess 521 retains the second ultrasonic transducer 506 with the second atomisation surface generally in the plane with the holder surface.

The transducer holder 514 may feature two channels 535, 536. Each channel 535, 536 may extend tangentially from a respective one of the recesses 520, 521 to an end of the transducer holder 514. Each channel 535, 536 is sized to accommodate a first and second electrical contact 518, 519 which may extend from the electrical connection 507, 508 to the longitudinal end of the mist generator 500 once assembled. The flow of electricity between the driver 202 and the ultrasonic transducers 505, 506 is therefore enabled.

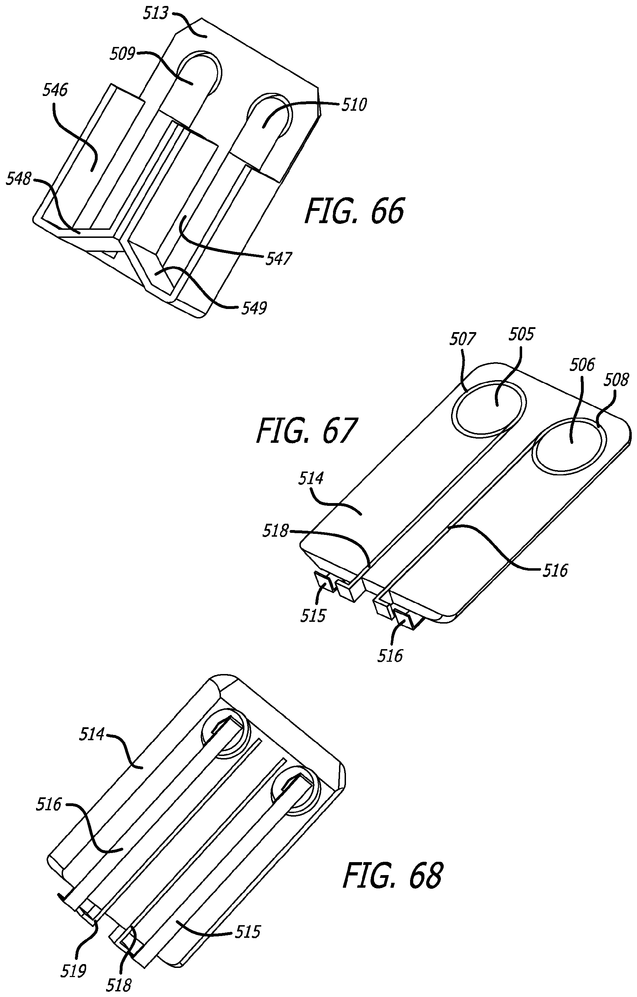

The recesses 520, 521 may comprise respective through holes 522, 523. The through holes 522, 523 are of a smaller diameter than the recesses 520, 521 and the ultrasonic transducers 505, 506, and so the ultrasonic transducers 505, 506 are supported annularly on their undersides. The through holes 522, 523 provide convenient locations for rear mounted third and fourth electrical contacts 515, 516. The rear mounted electrical contacts 515, 516 are, similarly to the first and second electrical contacts 518, 519, elongate so as to extend from the ultrasonic transducers 505, 506 to the end of the mist generator 500 once assembled. All four electrical contacts 518, 519, 515, 516 may terminate at the same axial point, the axial point being planar to a surface of the mist generator opposite the mouthpiece 401. The electrical contacts 518, 519, 515, 516 can be seen on the assembled mist generator 500 in figure 67. As shown in figure 63, the electrical contacts 518, 519, 515, 516 may have ends 533, 534, 529, 530 folded at approximately 90 degrees to allow a more stable electrical connection to the driver 202. It will of course be appreciated that other electrical connection methods may be employed.

The rear electrical contacts have folded ends 527, 528 that are configured to protrude through the through holes 522, 523 and into contact with the rear planar surface of the ultrasonic transducers 505, 506. The folded ends 527, 528 are geometrically sized so that once assembled, the ends 527, 528 are forced to bend further and towards the elongate portions 525, 526 of the rear electrical contacts 515, 516. At least the third and fourth electrical contacts 515, 516 are made of a material which both conducts electricity and can elastically deform, thereby causing the folded ends 527, 528 to be spring loaded against the rear surface of the ultrasonic transducers 505, 506 and ensuring suitable electrical engagement even when the ultrasonic transducers 505, 506 are vibrating.

The transducer holder 514 may be of a resiliently deformable material. In this example, the holder 514 is of silicone, but in other examples the holder 514 is of a different resiliently deformable plastic.

Similar to the base 408 described herein, the transducer holder 514 ensures that the ultrasonic transducers 505, 506 vibrate without the transducer holder 514 dampening the vibrations.

While in this example, the transducer holder 514 is generally rectangular, in other examples, the ultrasonic transducer holder 514 may be a different shape.

Referring again to figure 62 of the accompanying drawings, the mist generator 500 comprises a sonication assembly which receives the ultrasonic transducer holder 514. The sonication assembly comprises a first assembly portion 511; a second assembly portion 512; a third assembly portion 513; the transducer holder 514; and a fourth assembly portion 517, the transducer holder 514 being coupled to the third assembly portion 513 and the fourth assembly portion 517.

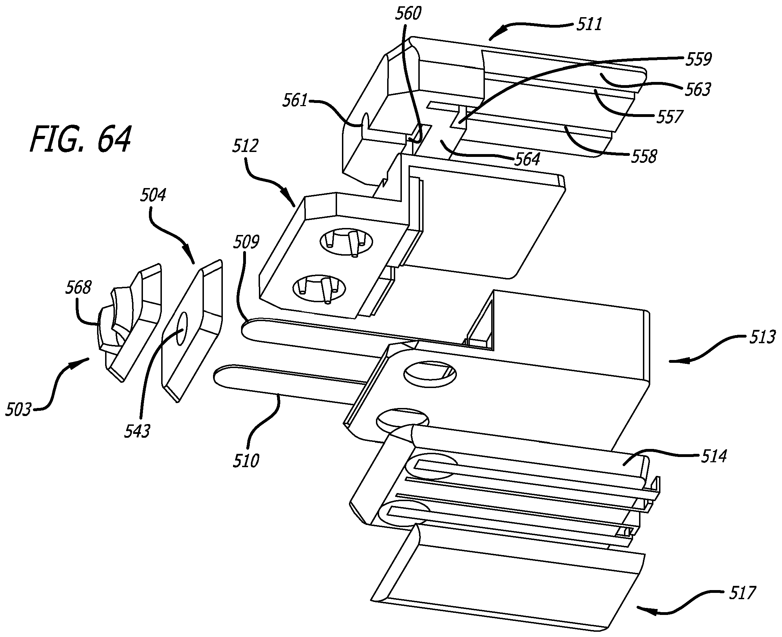

The first assembly portion 511 may comprise a planar or generally planar first surface 563 which preferably extends at least the majority of the length of the sonication assembly. The first assembly portion may also comprise a planar or generally planar second surface 564. The second surface 564 is approximately parallel to, but offset from, the first surface 563. The first assembly portion 511 therefore conforms to the stepped geometry of the second assembly portion 512. The first assembly portion 511 comprises two air inlet channels 557, 558. The air inlet channels 557, 558 may be of any cross-sectional shape. The air inlet channels 557, 558 extend the full length of the first surface 563 and divert approximately 90 degrees toward the second surface 564 of the first assembly portion 511. The air inlet channels 557, 558 then form a path across the second surface 564 to terminate at sonication chamber air inlets 552, 553 (most clearly seen in figure 64) of the second assembly portion 512.

It will be appreciated that whilst the above described the figures, other air inlet channel 557, 558 paths may be implemented. The path length of the air inlet channels 557, 558 define a portion of an air flow path through the device. This configuration may be selected to finely tune the flow of gas through the mist generator 500 and the volume and tightness of the draw experienced by a user of the device 500.

The first assembly portion 511 further comprises a mist outlet hole 561 configure to align to the mist outlet port 208 once the mist generator 500 is assembled, and a mist outlet channel 560 to connect the sonication chamber mist outlets 554, 555 of the second assembly portion 512 to the mist outlet hole 561.

The second assembly portion 512 comprises a lower section 565, an upper section 566, and a middle section 567. The upper section 566 and lower section 566 may lie approximately parallel to one another but be spaced apart in a direction normal to their planar surfaces. The middle section 567 connects the upper and lower sections 565, 566.

The lower section 565 of the second assembly portion 512 comprises channels 550, 551 sized and positioned complimentary to the air inlet channels 557, 558 of the first assembly portion 511 such that when the first assembly portion 511 and the second assembly portion 512 are coupled to one another, the channels 550, 551, 557, 558 form a complete inlet duct from the exterior of the mist delivery device 500 to the sonication chamber. Similarly, the upper section 566 of the second assembly portion 512 features a groove 556 complimentary to the position and size of the mist outlet hole 561 in the first assembly portion 511 so as to provide a conduit from the mist outlets 554, 555 toward the mouthpiece 401.

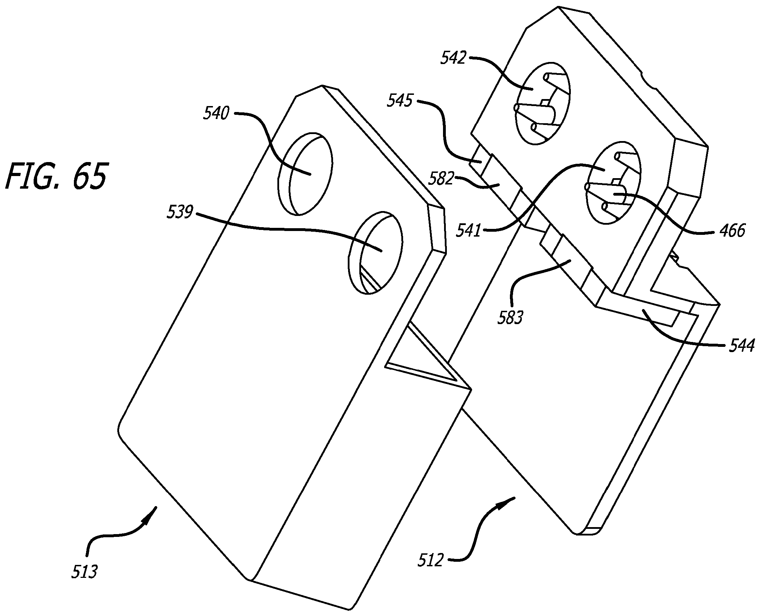

Referring to figure 65, the opposing side of the second assembly portion 512 may comprise, on the upper portion 566, sonication recesses 541, 542. The sonication recesses 541, 542 are fluidly coupled to both the sonication chamber air inlets 552, 553, and the sonication chamber mist outlets 554, 555. The generally circular surface of the sonication recesses 541, 542 may comprise a plurality of biasing members 466. The biasing members 466 are the same as those described with reference to the mist generator 400 and are configured to serve the same purpose of biasing capillaries 509, 510 against at least a portion of the atomisation surface of the ultrasonic transducers 505, 506.

In this example, the second assembly portion 512 comprises three biasing members 466 in each sonication recess 541, 542. In other examples, the second assembly portion 512 comprises only one biasing member 466 in each sonication recess 541, 542. In further examples, the second assembly portion 512 comprises a plurality of biasing members 466. Each biasing member 466 exerts a biasing force on the second portion of the capillaries 509, 510 which biases the second portion of each capillary 509, 510 against at least a portion of the atomisation surface of a respective ultrasonic transducer 505, 506. The biasing force exerted by each biasing member 466 optimises the operation of the mist generator 500 by ensuring a tight contact between the capillary 509, 510 and the atomisation surface. Consequently, liquid carried by each capillary 509, 510 is delivered directly on to or proximate to the atomisation surface for atomisation.

At least part of the first capillary 509 is generally parallel to a plane of the holder surface and extends along the holder surface from the first liquid chamber 546 to the first ultrasonic transducer 505. One part of the first capillary 509 is superimposed on the holder surface and another part of the first capillary 509 is in contact with at least a portion of the first atomisation surface so that the first capillary 509 can transport the first liquid by capillary action from the first liquid chamber 546 to the first atomisation surface.

At least part of the second capillary 510 is generally parallel to a plane of the holder surface and extends along the holder surface from the second liquid chamber 547 to the second ultrasonic transducer 506. One part of the second capillary 510 is superimposed on the holder surface and another part of the second capillary 510 is in contact with at least a portion of the second atomisation surface so that the second capillary 510 can transport the second liquid by capillary action from the second liquid chamber 547 to the second atomisation surface.

The first ultrasonic transducer 505 is configured to generate and transmit ultrasonic waves to generate a first portion of the mist from the first liquid at the interface between the first capillary 509 and the first ultrasonic transducer 505. The second ultrasonic transducer 506 is configured to generate and transmit ultrasonic waves to generate a second portion of the mist from the second liquid at the interface between the second capillary 510 and the second ultrasonic transducer 506. The first portion of the mist and the second portion of the mist combine and flow out through the mist outlet port 208 to the user when the user draws on the mouthpiece.

The flat or substantially flat configuration of the capillaries 509, 510 in the examples described herein is advantageous since it facilitates the delivery of liquid from the liquid chambers 546, 547 to the ultrasonic transducers 505, 506. The flat or substantially flat configuration of the capillaries 509, 510 provides a linear or substantially linear path generally within a plane to facilitate the transportation of liquid by capillary action along the capillaries 509, 510. This ensures continuous and consistent delivery of liquid to the ultrasonic transducers 505, 506 which permits accurate dosing of a therapeutic to a user.

The flat or substantially flat configuration of the capillaries 509, 510 also allows liquid to be transported by the multiple capillaries 509, 510 without restriction at the same rate as one another. All the capillaries 509, 510 can therefore deliver liquid to the ultrasonic transducers 505, 506 consistently to one another.

In this example, each biasing member 466 is formed integrally with the second assembly portion 512. This simplifies the process for manufacturing and assembly of the mist generator 500 compared with conventional devices that require a separate biasing component to be attached within the mist generator in an additional manufacturing step. A further benefit is that each biasing member 466 may be positioned precisely on the second assembly portion 512 to ensure that the capillaries 509, 510 are biased at optimal positions against the ultrasonic transducers 505, 506.

The middle section 567 of the second assembly portion 512 comprises two extrusions 544, 545 of a size and shape complimentary to the size and shape of the openings of the liquid chambers 546, 547. The extrusions 544, 545 are configured to be inserted into and substantially seal the two liquid chambers 546, 547 when the mist generator 500 is assembled. A surface of each of the extrusions 544, 545 may feature a slot 582, 583 to allow the capillaries 509, 510 to extend into the liquid chambers 546, 547. The slots 582, 583 are sized such that the extrusions 544, 545 and capillaries 509, 510 serve to seal the liquid chambers 546, 547 to prevent or at least minimise fluid leakage.

The two liquid chambers 546, 547 of the third assembly portion 513, most clearly seen in figure 66, may each comprise at least a partially angled lower surface 548, 549. Such an angled lower surface means that when the liquid level is very low, the surface area contact between the liquid and the capillaries 509, 510 is maximised so as to provide the same experience to the user.

The third assembly portion 513 comprises through holes 539, 540 such that when the second assembly portion 512, third assembly portion 513, and transducer holder 514 are coupled to one another, two cylindrical sonication chambers are formed.

The third assembly portion 513 may further comprise two depressions 537, 538. The depressions 537, 538 may feature three closed sides and one open side, the open side being the through holes 539, 540. The depressions may extend away from the through holes 539, 540 as far as or slightly into the liquid chambers 546, 547. Once assembled, a first portion of the capillaries 509, 510 extends into the liquid chambers 546, 547, and a second portion is held within the through holes 539, 540 by the biasing elements 466 so as to be superimposed on at least a portion of the atomisation surfaces of the ultrasonic transducers 505, 506. The extrusions 544, 545 seal the liquid chambers 546, 547, and the depressions 537, 538 allow the capillaries 509, 510 to pass from the liquid chambers 546, 547 to the ultrasonic transducers 505, 506 without liquid leaking from the liquid chambers 546, 547. In examples including the slots 582, 583 in the second assembly portion 512, the depressions 537, 538 and the slots 582, 583 may overlap once the mist generator device 500 is assembled to allow a path for the capillaries 509, 510 to extend from the through holes 539, 540 into the liquid chambers 546, 547. The depressions 537, 538 further serve to ensure that the capillaries 509, 510 lie flat on the ultrasonic transducers 505, 506 to enable efficient mist production. In some examples, the depressions 537, 538 are not present and the extrusions 544, 545 are sized and/or shaped to allow the capillaries 509, 510 to extend from within the liquid chambers 546, 547 to the through holes 539, 540.

The fourth assembly portion 517 may be a generally planar sheet that serves to encase the third and fourth electrical contacts 515, 516. During assembly, the fourth assembly portion 517 is coupled to the transducer holder 514.

As described above, the configuration of the first assembly portion 511, the second assembly portion 512, the third assembly portion 513, the transducer holder 514, and the fourth assembly portion 517 enables the sonication assembly to fit together easily, which facilitates assembly of the mist generator 500. The parts of the sonication assembly may be assembled using automated robots on a production line with minimal human intervention. The mist generator 500 is therefore configured to be mass produced on a production line relatively easily and at low cost compared with conventional mist generators.

At least one of the first assembly portion 511, the second assembly portion 512, the third assembly portion 513, the transducer holder 514, and the fourth assembly portion 517 comprises at least a resiliently deformable section that forms a seal between the neighbouring parts of the sonication assembly. The seal minimises or prevents a fluid from leaking between components of the sonication assembly. The resiliently deformable configuration of the sonication assembly avoids the need for a sealant or adhesive to be applied, which reduces the complexity and cost of manufacturing the mist generator 500.

In this example, all of the first assembly portion 511, the second assembly portion 512, the third assembly portion 513, the transducer holder 514, and the fourth assembly portion 517 are of a resiliently deformable material. In other examples, only one of the first assembly portion 511, the second assembly portion 512, the third assembly portion 513, the transducer holder 514, and the fourth assembly portion is of a resiliently deformable material.

In this example, the resiliently deformable material is silicone. In other examples, the resiliently deformable material is a different resiliently deformable plastic material.

In this example, the ultrasonic transducers 505, 506 are oriented such that their atomisation surfaces are substantially parallel to the major axis of the mist generator 500.

The mist generator 500 may further comprise a foam layer 504. The foam layer 504 comprises a hole 543. The hole 543 is aligned with the mist outlet conduit of the sonication assembly once the mist generator 500 is assembled. The hole 543 provides a path for mist to flow from the conduit, formed by the channel 561 and the groove 556, to the mouthpiece 401. In other examples, the hole 543 is omitted and the mist instead passes through the foam layer 504 as it is drawn out from the mist generator 500 by a user.

The foam layer 504 absorbs droplets of liquid that are in excess of a predetermined size from the mist flowing from the conduit. The foam layer 472 also absorbs any condensed liquid and helps to minimise liquid leaking from the mist outlet port 208 of the mist generator 500. While the foam layer 504 optimises the operation of the mist generator 500 by minimising liquid leaking from the device, in other examples, the foam layer 504 may be omitted.

The mist generator further comprises a divider 503 in some examples. The divider 503 is preferably of a resiliently deformable material, such as silicone. The divider 503 has a generally planar base, and is sized to cover the surface of the foam layer 504 furthest from the sonication assembly. The divider 503 comprises an upwardly projecting portion 568 sized to seal an end of the mist outlet port 208 so as to prevent leakage of mist or fluid between the gas flow path and the mouthpiece 401. The divider 503 further comprises a hole 571 positioned concentric with the upwardly projecting portion 568 such that when assembled, the hole aligns with the mist outlet port 208 and allows passage of the mist therethrough.

The direction of the gas flow path along which mist flows from the ultrasonic transducers 505, 506 to the mist outlet port 208 is in the same of substantially the same plane or in a plane which is parallel or generally parallel to the plane of the holder surface. Consequently, the mist flows in the same or substantially the same direction as liquid flows along each of the capillaries 509, 510. This improves the operation of the mist generator 500 by minimising the risk of liquid from the mist building up and over saturating the parts of the capillaries 509, 510 that are superimposed on the atomisation surfaces. The risk of oversaturated capillaries 509, 510 degrading the operation of the mist generator 500 is therefore reduced compared with conventional mist generator devices that may have a more complex mist flow path out from the device.

The mist generator 500 minimises or eliminates the possibility of any liquid leakage. This is critical for consistency of dosage delivery. A leaking device may have less liquid (containing therapeutic, drug, etc.) in the liquid chambers 546, 547 than what would be prescribed for treatment of a condition.

The mist generator 500 comprises an end cap 485 and printed circuit board 240 as in the mist generator 400.

The mist generator 500 is designed with ease of manufacture and assembly in mind. Figures 67 and 68 show the transducer holder 514 in an assembled state. The transducer holder 514 is assembled by fitting the annular electrical connections 507, 508 to the ultrasonic transducers 505, 506, and placing the ultrasonic transducers 505, 506 in the recesses 520, 521. The first and second electrical contacts 518, 519 are placed in the channels 535, 536, with an end of each contact 518, 519 tangentially coupled to each of the two annular electrical connections 507, 508. The third and fourth electrical contacts 515, 516 are coupled to the rear of the ultrasonic transducers 505, 506.

The capillaries 509, 510 are placed such that a first portion of each capillary 509, 510 lies within a respective liquid chamber 546, 547, and at least some of the second portion of each capillary 509, 510 substantially covers or lies within the through holes 539, 540.

The second assembly portion 512 and the third assembly portion 513 are coupled such that the extrusions 544, 545 seal the liquid chambers 546, 547 after the liquids have been transferred into the liquid chambers 546, 547. It will be appreciated that the sonication assembly may alternatively be assembled before the liquid chambers 546, 547 are at least partially filled. A filling configuration similar to that described in relation to mist generator 400 may be employed.

The first assembly portion 511; second and third assembly portions 512, 513; transducer holder 514; and fourth assembly portion 517 are coupled in a direction normal to the planar surfaces of the sonication assembly. Once coupled, the sonication assembly is inserted at least partly into the internal cavity of the housing 204.

The sonication assembly contacts the side walls of the housing 204 and forms a seal which minimises or prevents the gas flowing between the sonication assembly and the side walls of the housing 204. In this example, the seal is achieved by deformation of the resilient material of the sonication assembly against the walls of the housing 204. In this example, since the seal is achieved by the deformation of the resilient material, no additional sealant or adhesive is required between the sonication assembly and the housing, or within parts of the sonication assembly itself. However, the present disclosure does not preclude the use of an adhesive or sealant.

Once the foam layer 504 and divider 503 are in place, the mouthpiece 401 is attached to the housing 204 to substantially close the internal cavity of the housing 204. The mouthpiece 401 comprises a mist outlet port 208 which fluidly couples with the upwardly projecting portion 568 of the divider 503.

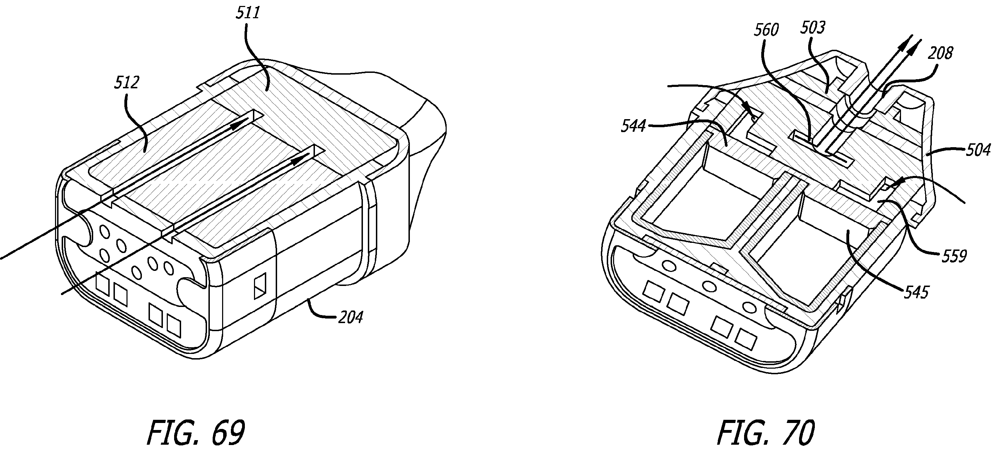

As shown in figures 69 and 70, the assembled mist generator 500 defines an air flow path from the air inlet ports 569, 570 to the mist outlet port 208. In some examples, the mist generator 500 may include a plurality of mist outlet ports 208, and so the flow path will differ from that described below.

Air enters the mist generator 500 through air inlet ports 569, 570 and flows along the inlet ducts formed by the air inlet channels 557, 558 of the first assembly portion 511, and the channels 550, 551 of the second assembly portion 512. The air then enters the sonication chambers through sonication chamber air inlets 552, 553.

The air passes across the sonication chamber where it mixes with the mist generated by the ultrasonic transducers 505, 506, and flows to the mist outlets 554, 555. In order to improve flow through the sonication chamber, the inlets 552, 553 and outlets 554, 555 may be diametrically opposed within the sonication chambers.

The mist flows through the mist outlets 554, 555 and into the mist outlet channel 560 formed in the first assembly portion 511. The mist then flows through the conduit formed by the mist outlet hole 561 in the first assembly portion 511 and the groove 556 in the second assembly portion 512, and finally flows to the outlet port 208 via the hole in the foam layer 504 and the hole in the divider 503.

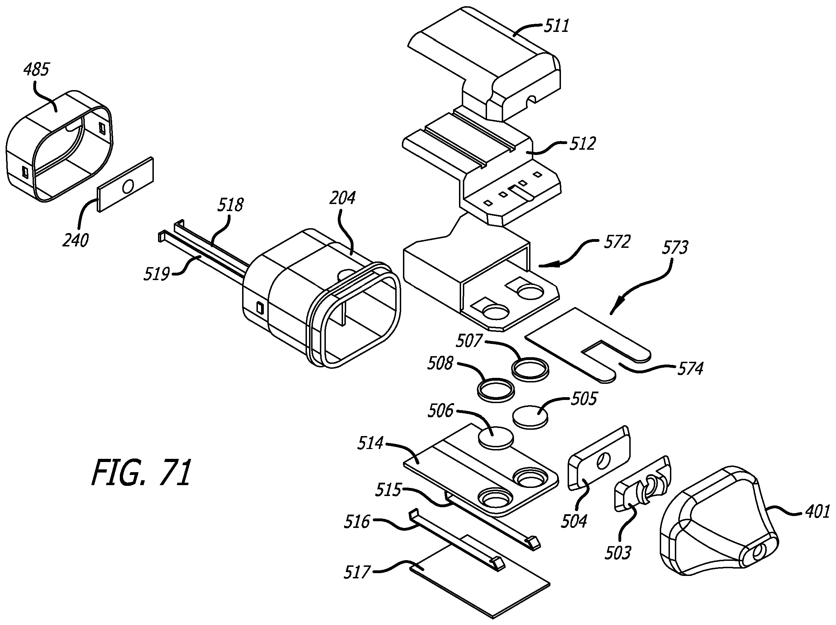

Referring to figure 71 of the accompanying drawings, in some examples a single absorbent capillary 573 serves to provide liquid from a single liquid chamber 572. In such examples, the capillary 573 may comprise an open-ended slot 574 to ensure that a liquid present in the capillary does not travel from one ultrasonic transducer 505, 506 to another. Although the example shows vertical members of the capillary 573, other shapes, such as a "Y" shape capillary, are envisaged.

At least part of the capillary 573 is generally parallel to a plane of the holder surface and extends along the holder surface from the single liquid chamber 572 to the first ultrasonic transducer 505 and to the second ultrasonic transducer 506. A first portion of the capillary 573 is superimposed on the holder surface, a second portion of the capillary 573 is in contact with at least a portion of the first atomisation surface and a third portion of the capillary 573 is in contact with at least a portion of the second atomisation surface. The capillary 573 is thus configured to transport the liquid by capillary action from the liquid chamber 572 to the first atomisation surface and the second atomisation surface,

The example of figure 71 shows the single liquid chamber having two sloped portions on its base. The capillary 573 may include a complimentary shape in order to reach the bottom of the liquid chamber 572. The geometry of the bottom of the liquid chamber 572 may be of any shape.

Further, in some examples having a single liquid chamber 572, two capillaries 509, 510 are still used.

Figures 60 to 71 and the above description disclose examples comprising two ultrasonic transducers 505, 506. The disclosure is equally relevant in examples comprising one, three, four, or any number of ultrasonic transducers.

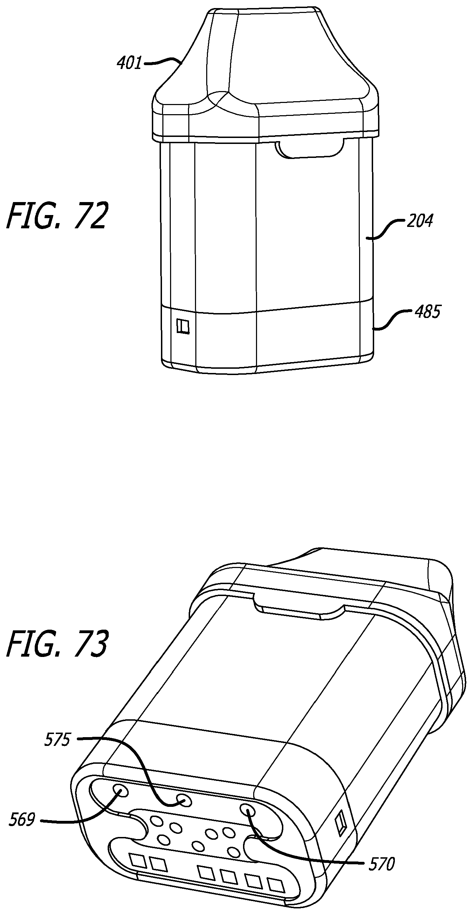

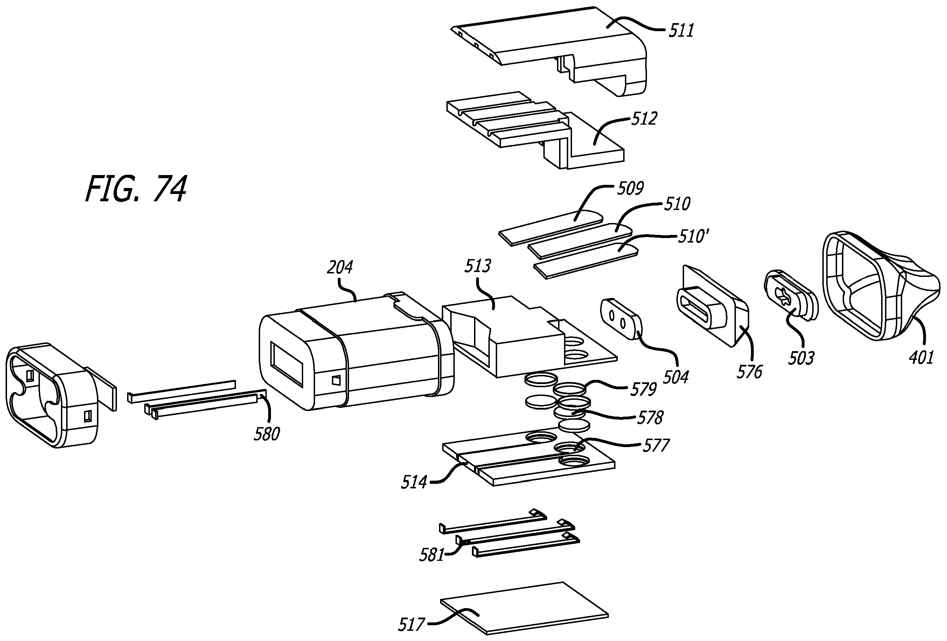

Figures 72 to 74 show an example including three ultrasonic transducers 505, 506, 578 (note that only the third instance of the components include reference numerals in figure 74 for clarity). The addition of one further ultrasonic transducer 578 means that a further instance of many other components may be required, such as but not limited to: a fifth and sixth electrical contact 580, 581; a further annular electrical connection 579; a further recess 577 in the transducer holder 514; a further inlet port 575; a further capillary 510', or at least a further portion thereof in examples utilising a capillary similar to that shown in figure 71. A further liquid chamber may also be provided in some examples.

The addition of a further ultrasonic transducer 578, and its associated ancillaries, may require the mist generator 500 to be of a larger dimension. Where this is the case, a funnelling portion 576 may be provided to enable the mouthpiece 401 to remain a practical and usable size.

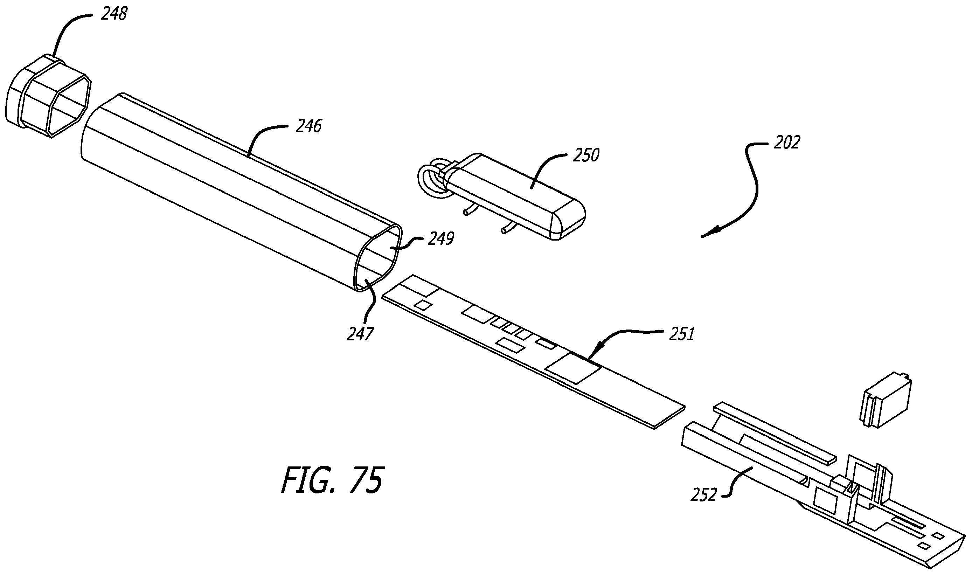

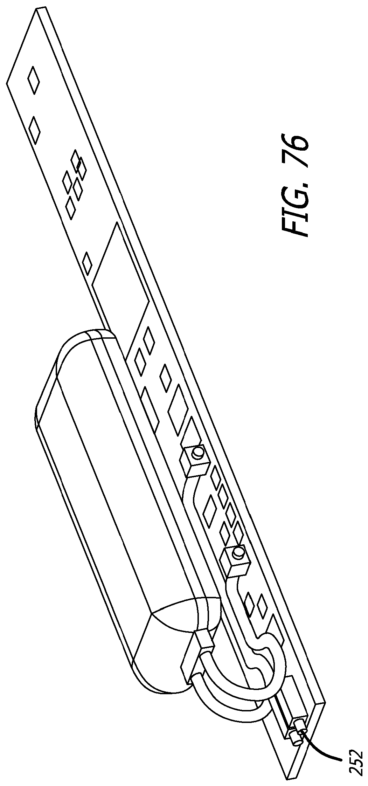

The driver 202 will now be described with reference to Figures 75 and 76 initially. The driver 202 comprises a driver housing 246 which is at least partly of metal. In some examples, the driver housing 246 is entirely of aluminium (AL6063 T6) which protects the internal components from the environment (dust, water splashes, etc.) and also protects from damage from shocks (accidental drops, etc.).

In some examples, the driver housing 246 is provided with vents on its sides that allow ambient air to enter the device for two purposes; one to have ventilation around the electronic components and keep them within operating temperatures, and these vents also act as air inlets with air entering through these vents into the device, and then through the airflow bridge into the mist generator 201.

The driver housing 246 is elongate with an internal chamber 247 which houses the components of the driver 202. One end of the driver housing 246 is closed by an end cap 248. The other end of the driver housing 247 has an opening 249 which provides an opening for the recess 203 of the driver 202.

The driver 202 comprises a battery 250 which is connected to a printed circuit board 251. In some examples, the battery 250 is a 3.7V DC Li-Po battery with 1140 mAh capacity and 10C discharge rate. The high discharge rate is required for voltage amplification of up to 15V that is required by the ultrasonic transducer 215 for desirable operation. The shape and size of the battery is designed, within physical constraints, as per the shape and size of the device and space allocated for the power source.

The printed circuit board 251 incorporates a processor and a memory and other electronic components for implementing the electrical functions of the driver 202. Charging pins 258 are provided on one end of the printed circuit board 251 and which extend through the end cap 248 to provide charging connections to charge the battery 250.

The printed circuit board 251 is retained within the driver housing 246 by a skeleton 252. The skeleton 252 has a channel 253 which receives the printed circuit board 251. The skeleton 252 incorporates raised side portions 254, 255 which support the battery 250.

In some examples, the skeleton 252 is manufactured using industrial injection moulding processes. The moulded plastic skeleton ensures all parts are fixed and not loosely fitting inside the case. It also forms a cover over the front part of the PCB (Printed Circuit Board) which received the mist generator 201 when it is inserted into the driver 202.

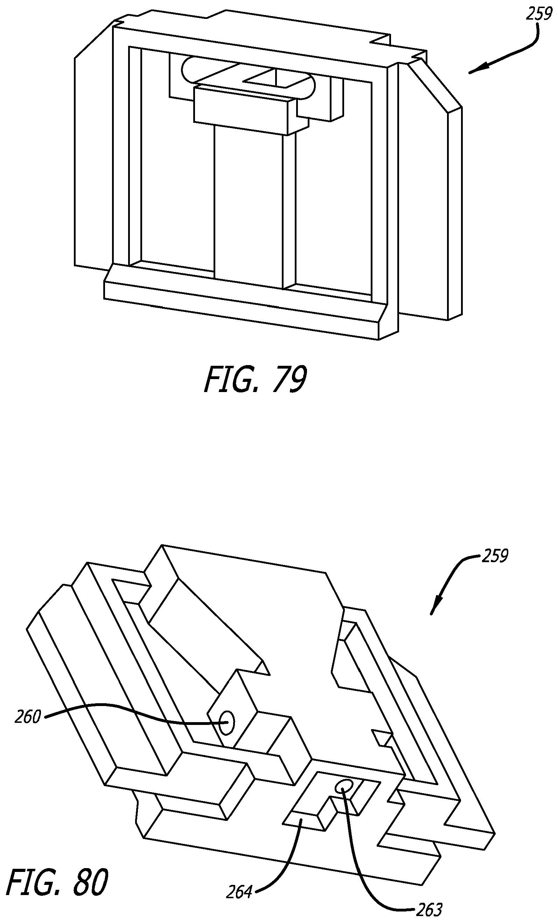

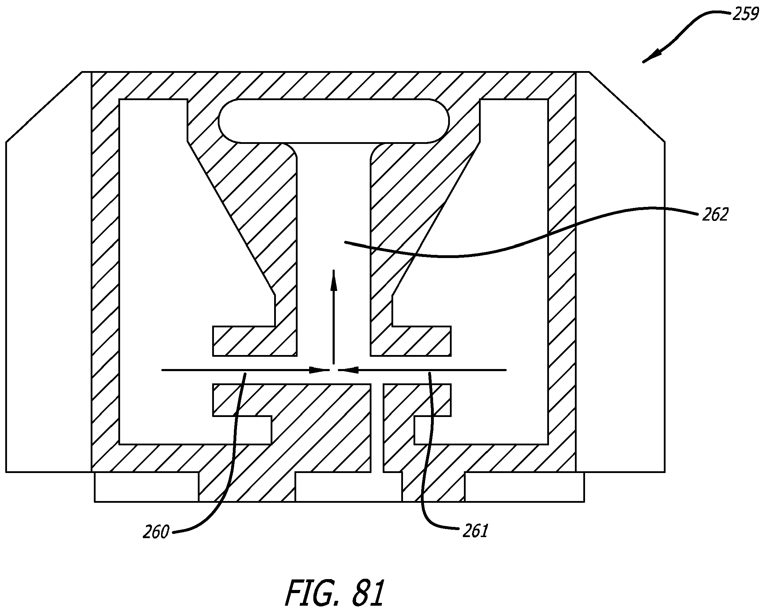

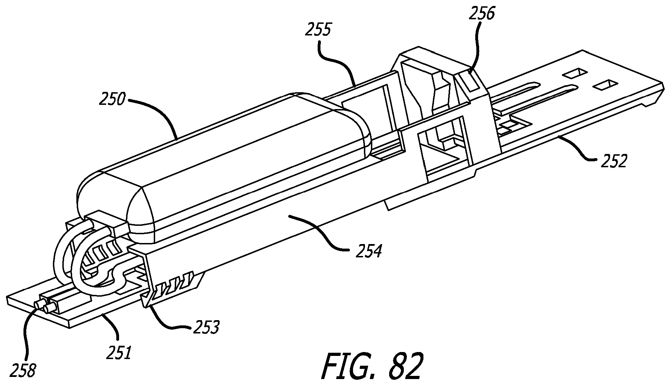

The driver 202 comprises an airflow sensor which acts as a switch for activating and supplying power to the transducer for sonication and aerosol production. The airflow sensor is mounted onto the PCB in the device and requires a certain atmospheric pressure drop around it to activate the driver 202. For this, an airflow bridge 259 as shown in Figures 79 to 81 is designed with internal channels 260, 261 that direct air from the surrounding in through the airflow bridge 259 into the aerosol chamber 262. The skeleton 252 comprises opposing channels 256, 257 for receiving portions of the airflow bridge 259, as shown in Figure 82.

The internal channels in the airflow bridge 259 have a micro-channel 263 (0.5mm diameter) that extends down towards a chamber 264 that completely covers the airflow sensor. As the air flows in from the side inlets and upwards to the aerosol chamber 262, it creates a negative pressure in the micro-channel 263 that triggers the airflow sensor to activate the device.

The device is a compact, portable and highly advanced device that allows precise, safe and monitored aerosolisation. This is done by incorporating high-quality electronic components designed with IPC class 3 - medical grade - in mind.

The electronics of the driver 202 are divided as such:

## 1. Sonication section

In order to obtain the most efficient aerosolisation to date for inhalation in a portable device, with particle size below 1um, the sonication section has to provide the contacts pads receiving the ultrasonic transducer 215 (piezoelectrical ceramic disc (PZT)) with high adaptive frequency (approximately 3MHz).

This section not only has to provide high frequency but also protect the ultrasonic transducer 215 against failures while providing constant optimised cavitation.

PZT mechanical deformation is linked to the AC Voltage amplitude that is applied to it, and in order to guarantee optimal functioning and delivery of the system at every sonication, the maximum deformation must be supplied to the PZT all the time.

However, in order to prevent the failure of the PZT, the active power transferred to it must be precisely controlled.

This could only be achieved by designing a custom, not existing in the market, Power Management Integrated Circuit (PMIC) chip which is provided on the printed circuit board of the driver 202. This PMIC allows modulation of the active power given to the PZT at every instant without compromising the mechanical amplitude of vibration of the PZT. By Pulse Width Modulation (PWM) of the AC voltage applied to the PZT, the mechanical amplitude of the vibration remains the same.

The only 'on the shelf' option available would have been to modify the output AC voltage via the use of a Digital to Analog Converter (DAC). The energy transmitted to the PZT would be reduced but so would the mechanical deformation which as a result completely degrades and prevents proper aerosolisation. Indeed, the RMS voltage applied would be the same with effective Duty Cycle modulation as with Voltage modulation, but the active power transferred to the PZT would degrade. Indeed, given the formula below:

$$\text{Active Power displayed to the PZT being } Pa = \frac{2\sqrt{2}}{\pi} Irms * Vrms * cos\varphi,$$

Where

$\varphi$ *is* the shift in phase between current and voltage
$I_{rms}$ is the root mean square Current
$V_{rms}$ is the root mean square Voltage.

When considering the first harmonic, Irms is a function of the real voltage amplitude applied to the transducer, as the pulse width modulation alters the duration of voltage supplied to the transducer, controlling Irms.

The specific design of the PMIC uses a state-of-the-art design, enabling ultra-precise control of the frequency range and steps to apply to the PZT including a complete set of feedback loops and monitoring path for the control section to use.

The rest of the aerosolisation section is composed of the DC/DC boost converter and transformer that carry the necessary power from a 3.7V battery to the PZT contact pads.

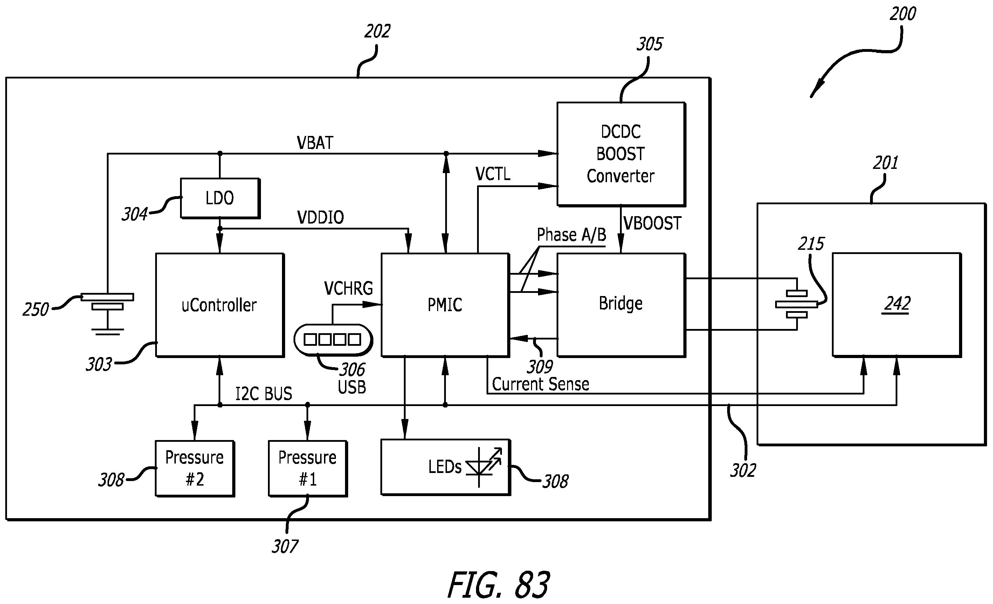

Referring now to Figure 83 of the accompanying drawings, the driver 202 comprises an ultrasonic transducer driver microchip which is referred to herein as a power management integrated circuit or PMIC 300. The PMIC 300 is a microchip for driving a resonant circuit.

The resonant circuit is an inductance (L) capacitance (C) circuit (LC tank), an antenna or, in this case, a piezoelectric transducer (the ultrasonic transducer 215).

In this disclosure, the terms chip, microchip and integrated circuit are interchangeable. The microchip or integrated circuit is a single unit which comprises a plurality of interconnected embedded components and subsystems. The microchip is, for example, at least partly of a semiconductor, such as silicon, and is fabricated using semiconductor manufacturing techniques.

The driver 202 also comprises a second microchip which is referred to herein as a bridge integrated circuit or bridge IC 301 which is electrically connected to the PMIC 300. The bridge IC 301 is a microchip for driving a resonant circuit, such as an LC tank, an antenna or a piezoelectric transducer. The bridge IC 301 is a single unit which comprises a plurality of interconnected embedded components and subsystems.

In this example, the PMIC 300 and the bridge IC 301 are mounted to the same PCB of the driver 202. In this example, the physical dimensions of the PMIC 300 are 1-3mm wide and 1-3mm long and the physical dimensions of the bridge IC 301 are 1-3mm wide and 1-3mm long.

The mist generator 201 comprises a programmable or one time programmable integrated circuit or OTP IC 242. When the mist generator 201 is coupled to the driver 202, the OTP IC is electrically connected to the PMIC 300 to receive power from the PMIC 300 such that the PMIC 300 can manage the voltage supplied to the OTP IC 242. The OTP IC 242 is also connected to a communication bus 302 in the driver 202. In this example, the communication bus 302 is an I2C bus but in other examples the communication bus 302 is another type of digital serial communication bus.

The ultrasonic transducer 215 in the mist generator 201 is electrically connected to the bridge IC 301 so that the ultrasonic transducer 215 may be driven by an AC drive signal generated by the bridge IC 301 when the device 200 is in use.

The driver 202 comprises a processor in the form of a microcontroller 303 which is electrically coupled for communication with the communication bus 302. In this example, the microcontroller 303 is a Bluetooth™ low energy (BLE) microcontroller. The microcontroller 303 receives power from a low dropout regulator (LDO) 304 which is driven by the battery 250. The LDO 304 provides a stable regulated voltage to the microcontroller 303 to enable the microcontroller 303 to operate consistently even when there is a variation in the voltage of the battery 250.

The driver 202 comprises a voltage regulator in the form of a DC-DC boost converter 305 which is powered by the battery 250. The boost converter 305 increases the voltage of the battery 250 to a programmable voltage VBOOST. The programmable voltage VBOOST is set by the boost converter 305 in response to a voltage control signal VCTL from the PMIC 300. As will be described in more detail below, the boost converter 305 outputs the voltage VBOOST to the bridge IC 301. In other examples, the voltage regulator is a buck converter or another type of voltage regulator which outputs a selectable voltage.

The voltage control signal VCTL is generated by a digital to analogue converter (DAC) which, in this example, is implemented within the PMIC 300. The DAC is not visible in Figure 83 since the DAC is integrated within the PMIC 300. The DAC and the technical benefits of integrating the DAC within the PMIC 300 are described in detail below.

In this example, the PMIC 300 is connected to a power source connector in the form of a universal serial bus (USB) connector 306 so that the PMIC 300 can receive a charging voltage VCHRG when the USB connector 306 is coupled to a USB charger.

The driver 202 comprises a first pressure sensor 307 which, in this example, is a static pressure sensor. The driver 202 also comprises a second pressure sensor 308 which, in this example, is a dynamic pressure sensor. However, in other examples, the driver 202 comprises only one of the two pressure sensors 307, 308. As described above, the pressure sensors 307, 308 sense a change in the pressure in the aerosol chamber 262 to sense when a user is drawing on the mist inhaler 200.

In this example, the driver 202 comprises a plurality of LEDs 321-326 which are controlled by the PMIC 300.

The microcontroller 303 functions as a master device on the communication bus 302, with the PMIC 300 being a first slave device, the OTP IC 242 being a second slave device, the second pressure sensor 308 being a third slave device and the first pressure sensor 307 being a fourth slave device. The communication bus 302 enables the microcontroller 303 to control the following functions within the driver 202:

1. All functions of the PMIC are highly configurable by the microcontroller 303.

2. The current flowing through the ultrasonic transducer 215 is sensed by a high bandwidth sense and rectifier circuit at a high common mode voltage (high side of the bridge). The sensed current is converted into a voltage proportional to the rms current and provided as a buffered voltage at a current sense output pin 309 of the bridge IC 301. This voltage is fed to and sampled in the PMIC 300 and made available as a digital representation via I2C requests. Sensing the current flowing through the ultrasonic transducer 215 forms part of the resonant frequency tracking functionality. As described herein, the ability of the device to enable this functionality within the bridge IC 301 provides significant technical benefits.

3. The DAC (not shown in Figure 83) integrated within the PMIC 300 enables the DC-DC boost converter voltage VBOOST to be programmed to be between 10V and 20V.

4. The microcontroller 303 enables the charger sub-system of the driver 202 to manage the charging of the battery 250, which in this example is a single cell battery.

5. A Light Emitting Diode (LED) driver module (not shown) is powered by the PMIC 300 to drive and dim digitally the LEDs 321-326 either in linear mode or in gamma corrected mode.

6. The microcontroller 303 is able to read Pressure#1 and Pressure#2 sensor values from the pressure sensors 307, 308.

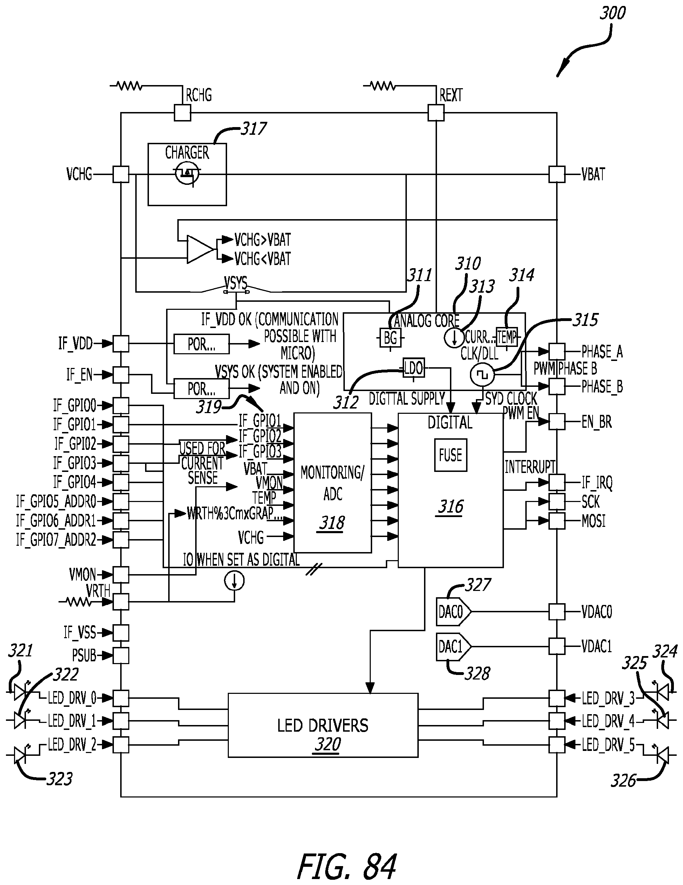

Referring now to Figure 84 of the accompanying drawings, the PMIC 300 is, in this example, a self-contained chip or integrated circuit which comprises integrated subsystems and a plurality of pins which provide electrical inputs and outputs to the PMIC 300. The references to an integrated circuit or chip in this disclosure are interchangeable and either term encompasses a semiconductor device which may, for instance, be of silicon.

The PMIC 300 comprises an analogue core 310 which comprises analogue components including a reference block (BG) 311, a LDO 312, a current sensor 313, a temperature sensor 314 and an oscillator 315.

As described in more detail below, the oscillator 315 is coupled to a delay locked loop (DLL) which outputs pulse width modulation (PWM) phases A and B. The oscillator 315 and the DLL generate a two phase centre aligned PWM output which drives an H bridge in the bridge IC 301.

The DLL comprises a plurality of delay lines connected end to end, wherein the total delay of the delay lines is equal to the period of the main clock signal clk_m. In this example, the DLL is implemented in a digital processor subsystem, referred to herein as a digital core 316, of the PMIC 300 which receives a clock signal from the oscillator 315 and a regulated power supply voltage from the LDO 312. The DLL is implemented in a large number (e.g. in the order of millions) of delay gates which are connected end to end in the digital core 316.

The implementation of the oscillator 315 and the DLL in the same integrated circuit of the PMIC 300 in order to generate a two phase centre aligned PWM signal is unique since at present no signal generator component in the integrated circuit market comprises this implementation.

As described herein, PWM is part of the functionality which enables the driver 202 to track the resonant frequency of the ultrasonic transducer 215 accurately in order to maintain an efficient transfer from electrical energy to kinetic energy in order to optimise the generation of mist.

In this example, the PMIC 300 comprises a charger circuit 317 which controls the charging of the battery 250, for instance by power from a USB power source.

The PMIC 300 comprises an integrated power switch VSYS which configures the PMIC 300 to power the analogue core 310 by power from the battery 250 or by power from an external power source if the battery 250 is being charged.

The PMIC 300 comprises an embedded analogue to digital converter (ADC) subsystem 318. The implementation of the ADC 318 together with the oscillator 315 in the same integrated circuit is, in itself, unique since there is no other integrated circuit in the integrated circuit market which comprises an oscillator and an ADC implemented as sub-blocks within the integrated circuit. In a conventional device, an ADC is typically provided as a separate discrete component from an oscillator with the separate ADC and oscillator being mounted to the same PCB. The problem with this conventional arrangement is that the two separate components of the ADC and the oscillator take up space unnecessarily on the PCB. A further problem is that the conventional ADC and oscillator are usually connected to one another by a serial data communication bus, such as an I2C bus, which has a limited communication speed of up to only 400 kHz. In contrast to conventional devices, the PMIC 300 comprises the ADC 318 and the oscillator 315 integrated within the same integrated circuit which eliminates any lag in communication between the ADC 318 and the oscillator 315, meaning that the ADC 318 and the oscillator 315 can communicate with one another at high speed, such as at the speed of the oscillator 315 (e.g. 3 MHz to 5 MHz).

In the PMIC 300 of this example, the oscillator 315 is running at 5 MHz and generates a clock signal SYS CLOCK at 5 MHz. However, in other examples, the oscillator 315 generates a clock signal at a much higher frequency of up to 105 MHz. The integrated circuits described herein are all configured to operate at the high frequency of the oscillator 315.

The ADC 318 comprises a plurality of feedback input terminals or analogue inputs 319 which comprise a plurality of GPIO inputs (IF_GPIO1-3). At least one of the feedback input terminals or the analogue inputs 319 receives a feedback signal from an H-bridge circuit in the bridge IC 301, the feedback signal being indicative of a parameter of the operation of the H-bridge circuit or an AC drive signal when the H-bridge circuit is driving a resonant circuit, such as the ultrasonic transducer 215, with the AC drive signal. As described below, the GPIO inputs are used to receive a current sense signal from the bridge IC 301 which is indicative of the route mean square (rms) current reported by the bridge IC 301. In this example, one of the GPIO inputs is a feedback input terminal which receives a feedback signal from the H-bridge in the bridge IC 301.

The ADC subsystem 318 samples analogue signals received at the plurality of ADC input terminals 319 at a sampling frequency which is proportional to the frequency of the main clock signal. The ADC subsystem 318 then generates ADC digital signals using the sampled analogue signals.

In this example, the ADC 318 which is incorporated in the PMIC 300 samples not only the RMS current flowing through the H-bridge 334 and the ultrasonic transducer 215 but also voltages available in the system (e.g. VBAT, VCHRG, VBOOST), the temperature of the PMIC 300, the temperature of the battery 250 and the GPIO inputs (IF_GPIO1-3) which allow for future extensions.

The digital core 316 receives the ADC generated digital signals from the ADC subsystem and processes the ADC digital signals to generate the driver control signal. The digital core 316 communicates the driver control signal to the PWM signal generator subsystem (DLL 332) to control the PWM signal generator subsystem.

Rectification circuits existing in the market today have a very limited bandwidth (typically less than 1 MHz). Since the oscillator 315 of the PMIC 300 is running at up to 5 MHz or even up to 105 Mhz, a high bandwidth rectifier circuit is implemented in the PMIC 300. As will be described below, sensing the RMS current within an H bridge of the bridge IC 301 forms part of a feedback loop which enables the driver 202 to drive the ultrasonic transducer 215 with high precision. The feedback loop is a game changer in the industry of driving ultrasound transducers since it accommodates for any process variation in the piezo electric transducer production (variations of resonance frequencies) and it compensates for temperature effects of the resonance frequency. This is achieved, in part, by the realisation of integrating the ADC 318, the oscillator 315 and the DLL within the same integrated circuit of the PMIC 300. The integration enables these subsystems to communicate with one another at high speed (e.g. at the clock frequency of 5 MHz or up to 105 MHz). Reducing the lag between these subsystems is a game changer in the ultrasonics industry, particularly in the field of mist generators.

The ADC 318 comprises a battery voltage monitoring input VBAT and a charger input voltage monitoring input VCHG as well as voltage monitoring inputs VMON and VRTH as well as a temperature monitoring input TEMP.

The temperature monitoring input TEMP receives a temperature signal from the temperature sensor 314 which is embedded within the PMIC 300. This enables the PMIC 300 to sense the actual temperature within the PMIC 300 accurately so that the PMIC 300 can detect any malfunction within the PMIC 300 as well as malfunction to other components on the printed circuit board which affect the temperature of the PMIC 300. The PMIC 300 can then control the bridge IC 301 to prevent excitation of the ultrasonic transducer 215 if there is a malfunction in order to maintain the safety of the mist delivery device 200.

The additional temperature sensor input VRTH receives a temperature sensing signal from an external temperature sensor within the driver 202 which monitors the temperature of the battery 250. The PMIC 300 can thus react to stop the battery 250 from being charged in the event of a high battery temperature or otherwise shut down the driver 202 in order to reduce the risk of damage being caused by an excessively high battery temperature.

The PMIC 300 comprises an LED driver 320 which, in this example, receives a digital drive signal from the digital core 316 and provides LED drive output signals to six LEDs 321-326 which are configured to be coupled to output pins of the PMIC 300. The LED driver 320 can thus drive and dim the LEDs 321-326 in up to six independent channels.

The PMIC 300 comprises a first digital to analogue converter (DAC) 327 which converts digital signals within the PMIC 300 into an analogue voltage control signal which is output from the PMIC 300 via an output pin VDAC0. The first DAC 327 converts a digital control signal generated by the digital core 316 into an analogue voltage control signal which is output via the output pin VDAC0 to control a voltage regulator circuit, such as the boost converter 305. The voltage control signal thus controls the voltage regulator circuit to generate a predetermined voltage for modulation by the H-bridge circuit to drive a resonant circuit, such as the ultrasonic transducer 215, in response to feedback signals which are indicative of the operation of the resonant circuit (the ultrasonic transducer 215).

In this example, the PMIC 300 comprises a second DAC 328 which converts digital signals within the PMIC 300 into an analogue signal which is output from the PMIC 300 via a second analogue output pin VDAC1.

Embedding the DACs 327, 328 within the same microchip as the other subsystems of the PMIC 300 allows the DACs 327, 328 to communicate with the digital core 316 and other components within the PMIC 300 at high speed with no or minimal communication lag. The DACs 327, 328 provide analogue outputs which control external feedback loops. For instance, the first DAC 327 provides the control signal VCTL to the boost converter 305 to control the operation of the boost converter 305. In other examples, the DACs 327, 328 are configured to provide a drive signal to a DC-DC buck converter instead of or in addition to the boost converter 305. Integrating the two independent DAC channels in the PMIC 300 enables the PMIC 300 to manipulate the feedback loop of any regulator used in the driver 202 and allows the driver 202 to regulate the sonication power of the ultrasonic transducer 215 or to set analogue thresholds for absolute maximum current and temperature settings of the ultrasonic transducer 215.

The PMIC 300 comprises a serial communication interface which, in this example, is an I2C interface which incorporates external I2C address set through pins.

The PMIC 300 also comprises various functional blocks which include a digital machine (FSM) to implement the functionality of the microchip. These blocks will be described in more detail below.

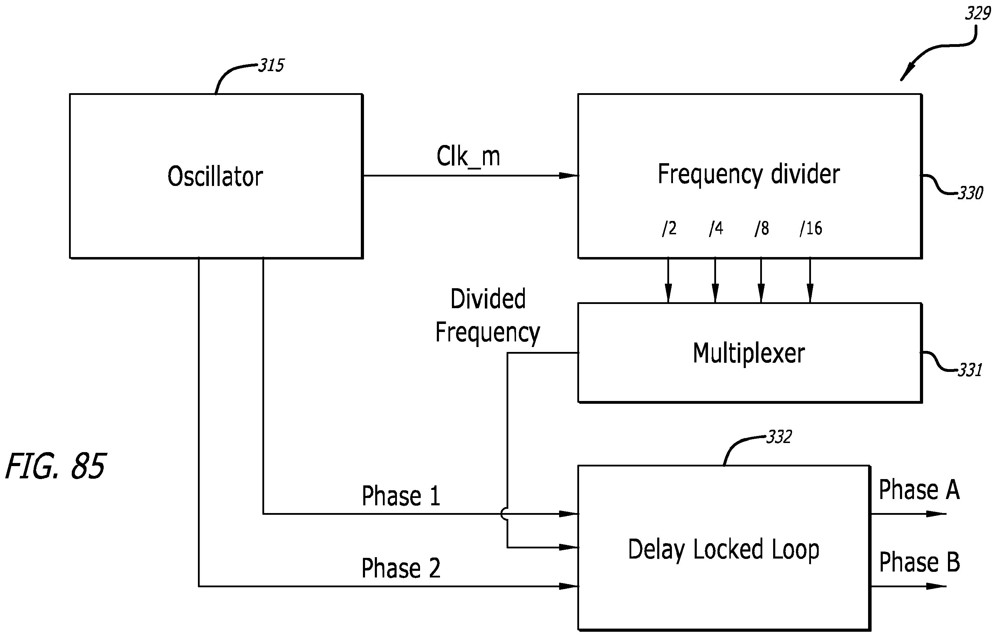

Referring now to Figure 85 of the accompanying drawings, a pulse width modulation (PWM) signal generator subsystem 329 is embedded within the PMIC 300. The PWM generator system 329 comprises the oscillator 315, and frequency divider 330, a multiplexer 331 and a delay locked loop (DLL) 332. As will be described below, the PWM generator system 329 is a two phase centre aligned PWM generator.

The frequency divider 330, the multiplexer 331 and the DLL 332 are implemented in digital logic components (e.g. transistors, logic gates, etc.) within the digital core 316.

In examples of this disclosure, the frequency range which is covered by the oscillator 315 and respectively by the PWM generator system 329 is 50 kHz to 5 MHz or up to 105 MHz. The frequency accuracy of the PWM generator system 329 is $\pm$1% and the spread over temperature is $\pm$1%. In the IC market today, no IC has an embedded oscillator and two phase centre aligned PWM generator that can provide a frequency range of 50 kHz to 5 MHz or up to 105 MHz.

The oscillator 315 generates a main clock signal (clk_m) with a frequency of 50 kHz to 5 MHz or up to 105 MHz. The main clock clk_m is input to the frequency divider 330 which divides the frequency of the main clock clk_m by one or more predetermined divisor amounts. In this example, the frequency divider 330 divides the frequency of the main clock clk_m by 2, 4, 8 and 16 and provides the divided frequency clocks as outputs to the multiplexer 331. The multiplexer 331 multiplexes the divided frequency clocks and provides a divided frequency output to the DLL 332. This signal which is passed to the DLL 332 is a frequency reference signal which controls the DLL 332 to output signals at a desired frequency. In other examples, the frequency divider 330 and the multiplexer 331 are omitted.

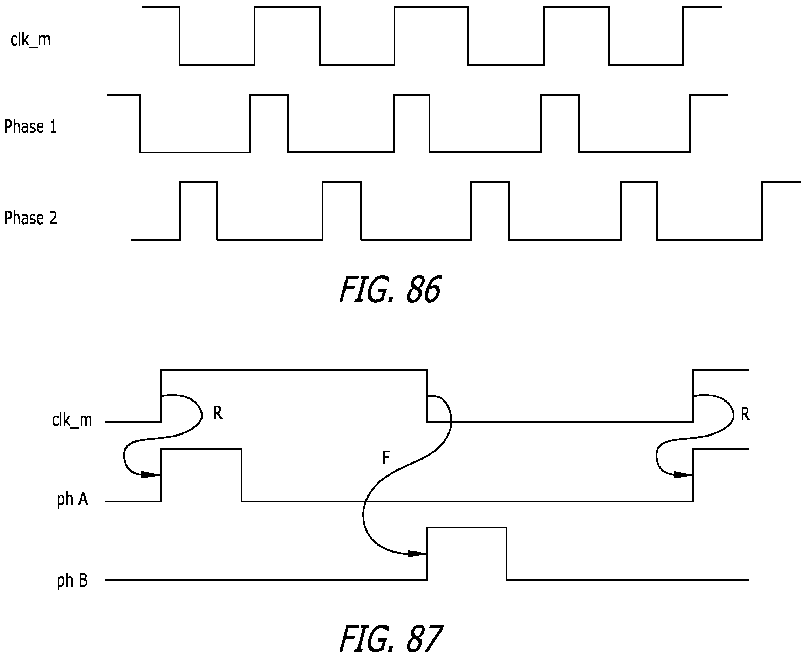

The oscillator 315 also generates two phases; a first phase clock signal Phase 1 and a second phase clock signal Phase 2. The phases of the first phase clock signal and the second phase clock signal are centre aligned. As illustrated in Figure 86:

- The first phase clock signal Phase 1 is high for a variable time of clk_m's positive half-period and low during clk_m's negative half-period.
- The second phase clock signal Phase 2 is high for a variable time of clk_m's negative half-period and low during clk_m's positive half-period.

Phase 1 and Phase 2 are then sent to the DLL 332 which generates a double frequency clock signal using the first phase clock signal Phase 1 and the second phase clock signal Phase 2. The double frequency clock signal is double the frequency of the main clock signal clk_m. In this example, an "OR" gate within the DLL 332 generates the double frequency clock signal using the first phase clock signal Phase 1 and the second phase clock signal Phase 2. This double frequency clock or the divided frequency coming from the frequency divider 330 is selected based on a target frequency selected and then used as reference for the DLL 332.

Within the DLL 332, a signal referred to hereafter as "clock" represents the main clock clk_m multiplied by 2, while a signal referred to hereafter as "clock_del" is a replica of clock delayed by one period of the frequency. Clock and clock_del are passed through a phase frequency detector. A node Vc is then charged or discharged by a charge-pump based on the phase error polarity. A control voltage is fed directly to control the delay of every single delay unit within the DLL 332 until the total delay of the DLL 332 is exactly one period.

The DLL 332 controls the rising edge of the first phase clock signal Phase 1 and the second phase clock signal Phase 2 to be synchronous with the rising edge of the double frequency clock signal. The DLL 332 adjusts the frequency and the duty cycle of the first phase clock signal Phase 1 and the second phase clock signal Phase 2 in response to a respective frequency reference signal and a duty cycle control signal to produce a first phase output signal Phase A and a second phase output signal Phase B to drive an H-bridge or an inverter to generate an AC drive signal to drive an ultrasonic transducer.

The PMIC 300 comprises a first phase output signal terminal PHASE_A which outputs the first phase output signal Phase A to an H-bridge circuit and a second phase output signal terminal PHASE_B which outputs the second phase output signal Phase B to an H-bridge circuit.

In this example, the DLL 332 adjusts the duty cycle of the first phase clock signal Phase 1 and the second phase clock signal Phase 2 in response to the duty cycle control signal by varying the delay of each delay line in the DLL 332 response to the duty cycle control signal.

The clock is used at double of its frequency because guarantees better accuracy. As shown in Figure 87, for the purpose of explanation if the frequency of the main clock clk_m is used (which it is not in examples of this disclosure), Phase A is synchronous with clock's rising edge R, while Phase B is synchronous with clock's falling edge F. The delay line of the DLL 332 controls the rising edge R and so, for the falling edge F, the PWM generator system 329 would need to rely on a perfect matching of the delay units of the DLL 332 which can be imperfect. However, to remove this error, the PWM generator system 329 uses the double frequency clock so that both Phase A and Phase B are synchronous with the rising edge R of the double frequency clock.

To perform a duty-cycle from 20% to 50% with a 2% step size, the delay line of the DLL 332 comprises 25 delay units, with the output of each respective delay unit representing a Phase nth. Eventually the phase of the output of the final delay unit will correspond to the input clock. Considering that all delays will be almost the same, a particular duty cycle is obtained with the output of the specific delay unit with simple logic in the digital core 316.

It is important to take care of the DLL 332 startup as the DLL 332 might not be able to lock a period of delay but two or more periods, taking the DLL 332 to a non-convergence zone. To avoid this issue, a start-up circuit is implemented in the PWM generator system 329 which allows the DLL 332 to start from a known and deterministic condition. The start-up circuit furthermore allows the DLL 332 to start with the minimum delay.

In examples of this disclosure, the frequency range covered by the PWM generator system 329 is extended and so the delay units in the DLL 332 can provide delays of 4 ns (for an oscillator frequency of 5 MHz) to 400 ns (for an oscillator frequency of 50 kHz). In order to accommodate for these differing delays, capacitors Cb are included in the PWM generator system 329, with the capacitor value being selected to provide the required delay.

The Phase A and Phase B are output from the DLL 332 and passed through a digital IO to the bridge IC 301 so that the Phase A and Phase B can be used to control the operation of the bridge IC 301.

The battery charging functionality of the driver 202 will now be described in more detail. The battery charging sub-system comprises the charger circuit 317 which is embedded in the PMIC 300 and controlled by a digital charge controller hosted in the PMIC 300. The charger circuit 317 is controlled by the microcontroller 303 via the communication bus 302. The battery charging sub-system is able to charge a single cell lithium polymer (LiPo) or lithium-ion (Li-ion) battery, such as the battery 250 described above.

In this example, the battery charging sub-system is able to charge a battery or batteries with a charging current of up to 1A from a 5V power supply (e.g. a USB power supply). One or more of the following parameters can be programmed through the communication bus 302 (I2C interface) to adapt the charge parameters for the battery:

- Charge voltage can be set between 3.9V and 4.3V in 100mV steps.
- The charge current can be set between 150 mA and 1000mA in 50mA steps.
- The pre-charge current is 1/10 of the charge current.
- Pre-charge and fast charge timeouts can be set between 5 and 85 min respectively 20 and 340 min.
- Optionally an external negative temperature coefficient (NTC) thermistor can be used to monitor the battery temperature.

In some examples, the battery charging sub-system reports one or more of the following events by raising an interrupt to the host microcontroller 303:

- Battery detected
- Battery is being charged
- Battery is fully charged
- Battery is not present
- Charge timeout reached
- Charging supply is below the undervoltage limit

The main advantage of having the charger circuit 317 embedded in the PMIC 300, is that it allows all the programming options and event indications listed to be implemented within the PMIC 300 which guarantees the safe operation of the battery charging sub-system. Furthermore, a significant manufacturing cost and PCB space saving can be accomplished compared with conventional mist delivery devices which comprise discrete components of a charging system mounted separately on a PCB. The charger circuit 317 also allows for highly versatile setting of charge current and voltage, different fault timeouts and numerous event flags for detailed status analysis.

The analogue to digital converter (ADC) 318 will now be described in more detail. The inventors had to overcome significant technical challenges to integrate the ADC 318 within the PMIC 300 with the high speed oscillator 315. Moreover, integrating the ADC 318 within the PMIC 300 goes against the conventional approach in the art which relies on using one of the many discrete ADC devices that are available in the IC market.

In this example, the ADC 318 samples at least one parameter within the ultrasonic transducer driver chip (PMIC 300) at a sampling rate which is equal to the frequency of the main clock signal clk_m. In this example, the ADC 318 is a 10 bit analogue to digital converter which is able to unload digital sampling from the microprocessor 303 to save the resources of the microprocessor 303. Integrating the ADC 318 within the PMIC 300 also avoids the need to use an I2C bus that would otherwise slow down the sampling ability of the ADC (a conventional device relies on an I2C bus to communicate data between a dedicated discrete ADC and a microcontroller at a limited clock speed of typically up to 400 kHz).

In examples of this disclosure, one or more of the following parameters can be sampled sequentially by the ADC 318:

i. An rms current signal which is received at the ultrasonic transducer driver chip (PMIC 300) from an external inverter circuit which is driving an ultrasonic transducer. In this is example, this parameter is a root mean square (rms) current reported by the bridge IC 301. Sensing the rms current is important to implementing the feedback loop used for driving the ultrasound transducer 215. The ADC 318 is able to sense the rms current directly from the bridge IC 301 via a signal with minimal or no lag since the ADC 318 does not rely on this information being transmitted via an I2C bus. This provides a significant speed and accuracy benefit over conventional devices which are constrained by the comparatively low speeds of an I2C bus.

ii. The voltage of a battery connected to the PMIC 300.

iii. The voltage of a charger connected to the PMIC 300.

iv. A temperature signal, such as a temperature signal which is indicative of the PMIC 300 chip temperature. As described above, this temperature can be measured very accurately due to the temperature sensor 314 being embedded in the same IC as the oscillator 315. For example, if the PMIC 300 temperature goes up, the current, frequency and PWM are regulated by the PMIC 300 to control the transducer oscillation which in turn controls the temperature.

v. Two external pins.

vi. External NTC temperature sensor to monitor battery pack temperature.

In some examples, the ADC 318 samples one or more of the above-mentioned sources sequentially, for instance in a round robin scheme. The ADC 318 samples the sources at high speed, such as the speed of the oscillator 315 which may be up to 5 MHz or up to 105 MHz.

In some examples, the driver 202 is configured so that a user or the manufacturer of the device can specify how many samples shall be taken from each source for averaging. For instance, a user can configure the system to take 512 samples from the rms current input, 64 samples from the battery voltage, 64 from the charger input voltage, 32 samples from the external pins and 8 from the NTC pin. Furthermore, the user can also specify if one of the above-mentioned sources shall be skipped.

In some examples, for each source the user can specify two digital thresholds which divide the full range into a plurality of zones, such as 3 zones. Subsequently the user can set the system to release an interrupt when the sampled value changes zones e.g. from a zone 2 to a zone 3.

No conventional IC available in the market today can perform the above features of the PMIC 300. Sampling with such flexibility and granularity is paramount when driving a resonant circuit or component, such as an ultrasound transducer.

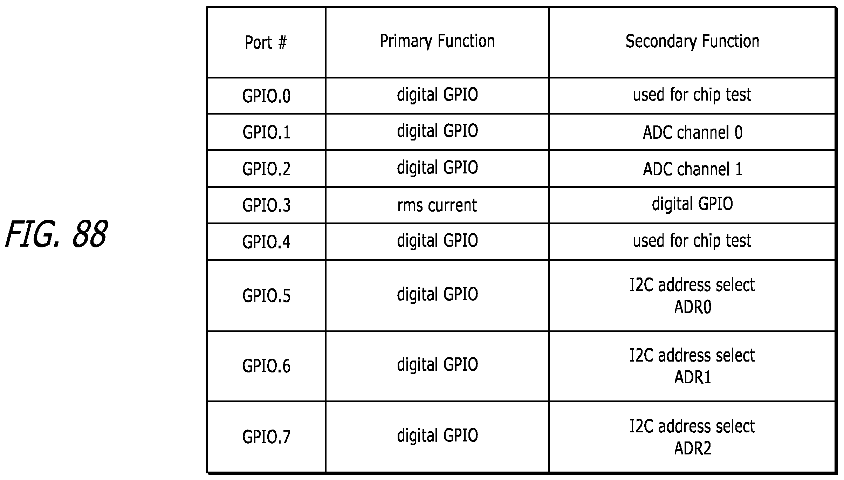

In this example, the PMIC 300 comprises an 8 bit general purpose digital input output port (GPIO). Each port can be configured as digital input and digital output. Some of the ports have an analogue input function, as shown in the table in Figure 88.

The GPIO7-GPIO5 ports of the PMIC 300 can be used to set the device's address on the communication (I2C) bus 302. Subsequently eight identical devices can be used on the same I2C bus. This is a unique feature in the IC industry since it allows eight identical devices to be used on the same I2C bus without any conflicting addresses. This is implemented by each device reading the state of GPIO7-GPIO5 during the first 100 µs after the startup of the PMIC 300 and storing that portion of the address internally in the PMIC 300. After the PMIC 300 has been started up the GPIOs can be used for any other purpose.

As described above, the PMIC 300 comprises a six channel LED driver 320. In this example the LED driver 320 comprises N-Channel Metal-Oxide Semiconductor (NMOS) current sources which are 5V tolerant. The LED driver 320 is configured to set the LED current in four discrete levels; 5mA, 10mA, 15mA and 20mA. The LED driver 320 is configured to dim each LED channel with a 12 bit PWM signal either with or without gamma correction. The LED driver 320 is configured to vary the PWM frequency from 300 Hz to 1.5 KHz. This feature is unique in the field of ultrasonic mist inhalers as the functionality is embedded as a sub-system of the PMIC 300.

In this example, the PMIC 300 comprises two independent 6 Bit Digital to Analog Converters (DAC) 327, 328 which are incorporated into the PMIC 300. The purpose of the DACs 327, 328 is to output an analogue voltage to manipulate the feedback path of an external regulator (e.g. the DC-DC Boost converter 305 a Buck converter or a LDO). Furthermore, in some examples, the DACs 327, 328 can also be used to dynamically adjust the over current shutdown level of the bridge IC 301, as described below.

The output voltage of each DAC 327, 328 is programmable between 0V and 1.5V or between 0V and V_battery (Vbat). In this example, the control of the DAC output voltage is done via I2C commands. Having two DAC incorporated in the PMIC 300 is unique and will allow the dynamic monitoring control of the current. If either DAC 327, 328 was an external chip, the speed would fall under the same restrictions of speed limitations due to the I2C protocol. The active power monitoring arrangement of the driver 202 works with optimum efficiency if all these embedded features are in the PMIC. Had they been external components, the active power monitoring arrangement would be totally inefficient.

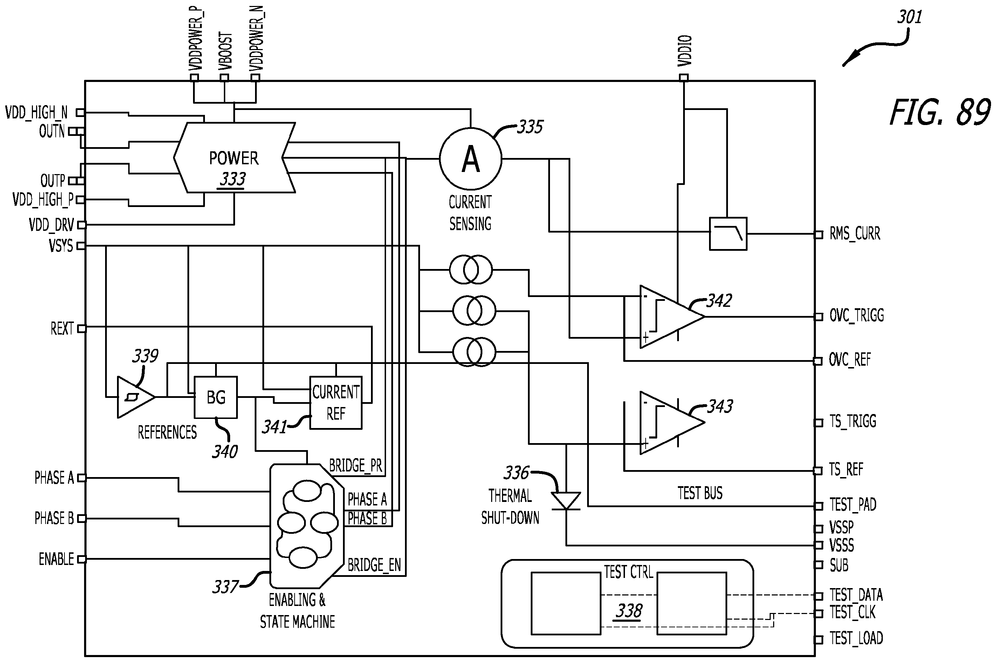

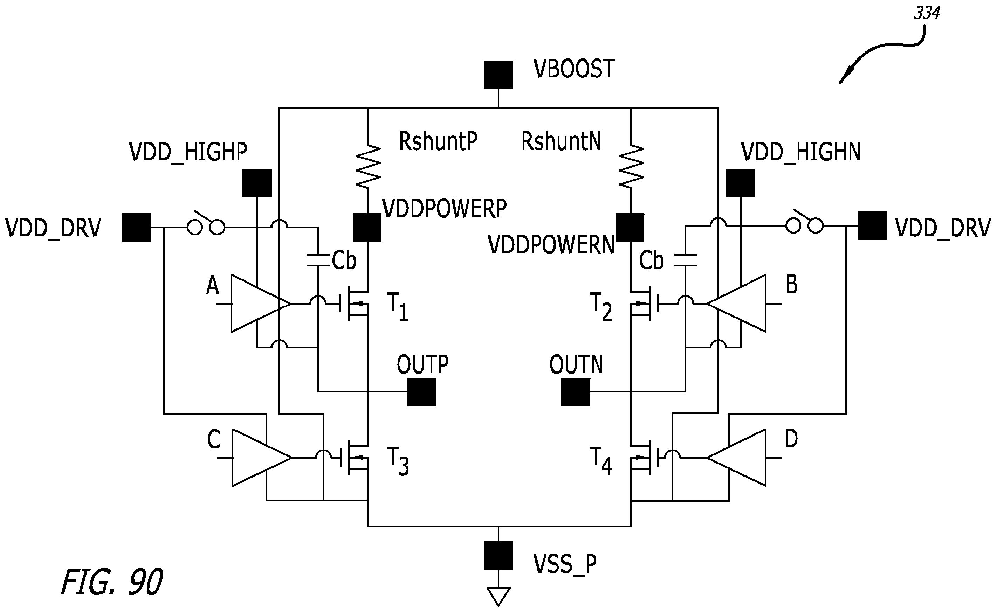

Referring now to Figure 89 of the accompanying drawings, the bridge IC 301 is a microchip which comprises an embedded power switching circuit 333. In this example, the power switching circuit 333 is an H-bridge 334 which is shown in Figure 90 and which is described in detail below. It is, however, to be appreciated that the bridge IC 301 of other examples may incorporate an alternative power switching circuit to the H-bridge 334, provided that the power switching circuit performs an equivalent function for generating an AC drive signal to drive the ultrasonic transducer 215.

The bridge IC 301 comprises a first phase terminal PHASE A which receives a first phase output signal Phase A from the PWM signal generator subsystem of the PMIC 300. The bridge IC 301 also comprises a second phase terminal PHASE B which receives a second phase output signal Phase B from the PWM signal generator subsystem of the PMIC 300.

The bridge IC 301 comprises a current sensing circuit 335 which senses current flow in the H-bridge 334 directly and provides an RMS current output signal via the RMS_CURR pin of the bridge IC 301. The current sensing circuit 335 is configured for over current monitoring, to detect when the current flowing in the H-bridge 334 is above a predetermined threshold. The integration of the power switching circuit 333 comprising the H-bridge 334 and the current sensing circuit 335 all within the same embedded circuit of the bridge IC 301 is a unique combination in the IC market. At present, no other integrated circuit in the IC market comprises an H-bridge with embedded circuitry for sensing the RMS current flowing through the H-bridge.

The bridge IC 301 comprises a temperature sensor 336 which includes over temperature monitoring. The temperature sensor 336 is configured to shut down the bridge IC 301 or disable at least part of the bridge IC 336 in the event that the temperature sensor 336 detects that the bridge IC 301 is operating at a temperature above a predetermined threshold. The temperature sensor 336 therefore provides an integrated safety function which prevents damage to the bridge IC 301 or other components within the driver 202 in the event that the bridge IC 301 operates at an excessively high temperature.

The bridge IC 301 comprises a digital state machine 337 which is integrally connected to the power switching circuit 333. The digital state machine 337 receives the phase A and phase B signals from the PMIC 300 and an ENABLE signal, for instance from the microcontroller 303. The digital state machine 337 generates timing signals based on the first phase output signal Phase A and the second phase output signal Phase B.

The digital state machine 337 outputs timing signals corresponding to the phase A and phase B signals as well as a BRIDGE_PR and BRIDGE_EN signals to the power switching circuit 333 in order to control the power switching circuit 333. The digital state machine 337 thus outputs the timing signals to the switches $T_1$-$T_4$ of the H-bridge circuit 334 to control the switches $T_1$-$T_4$ to turn on and off in a sequence such that the H-bridge circuit outputs an AC drive signal for driving a resonant circuit, such as the ultrasonic transducer 215.

As described in more detail below, the switching sequence comprises a free-float period in which the first switch $T_1$ and the second switch $T_2$ are turned off and the third switch $T_3$ and the fourth switch $T_4$ are turned on in order to dissipate energy stored by the resonant circuit (the ultrasonic transducer 215).

The bridge IC 301 comprises a test controller 338 which enables the bridge IC 301 to be tested to determine whether the embedded components within the bridge IC 301 are operating correctly. The test controller 338 is coupled to TEST_DATA, TEST_CLK and TEST_LOAD pins so that the bridge IC 301 can be connected to an external control device which feeds data into and out from the bridge IC 301 to test the operation of the bridge IC 301. The bridge IC 301 also comprises a TEST BUS which enables the digital communication bus within the bridge IC 301 to be tested via a TST_PAD pin.

The bridge IC 301 comprises a power on reset circuit (POR) 339 which controls the startup operation of the bridge IC 301. The POR 339 ensures that the bridge IC 301 starts up properly only if the supply voltage is within a predetermined range. If the power supply voltage is outside of the predetermined range, for instance if the power supply voltage is too high, the POR 339 delays the startup of the bridge IC 301 until the supply voltage is within the predetermined range.

The bridge IC 301 comprises a reference block (BG) 340 which provides a precise reference voltage for use by the other subsystems of the bridge IC 301.

The bridge IC 301 comprises a current reference 341 which provides a precise current to the power switching circuit 333 and/or other subsystems within the bridge IC 301, such as the current sensor 335.

The temperature sensor 336 monitors the temperature of the silicon of the bridge IC 301 continuously. If the temperature exceeds the predetermined temperature threshold, the power switching circuit 333 is switched off automatically. In addition, the over temperature may be reported to an external host to inform the external host that an over temperature event has occurred.

The digital state machine (FSM) 337 generates the timing signals for the power switching circuit 333 which, in this example, are timing signals for controlling the H-bridge 334.

The bridge IC 301 comprises comparators 342,343 which compare signals from the various subsystems of the bridge IC 301 with the voltage and current references 340,341 and provide reference output signals via the pins of the bridge IC 301.

Referring again to Figure 90 of the accompanying drawings, the H-bridge 334 of this example comprises four switches in the form of NMOS field effect transistors (FET) switches on both sides of the H-bridge 334. The H-bridge 334 comprises four switches or transistors $T_1$-$T_4$ which are connected in an H-bridge configuration, with each transistor $T_1$-$T_4$ being driven by a respective logic input A-D. The transistors $T_1$-$T_4$ are configured to be driven by a bootstrap voltage which is generated internally with two external capacitors Cb which are connected as illustrated in Figure 90.

The H-bridge 334 comprises various power inputs and outputs which are connected to the respective pins of the bridge IC 301. The H-bridge 334 receives the programmable voltage VBOOST which is output from the boost converter 305 via a first power supply terminal, labelled VBOOST in Figure 90. The H-bridge 334 comprises a second power supply terminal, labelled VSS_P in Figure 90.

The H-bridge 334 comprises outputs OUTP, OUTN which are configured to connect to respective terminals of the ultrasonic transducer 215 so that the AC drive signal output from the H-bridge 334 can drive the ultrasonic transducer 215.

The switching of the four switches or transistors $T_1$-$T_4$ is controlled by switching signals from the digital state machine 337 via the logic input A-D. It is to be appreciated that, while Figure 90 shows four transistors $T_1$-$T_4$, in other examples, the H-bridge 334 incorporates a larger number of transistors or other switching components to implement the functionality of the H-bridge.

In this example, the H-bridge 334 operates at a switching power of 22 W to 50 W in order to deliver an AC drive signal with sufficient power to drive the ultrasonic transducer 215 to generate mist optimally. The voltage which is switched by the H-bridge 334 of this example is ±15 V. In other examples, the voltage is ±20 V.

In this example, the H-bridge 334 switches at a frequency of 3 MHz to 5 MHz or up to 105 MHz. This is a high switching speed compared with conventional integrated circuit H-bridges which are available in the IC market. For instance, a conventional integrated circuit H-bridge available in the IC market today is configured to operate at a maximum frequency of only 2 MHz. Aside from the bridge IC 301 described herein, no conventional integrated circuit H-bridge available in the IC market is able to operate at a power of 22 V to 50 V at a frequency of up to 5 MHz, let alone up to 105 MHz.

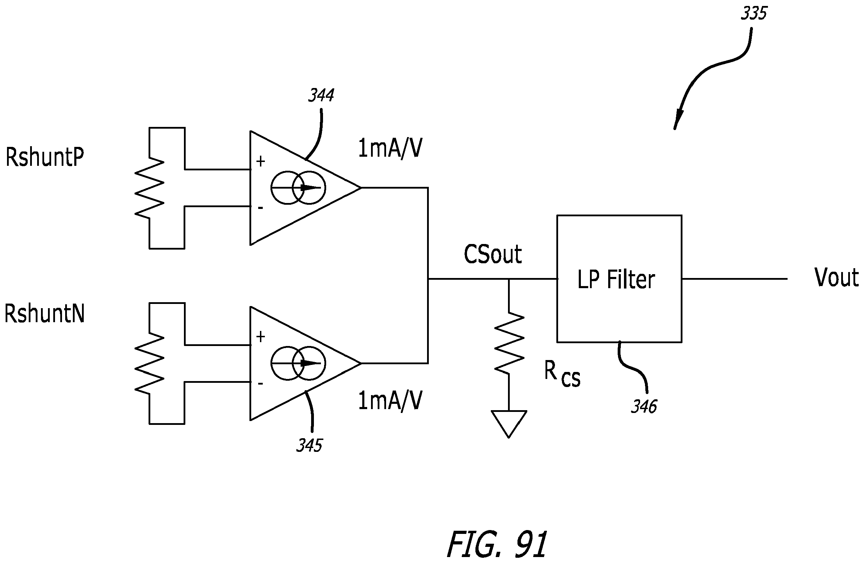

Referring now to Figure 91 of the accompanying drawings, the current sensor 335 comprises positive and negative current sense resistors RshuntP, RshuntN which are connected in series with the respective high and low sides of the H-bridge 334, as shown in Figure 90. The current sense resistors RshuntP, RshuntN are low value resistors which, in this example, are 0.1 Ω. The current sensor 335 comprises a first voltage sensor in the form of a first operational amplifier 344 which measures the voltage drop across the first current sensor resistor RshuntP and a second voltage sensor in the form of a second operational amplifier 345 which measures the voltage drop across the second current sensor resistor RshuntN. In this example, the gain of each operational amplifier 344, 345 is 2V/V. The output of each operational amplifier 344, 345 is, in this example, 1mA/V. The current sensor 335 comprises a pull down resistor $R_{cs}$ which, in this example, is 2kΩ. The outputs of the operational amplifiers 344, 345 provide an output CSout which passes through a low pass filter 346 which removes transients in the signal CSout. An output Vout of the low pass filter 346 is the output signal of the current sensor 335.

The current sensor 335 thus measures the AC current flowing through the H-bridge 334 and respectively through the ultrasonic transducer 215. The current sensor 335 translates the AC current into an equivalent RMS output voltage (Vout) relative to ground. The current sensor 335 has high bandwidth capability since the H-bridge 334 can be operated at a frequency of up to 5 MHz or, in some examples, up to 105 MHz. The output Vout of the current sensor 335 reports a positive voltage which is equivalent to the measured AC rms current flowing through the ultrasonic transducer 215. The output voltage Vout of the current sensor 335 is, in this example, fed back to the control circuitry within the bridge IC 301 to enable the bridge IC 301 to shut down the H-bridge 334 in the event that the current flowing through the H-bridge 334 and hence through the transducer 215 is in excess of a predetermined threshold. In addition, the over current threshold event is reported to the first comparator 342 in the bridge IC 301 so that the bridge IC 301 can report the over current event via the OVC_TRIGG pin of the bridge IC 301.

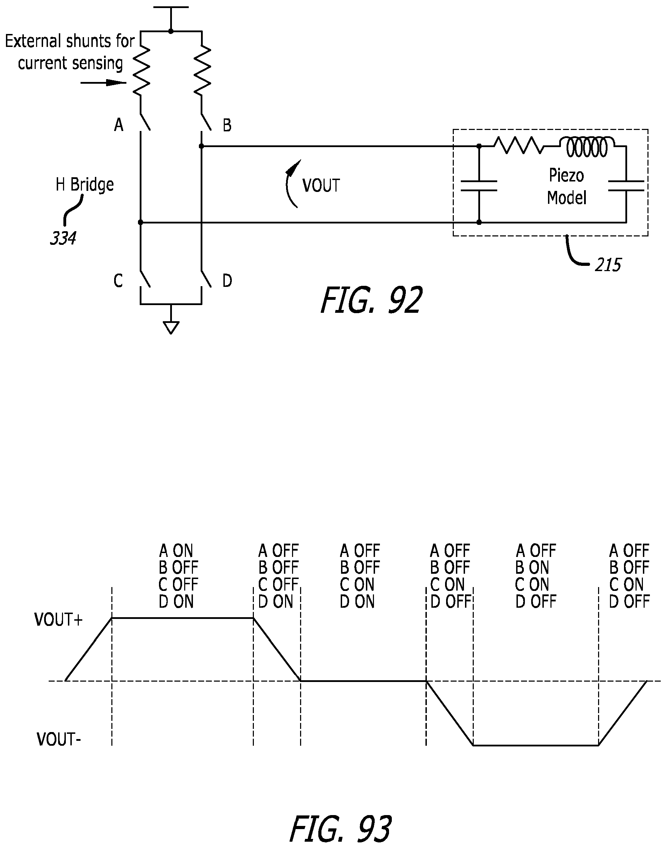

Referring now to Figure 92 of the accompanying drawings, the control of the H-bridge 334 will now be described also with reference to the equivalent piezoelectric model of the ultrasonic transducer 215.

To develop a positive voltage across the outputs OUTP, OUTN of the H-bridge 334 as indicated by V_out in Figure 92 (note the direction of the arrow) the switching sequence of the transistors $T_1$-$T_4$ via the inputs A-D is as follows:

1. Positive output voltage across the ultrasonic transducer 215: A-ON, B-OFF, C-OFF, D-ON
2. Transition from positive output voltage to zero: A-OFF, B-OFF, C-OFF, D-ON. During this transition, C is switched off first to minimise or avoid power loss by minimising or avoiding current flowing through A and C if there is a switching error or delay in A.
3. Zero output voltage: A-OFF, B-OFF, C-ON, D-ON. During this zero output voltage phase, the terminals of the outputs OUTP, OUTN of the H-bridge 334 are grounded by the C and D switches which remain on. This dissipates the energy stored by the capacitors in the equivalent circuit of the ultrasonic transducer, which minimises the voltage overshoot in the switching waveform voltage which is applied to the ultrasonic transducer.
4. Transition from zero to negative output voltage: A-OFF, B-OFF, C-ON, D-OFF.
5. Negative output voltage across the ultrasonic transducer 215: A-OFF, B-ON, CON, D-OFF

At high frequencies of up to 5 MHz or even up to 105 MHz, it will be appreciated that the time for each part of the switching sequence is very short and in the order of nanoseconds or picoseconds. For instance, at a switching frequency of 6 MHz, each part of the switching sequence occurs in approximately 80 ns.

A graph showing the output voltage OUTP, OUTN of the H-bridge 334 according to the above switching sequence is shown in Figure 93 of the accompanying drawings. The zero output voltage portion of the switching sequence is included to accommodate for the energy stored by the ultrasonic transducer 215 (e.g. the energy stored by the capacitors in the equivalent circuit of the ultrasonic transducer). As described above, this minimises the voltage overshoot in the switching waveform voltage which is applied to the ultrasonic transducer and hence minimises unnecessary power dissipation and heating in the ultrasonic transducer.

Minimising or removing voltage overshoot also reduces the risk of damage to transistors in the bridge IC 301 by preventing the transistors from being subject to voltages in excess of their rated voltage. Furthermore, the minimisation or removal of the voltage overshoot enables the bridge IC 301 to drive the ultrasonic transducer accurately in a way which minimises disruption to the current sense feedback loop described herein. Consequently, the bridge IC 301 is able to drive the ultrasonic transducer at a high power of 22 W to 50 W or even as high as 70 W at a high frequency of up to 5 MHz or even up to 105 MHz.

The bridge IC 301 of this example is configured to be controlled by the PMIC 300 to operate in two different modes, referred to herein as a forced mode and a native frequency mode. These two modes of operation are novel over existing bridge ICs. In particular, the native frequency mode is a major innovation which offers substantial benefits in the accuracy and efficiency of driving an ultrasonic transducer as compared with conventional devices.

**Forced Frequency Mode (FFM)**

In the forced frequency mode the H-bridge 334 is controlled in the sequence described above but at a user selectable frequency. As a consequence, the H-bridge transistors $T_1$-$T_4$ are controlled in a forced way irrespective of the inherent resonant frequency of the ultrasonic transducer 215 to switch the output voltage across the ultrasonic transducer 215. The forced frequency mode therefore allows the H-bridge 334 to drive the ultrasonic transducer 215, which has a resonant frequency f1, at different frequency f2.

Driving an ultrasonic transducer at a frequency which is different from its resonant frequency may be appropriate in order to adapt the operation to different applications. For example, it may be appropriate to drive an ultrasonic transducer at a frequency which is slightly off the resonance frequency (for mechanical reasons to prevent mechanical damage to the transducer). Alternatively, it may be appropriate to drive an ultrasonic transducer at a low frequency but the ultrasonic transducer has, because of its size, a different native resonance frequency.

The driver 202 controls the bridge IC 301 to drive the ultrasonic transducer 215 in the forced frequency mode in response to the configuration of the driver 202 for a particular application or a particular ultrasonic transducer. For instance, the driver 202 may be configured to operate in the forced frequency mode when the mist inhaler 200 is being used for a particular application, such as generating a mist from a liquid of a particular viscosity containing a drug for delivery to a user.

**Native Frequency Mode (NFM)**

The following native frequency mode of operation is a significant development and provides benefits in improved accuracy and efficiency over conventional ultrasonic drivers that are available on the IC market today.

The native frequency mode of operation follows the same switching sequence as described above but the timing of the zero output portion of the sequence is adjusted to minimise or avoid problems that can occur due to current spikes in the forced frequency mode operation. These current spikes occur when the voltage across the ultrasonic transducer 215 is switched to its opposite voltage polarity. An ultrasonic transducer which comprises a piezoelectric crystal has an electrical equivalent circuit which incorporates a parallel connected capacitor (e.g. see the piezo model in Figure 92). If the voltage across the ultrasonic transducer is hard-switched from a positive voltage to a negative voltage, due to the high dV/dt there can be a large current flow current flow as the energy stored in the capacitor dissipates.

The native frequency mode avoids hard switching the voltage across the ultrasonic transducer 215 from a positive voltage to a negative voltage (and vice versa). Instead, prior to applying the reversed voltage, the ultrasonic transducer 215 (piezoelectric crystal) is left free-floating with zero voltage applied across its terminals for a free-float period. The PMIC 300 sets the drive frequency of the bridge IC 301 such that the bridge 334 sets the free-float period such that current flow inside the ultrasonic transducer 215 (due to the energy stored within the piezoelectric crystal) reverses the voltage across the terminals of the ultrasonic transducer 215 during the free-float period.

Consequently, when the H-bridge 334 applies the negative voltage at the terminals of the ultrasonic transducer 215 the ultrasonic transducer 215 (the capacitor in the equivalent circuit) has already been reverse charged and no current spikes occur because there is no high dV/dt.

It is, however, to be appreciated that it takes time for the charge within the ultrasonic transducer 215 (piezoelectric crystal) to build up when the ultrasonic transducer 215 is first activated. Therefore, the ideal situation in which the energy within the ultrasonic transducer 215 is to reverse the voltage during the free-float period occurs only after the oscillation inside the ultrasonic transducer 215 has built up the charge. To accommodate for this, when the bridge IC 301 activates the ultrasonic transducer 215 for the first time, the PMIC 300 controls the power delivered through the H-bridge 334 to the ultrasonic transducer 215 to a first value which is a low value (e.g. 5 V). The PMIC 300 then controls the power delivered through the H-bridge 334 to the ultrasonic transducer 215 to increase over a period of time to a second value (e.g. 15 V) which is higher than the first value in order to build up the energy stored within the ultrasonic transducer 215. Current spikes still occur during this ramp of the oscillation until the current inside the ultrasonic transducer 215 developed sufficiently. However, by using a low first voltage at start up those current spikes are kept sufficiently low to minimise the impact on the operation of the ultrasonic transducer 215.

In order to implement the native frequency mode, the driver 202 controls the frequency of the oscillator 315 and the duty cycle (ratio of turn-on time to free-float time) of the AC drive signal output from the H-bridge 334 with high precision. In this example, the driver 202 performs three control loops to regulate the oscillator frequency and the duty cycle such that the voltage reversal at the terminals of the ultrasonic transducer 215 is as precise as possible and current spikes are minimised or avoided as far as possible. The precise control of the oscillator and the duty cycle using the control loops is a significant advance in the field of IC ultrasonic drivers.

During the native frequency mode of operation, the current sensor 335 senses the current flowing through the ultrasonic transducer 215 (resonant circuit) during the free-float period. The digital state machine 337 adapts the timing signals to switch on either the first switch $T_1$ or the second switch $T_2$ when the current sensor 335 senses that the current flowing through the ultrasonic transducer 215 (resonant circuit) during the free-float period is zero.

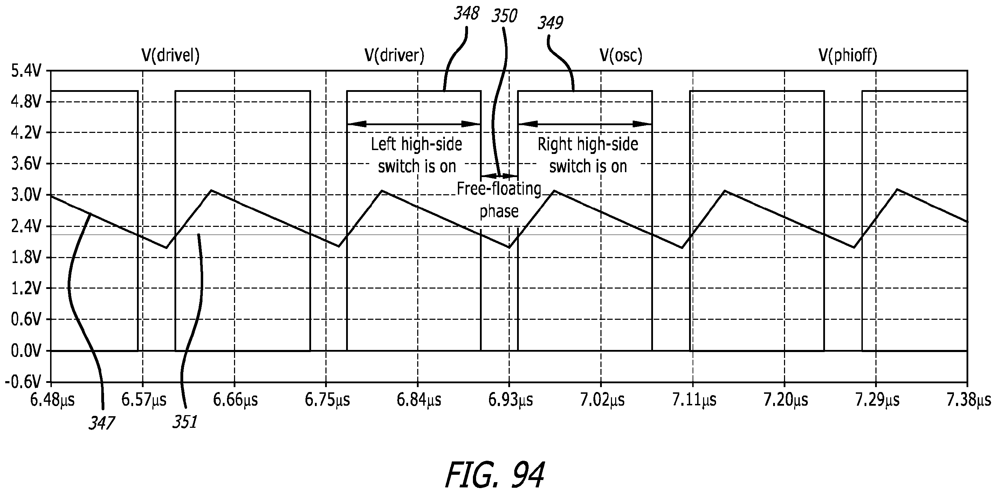

Figure 94 of the accompanying drawings shows the oscillator voltage waveform 347 (V(osc)), a switching waveform 348 resulting from the turn-on and turn-off the left hand side high switch $T_1$ of the H-bridge 334 and a switching waveform 349 resulting from the turn-on and turn-off the right hand side high switch $T_2$ of the H-bridge 334. For an intervening free-float period 350, both high switches $T_1$, $T_2$ of the H-bridge 334 are turned off (free-floating phase). The duration of the free-float period 350 is controlled by the magnitude of the free-float control voltage 351 (Vphioff).

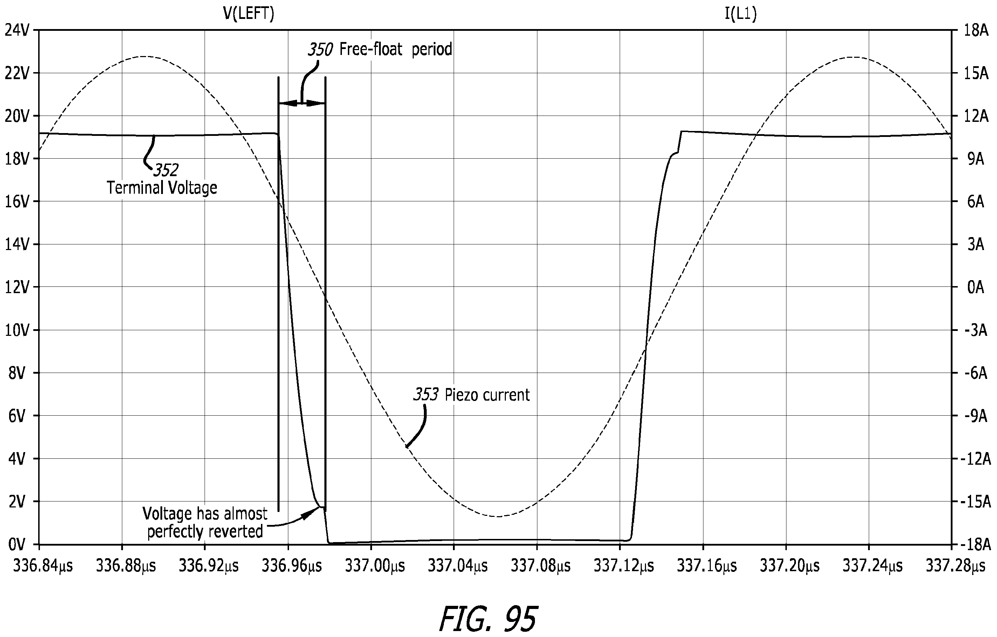

Figure 95 of the accompanying drawings shows the voltage waveform 352 at a first terminal of the ultrasonic transducer 215 (the voltage waveform is reversed at the second terminal of the ultrasonic transducer 215) and the piezo current 353 flowing through the ultrasonic transducer 215. The piezo current 353 represents an (almost) ideal sinusoidal waveform (this is never possible in the forced frequency mode or in any bridge in the IC market).

Before the sinusoidal wave of the piezo current 353 reaches zero, the left hand side high switch $T_1$ of the H-bridge 334 is turned off (here, the switch $T_1$ is turned off when the piezo current 353 is approximately 6 A). The remaining piezo current 353 which flows within the ultrasonic transducer 215 due to the energy stored in the ultrasonic transducer 215 (the capacitor of the piezo equivalent circuit) is responsible for the voltage reversal during the free-float period 350. The piezo current 353 decays to zero during the free-float period 350 and into negative current flow domain thereafter. The terminal voltage at the ultrasonic transducer 215 drops from the supply voltage (in this case 19 V) to less than 2 V and the drop comes to a stop when the piezo current 353 reaches zero. This is the perfect time to turn on the low-side switch $T_3$ of the H-bridge 334 in order to minimise or avoid a current spike.

Compared to the forced frequency mode described above, the native frequency mode has at least three advantages:

1. The current spike associated with hard switching of the package capacitor is significantly reduced or avoided completely.
2. Power loss due to hard switching is almost eliminated.
3. Frequency is regulated by the control loops and will be kept close to the resonance of the piezo crystal (i.e. the native resonance frequency of the piezo crystal).

In the case of the frequency regulation by the control loops (advantage 3 above), the PMIC 300 starts by controlling the bridge IC 301 to drive the ultrasonic transducer 215 at a frequency above the resonance of the piezo crystal. The PMIC 300 then controls the bridge IC 301 to that the frequency of the AC drive signal decays/reduces during start up. As soon as the frequency approaches resonance frequency of the piezo crystal, the piezo current will develop/increase rapidly. Once the piezo current is high enough to cause the desired voltage reversal, the frequency decay/reduction is stopped by the PMIC 300. The control loops of the PMIC 300 then take over the regulation of frequency and duty cycle of the AC drive signal.

In the forced frequency mode, the power delivered to the ultrasonic transducer 215 is controlled through the duty cycle and/or a frequency shift and/or by varying the supply voltage. However, in this example in the native frequency mode the power delivered to the ultrasonic transducer 215 controlled only through the supply voltage.

In this example, during a setup phase of operation of the driver, the bridge IC 301 is configured to measure the length of time taken for the current flowing through the ultrasonic transducer 215 (resonant circuit) to fall to zero when the first switch $T_1$ and the second switch $T_2$ are turned off and the third switch $T_3$ and the fourth switch $T_4$ are turned on. The bridge IC 301 then sets the length of time of the free-float period to be equal to the measured length of time.

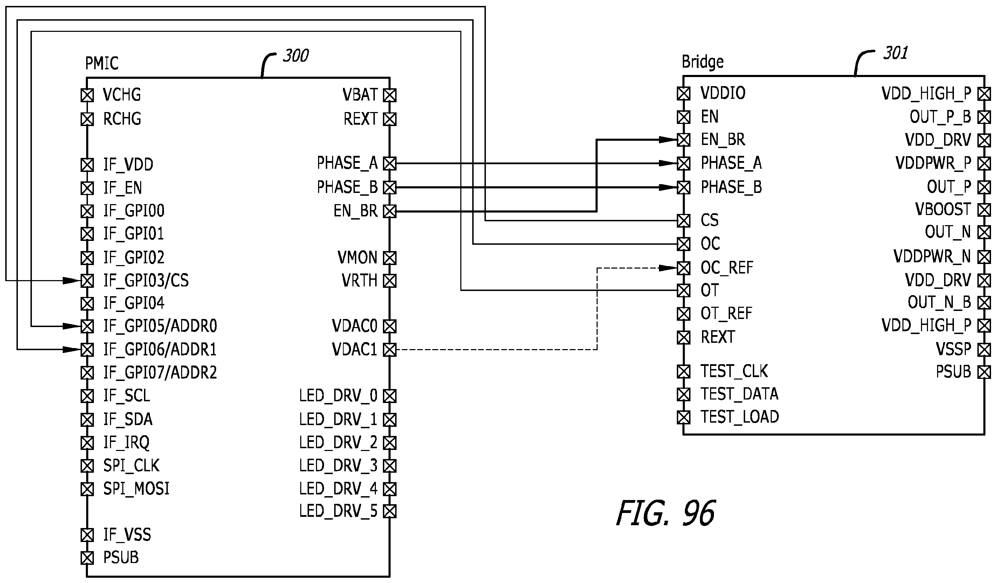

Referring now to Figure 96 of the accompanying drawings, the PMIC 300 and the bridge IC 301 of this example are designed to work together as a companion chip set. The PMIC 300 and the bridge IC 301 are connected together electrically for communication with one another. In this example, there are interconnections between the PMIC 300 and the bridge IC 301 which enable the following two categories of communication:

1. control signals
2. feedback signals

The connections between the PHASE_A and PHASE_B pins of the PMIC 300 and the bridge IC 301 carry the PWM modulated control signals which drive the H-bridge 334. The connection between the EN_BR pins of the PMIC 300 and the bridge IC 301 carries the EN_BR control signal which triggers the start of the H-bridge 334. The timing between the PHASE_A, PHASE_B and EN_BR control signals is important and handled by the digital bridge control of the PMIC 300.

The connections between the CS, OC and OT pins of the PMIC 300 and the bridge IC 301 carry CS (current sense), OC (over current) and OT (over temperature) feedback signals from the bridge IC 301 back to the PMIC 300. Most notably, the CS (current sense) feedback signal comprises a voltage equivalent to the rms current flowing through the ultrasonic transducer 215 which is measured by the current sensor 335 of the bridge IC 301.

The OC (over current) and OT (over temperature) feedback signals are digital signals indicating that either an over current or an over voltage event has been detected by the bridge IC 301. In this example, the thresholds for the over current and over temperature are set with an external resistor. Alternatively, the thresholds can also be dynamically set in response to signals passed to the OC_REF pin of the bridge IC 301 from one of the two DAC channels VDAC0, VDAC1 from the PMIC 300.

In this example, the design of the PMIC 300 and the bridge IC 301 allow the pins of these two integrated circuits to be connected directly to one another (e.g. via copper tracks on a PCB) so that there is minimal or no lag in the communication of signals between the PMIC 300 and the bridge IC 301. This provides a significant speed advantage over conventional bridges in the IC market which are typically controlled by signals via a digital communications bus. For example, a standard I2C bus is clocked at only 400 kHz, which is too slow for communicating data sampled at the high clock speeds of up to 5 MHz of examples of this disclosure.

While examples of this disclosure have been described above in relation to the microchip hardware, it is to be appreciated that other examples of this disclosure comprise a method of operating the components and subsystems of each microchip to perform the functions described herein. For instance, the methods of operating the PMIC 300 and the bridge IC 301 in either the forced frequency mode or the native frequency mode.

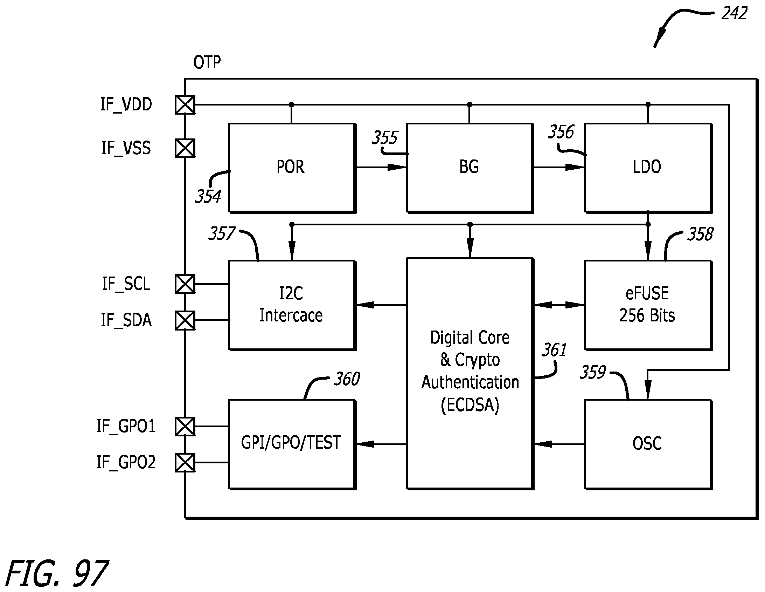

Referring now to Figure 97 of the accompanying drawings, the OTP IC 242 comprises a power on reset circuit (POR) 354, a bandgap reference (BG) 355, a cap-less low dropout regulator (LDO) 356, a communication (e.g. I2C) interface 357, a one-time programmable memory bank (eFuse) 358, an oscillator 359 and a general purpose input-output interface 360. The OTP IC 242 also comprises a digital core 361 which includes a cryptographic authenticator. In this example, the cryptographic authenticator uses the Elliptic Curve Digital Signature Algorithm (ECDSA) for encrypting/decrypting data stored within the OTP IC 242 as well as data transmitted to and from the OTP IC 242.

The POR 354 ensures that the OTP IC 242 starts up properly only if the supply voltage is within a predetermined range. If the supply voltage is outside the predetermined range, the POR 354 resets the OTP IC 242 and waits until the supply voltage is within the predetermined range.

The BG 355 provides precise reference voltages and currents to the LDO 356 and to the oscillator 359. The LDO 356 supplies the digital core 361, the communication interface 357 and the eFuse memory bank 358.

The OTP IC 242 is configured to operate in at least the following modes:

- Fuse Programming (Fusing): During efuse programming (programming of the one time programmable memory) a high current is required to burn the relevant fuses within the eFuse memory bank 358. In this mode higher bias currents are provided to maintain gain and bandwidth of the regulation loop.
- Fuse Reading: In this mode a medium level current is required to maintain efuse reading within the eFuse memory bank 358. This mode is executed during the startup of the OTP IC 242 to transfer the content of the fuses to shadow registers. In this mode the gain and bandwidth of the regulation loop is set to a lower value than in the Fusing Mode.
- Normal Operation: In this mode the LDO 356 is driven in a very low bias current condition to operate the OTP IC 242 with low power so that the OTP IC 242 consumes as little power as possible.

The oscillator 359 provides the required clock for the digital core/engine 361 during testing (SCAN Test), during fusing and during normal operation. The oscillator 359 is trimmed to cope with the strict timing requirements during the fusing mode.

In this example, the communication interface 357 is compliant with the FM+ specification of the I2C standard but it also complies with slow and fast mode. The OTP IC 242 uses the communication interface 357 to communicate with the driver 202 (the Host) for data and key exchange.

The digital core 361 implements the control and communication functionality of the OTP IC 242. The cryptographic authenticator of the digital core 361 enables the OTP IC 242 to authenticate itself (e.g. using ECDSA encrypted messages) with the driver 202 (e.g. for a particular application) to ensure that the OTP IC 242 is genuine and that the OTP IC 242 is authorised to connect to the driver 202 (or another product).

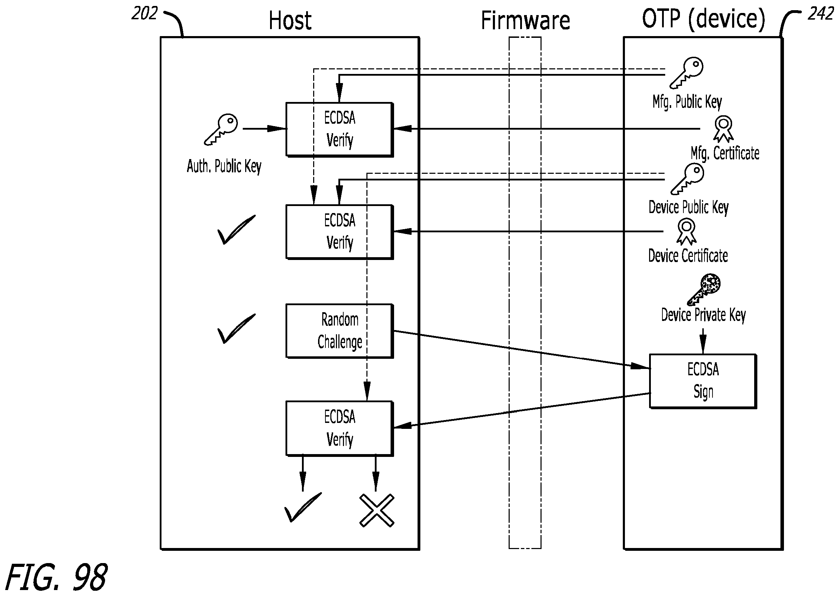

With reference to Figure 98 of the accompanying drawings, the OTP IC 242 performs the following PKI procedure in order to authenticate the OTP IC 242 for use with a Host (e.g. the driver 202):

1. Verify Signer Public Key: The Host requests the Manufacturing Public key and Certificate. The Host verifies the certificate with the Authority Public key.
2. Verify Device Public Key: If the verification is successful, the Host requests the Device Public key and Certificate. The Host verifies the certificate with the Manufacturing Public key.
3. Challenge - Response: If the verification is successful, the Host creates a random number challenge and sends it to the Device. The End Product signs the random number challenge with the Device Private key.
4. The signature is sent back to the Host for verification using the Device Public key.

If all steps of the authentication procedure complete successfully then the Chain of Trust has been verified back to the Root of Trust and the OTP IC 242 is successfully authenticated for use with the Host. However, if any of the steps of the authentication procedure fail then the OTP IC 242 is not authenticated for use with the Host and use of the device incorporating the OTP IC 242 is restricted or prevented.

The driver comprises an AC driver for converting a voltage from the battery into an AC drive signal at a predetermined frequency to drive the ultrasonic transducer.

The driver comprises an active power monitoring arrangement for monitoring the active power used by the ultrasonic transducer (as described above) when the ultrasonic transducer is driven by the AC drive signal. The active power monitoring arrangement provides a monitoring signal which is indicative of an active power used by the ultrasonic transducer.

The processor within the driver controls the AC driver and receives the monitoring signal drive from the active power monitoring arrangement.

The memory of driver stores instructions which, when executed by the processor, cause the processor to:

A. control the AC driver to output an AC drive signal to the ultrasonic transducer at a predetermined sweep frequency;

B. calculate the active power being used by the ultrasonic transducer based on the monitoring signal;

C. control the AC driver to modulate the AC drive signal to maximise the active power being used by the ultrasonic transducer;

D. store a record in the memory of the maximum active power used by the ultrasonic transducer and the sweep frequency of the AC drive signal;

E. repeat steps A-D for a predetermined number of iterations with the sweep frequency incrementing or decrementing with each iteration such that, after the predetermined number of iterations has occurred, the sweep frequency has been incremented or decremented from a start sweep frequency to an end sweep frequency;

F. identify from the records stored in the memory the optimum frequency for the AC drive signal which is the sweep frequency of the AC drive signal at which a maximum active power is used by the ultrasonic transducer; and

G. control the AC driver to output an AC drive signal to the ultrasonic transducer at the optimum frequency to drive the ultrasonic transducer to atomise a liquid.

In some examples, the active power monitoring arrangement comprises a current sensing arrangement for sensing a drive current of the AC drive signal driving the ultrasonic transducer, wherein the active power monitoring arrangement provides a monitoring signal which is indicative of the sensed drive current.

In some examples, the current sensing arrangement comprises an Analog-to-Digital Converter which converts the sensed drive current into a digital signal for processing by the processor.

In some examples, the memory stores instructions which, when executed by the processor, cause the processor to: repeat steps A-D above with the sweep frequency being incremented from a start sweep frequency of 2900kHz to an end sweep frequency of 2960kHz.

In some examples, the memory stores instructions which, when executed by the processor, cause the processor to: repeat steps A-D above with the sweep frequency being incremented from a start sweep frequency of 2900kHz to an end sweep frequency of 3100kHz.

In some examples, the memory stores instructions which, when executed by the processor, cause the processor to: in step G, control the AC driver to output an AC drive signal to the ultrasonic transducer at frequency which is shifted by a predetermined shift amount from the optimum frequency.

In some examples, the predetermined shift amount is between 1-10% of the optimum frequency.

## 2. Control and Information (CI) section

The Control and Information section comprises an external EEPROM for data storage, LEDs for user indications, a pressure sensor for airflow detection and a Bluetooth Low Energy (BLE) capable microcontroller for constant monitoring and managing of the aerosolisation section.

The pressure sensor used in the device serves two purposes. The first purpose is to prevent unwanted and accidental start of the sonic engine (driving the ultrasonic transducer). This functionality is implemented in the processing arrangement of the device, but optimised for low power, to constantly measures environmental parameters such as temperature and ambient pressure with internal compensation and reference setting in order to accurately detect and categorise what is called a true inhalation.

Unlike all the other mist generator devices on the market, this solution uses the strength of a micro-controller to allow the use of only one sensor.

The second purpose of the pressure sensor is to be able to monitor not only the exact duration of the inhalations by the user for precise inhalation volume measurement, but also to be able to determine the strength of the user inhalation which is a critical information in medical conditions both for proper prescription and health monitoring. All in all, we are able to completely draw the pressure profile of every inhalation and anticipate the end of an inhalation for both aerosolisation optimisation and medical data behaviour.

This was possible with the usage of a Bluetooth™ Low Energy (BLE) microcontroller. Indeed, this enables the setting to provide extremely accurate inhalation times, optimised aerosolisation, monitor numerous parameters to guarantee safe misting and prevent the use of non-genuine e-liquids or aerosol chambers and protect both the device against over-heating risks and the user against over-misting in one shot unlike any other products on the market.

The use of the BLE microcontroller allows over-the-air update to continuously provide improved software to users based on anonymised data collection and trained AI for PZT modelling.

## 3. Power Management (PM) section

The Power Management section is constituted by the 3.7V LiPo battery path to a low dropout regulator (LDO) that powers the Control and Information section and a battery management system (BMS) that provides high level of protection and charging to the internal LiPo battery.

The components in this section have been selected carefully and thoroughly to be able to provide such an integrated and compact device while providing high power to the sonication section and ensuring a steady powering of the control and information section.

Indeed, when providing high power to the aerosolisation section from a 3.7V LiPo battery, the supply voltage varies a lot during operation. Without a low dropout regulator, the Control and Information section could not be powered with a mandatory steady supply when the battery voltage drops to as low as 0.3V above the minimum ratings of the components in this section, which is why the LDO plays a crucial role here. A loss in the CI section would disturb or even stop the functioning of the entire device.

This is why the careful selection of components not only ensures high reliability of the device but also allows it to work under harsh conditions and for a longer consecutive time between recharge.

### Controlled Aerosolisation

The device is a precise, reliable and a safe aerosolisation solution for medical prescription and daily customer usage and, as such, must provide a controlled and trusted aerosolisation.

This is performed through an internal method that can be broken apart into several sections as follows:

### 1. Sonication

In order to provide the most optimal aerosolisation the ultrasonic transducer (PZT) needs to vibrate in the most efficient way.

### Frequency

The electromechanical properties of piezoelectrical ceramics state that the component has the most efficiency at the resonant frequency. But also, vibrating a PZT at resonance for a long duration will inevitably end with the failure and breaking of the component which renders the aerosol chamber unusable.

Another important point to consider when using piezoelectrical materials is the inherent variability during manufacturing and its variability over temperature and lifetime.

Resonating a PZT at 3MHz in order to create droplets of a size <1um requires an adaptive method in order to locate and target the 'sweet spot' of the particular PZT inside every aerosol chamber used with the device for every single inhalation.

### Sweep

Because the device has to locate the 'sweet spot' for every single inhalation and because of over-usage, the PZT temperature varies as the device uses an in-house double sweep method.

The first sweep is used when the device has not been used with a particular aerosol chamber for a time that is considered enough for all the thermal dissipation to occur and for the PZT to cool down to 'default temperature'. This procedure is also called a cold start. During this procedure the PZT needs a boost in order to produce the required aerosol. This is achieved by only going over a small subset of Frequencies between 2900kHz to 2960kHz which, considering extensive studies and experiments, covers the resonant point.

For each frequency in this range, the sonic engine in activated and the current going through the PZT is actively monitored and stored by the microcontroller via an Analog-to-Digital Converter (ADC), and converted back to current in order to be able to precisely deduct the Power used by the PZT.

This yields the cold profile of this PZT regarding frequency and the Frequency used throughout the inhalation is the one that uses the most current, meaning the lowest impedance Frequency.

The second sweep is performed during any subsequent inhalation and cover the entire range of frequencies between 2900kHz to 3100kHz due to the modification of the PZT profile with regards to temperature and deformation. This hot profile is used to determine the shift to apply.

### Shift

Because the aerosolisation must be optimal, the shift is not used during any cold inhalation and the PZT will hence vibrate at resonant frequency. This can only happen for a short and unrepeated duration of time otherwise the PZT would inevitably break.

The shift however is used during most of inhalations as a way to still target a low impedance frequency, thus resulting in quasi-optimal operation of the PZT while protecting it against failures.

Because the hot and cold profiles are stored during inhalation the microcontroller can then select the proper shifted frequency according to the measured values of current through the PZT during sweep and ensure a safe mechanical operation.

The selection of the direction to shift is crucial as the piezoelectrical component behaves in a different way if outside the duplet resonant/anti-resonant frequency or inside this range. The selected shift should always be in this range defined by Resonant to anti-Resonant frequencies as the PZT is inductive and not capacitive.

Finally, the percentage to shift is maintained below 10% in order to still remain close to the lowest impedance but far enough of the resonance.

## Adjustment

Because of the intrinsic nature of PZTs, every inhalation is different. Numerous parameters other than the piezoelectrical element influence the outcome of the inhalation, like the amount of e-liquid remaining inside the aerosol chamber, the wicking state of the gauze or the battery level of the device.

As of this, the device permanently monitors the current used by the PZT inside the aerosol chamber and the microcontroller constantly adjusts the parameters such as the frequency and the Duty Cycle in order to provide the aerosol chamber with the most stable power possible within a pre-defined range that follows the studies and experimental results for most optimal safe aerosolisation.

## Battery monitoring

In order to provide an AC voltage of 15V and maintain a current inside the PZT around 2.5A, the current drawn from the battery reaches around 7 to 8 Amps, which in turn, creates a drop in the battery voltage. Any common LiPo battery would not sustain this demanding resource for the duration of an inhalation that can top 6s.

This is the reason why a custom LiPo battery is developed that can handle around 11 Amps, which is 50% more than the maximum allowed in the PZT at all time, while still being simple to use in compact and integrated portable device.

Because the battery voltage drops and varies a lot when activating the sonication section, the microcontroller constantly monitors the power used by the PZT inside the aerosol chamber to ensure a proper but also safe aerosolisation.

And because the key to aerosolisation is control, the device ensures first that the Control and Information section of the device always function and does not stop in the detriment of the sonication section.

This is why the adjustment method also takes into great account the real time battery level and, if need be, modifies the parameters like the Duty Cycle to maintain the battery at a safe level, and in the case of a low battery before starting the sonic engine, the Control and Information section will prevent the activation.

## Power control

As being said, the key to aerosolisation is control and the method used in the device is a real time multi-dimensional function that takes into account the profile of the PZT, the current inside the PZT and the battery level of the device at all time.

All this is only achievable thanks to the use of a microcontroller that can monitor and control every element of the device to produce an optimal inhalation.

## 1. Inhalation control

The device is a safe device and confirmed by BNS (Broughton Nicotine Services) report, but in order to guarantee the safety of misting and the integrity of both the aerosol chamber and the device, each inhalation has to be controlled.

## Inhalation duration

In order to reduce the exposure to carbonyls and other toxic components that might result from the heating of e-liquid, the maximum duration of an inhalation is set to 6 seconds which completely ensure that the exposure to these components is contained.

## Interval

Because the device relies on a piezoelectrical component, the device prevents the activation of the sonication section if an inhalation stops. The safety delay in between two inhalations is adaptive depending on the duration of the previous one. This allows the gauze to wick properly before the next activation.

With this functioning, the device can safely operate and the aerosolisation is rendered more optimal with no risk of breaking the PZT element nor exposing the user to toxic components.

## Connectivity (BLE)

The device Control and Information section is composed of a wireless communication system in the form of a Bluetooth Low Energy capable microcontroller. The wireless communication system is in communication with the processor of the device and is configured to transmit and receive data between the driver and a computing device, such as a smartphone.

The connectivity via Bluetooth Low Energy to a companion mobile application ensures that only small power for this communication is required thus allowing the device to remain functioning for a longer period of time if not used at all, compared to traditional wireless connectivity solutions like Wi-Fi, classic Bluetooth, GSM or even LTE-M and NB-IOT.

Most importantly, this connectivity is what enables the OTP as a feature and the complete control and safety of the inhalations. Every data from resonant frequency of an inhalation to the one used, or the negative pressure created by the user and the duration are stored and transferred over BLE for further analysis and improvements of the embedded software.

Moreover, all these information are crucial when the device is used in smoke cessation programs because it gives doctors and users all the information regarding the process of inhalation and the ability to track in real-time the prescriptions and the usage.

Finally, this connectivity enables the update of the embedded firmware inside the device and over the air (OTA), which guarantees that the latest versions can always be deployed rapidly. This gives great scalability to the device and insurance that the device is intended to be maintained.

## Data collection for clinical purposes

The device can collect user data such as number of puffs and puff duration in order to determine the total amount of therapeutic consumed by the user in a session.

This data can be interpreted by an algorithm that sets consumption limits per time period based on a physician's recommendations.

This will allow a controlled therapeutic dose of drug to be administered to the user that is controlled by a physician or pharmacist and cannot be abused by the end user.

The physician would be able to gradually lower dosages over time in a controlled method that is safe for the user.

## Puff Limitations

The process of ultrasonic cavitation has a significant impact on the nicotine concentration in the produced mist.

A device limitation of <7 second puff durations will limit the user to exposure of carbonyls commonly produced by electronic nicotine delivery systems.

Based on Broughton Nicotine Services' experimental results, after a user performs 10 consecutive puffs of <7 seconds, the total amount of carbonyls is <2.67$\mu$g/10 puffs (average: 1.43$\mu$g/10 puffs) for formaldehyde, <0.87$\mu$g/10 puffs (average: 0.50$\mu$g/10 puffs) for acetaldehyde, <0.40$\mu$g/10 puffs (average: 0.28$\mu$g/10 puffs) for propionaldehyde, <0.16$\mu$g/10 puffs (average: 0.16$\mu$g/10 puffs) for crotonaldehyde, <0.19$\mu$g/10 puffs (average: 0.17$\mu$g/10 puffs) for butyraldehyde, <0.42$\mu$g/10 puffs (average: 0.25$\mu$g/10 puffs) for diacetyl, and acetylpropionyl was not detected at all in the emissions after 10 consecutive <7 second puffs.

Because the aerosolisation of the e-liquid is achieved via the mechanical action of the piezoelectric disc and not due to the direct heating of the liquid, the individual components of the e-liquid (propylene glycol, vegetable glycerine, etc.) remain largely in-tact and are not broken into smaller, harmful components such as acrolein, acetaldehyde, formaldehyde, etc. at the high rate seen in traditional ENDS.

In order to limit the user's exposure to carbonyls while using the ultrasonic device, puff length is limited to 6 seconds maximum so that the above results would be the absolute worst-case scenario in terms of exposure.

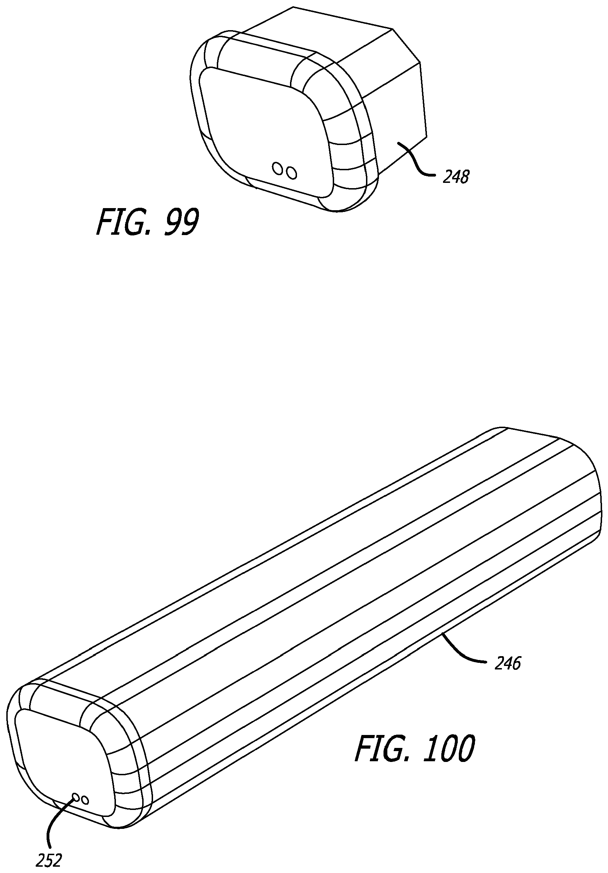

Referring now to Figures 99 and 100, when the end cap 248 is mounted to the driver housing 246, the driver housing 246, being aluminium, acts as a Faraday cage, preventing the device from emitting any electromagnetic waves. The device with the driver housing 246 has been tested for Electromagnetic Compatibility (EMC) and the tests reveal that the emissions are less than half the allowed limit for devices. The EMC test results are shown in the graph of Figure 76.

A mist delivery device of other examples of this disclosure comprises most of or preferably all of the elements of the mist delivery device described above, but with the memory of the driver 202 storing instructions which, when executed by the processor, provide additional functionality to the mist inhaler.

In one example, the mist delivery device comprises an active power monitor which incorporates a current sensor, such as the current sensor 335 described above, for sensing an rms drive current of the AC drive signal driving the ultrasonic transducer 215. The active power monitor provides a monitoring signal which is indicative of the sensed drive current, as described above.

The additional functionality of this example enables the mist inhaler 200 to monitor the operation of the ultrasonic transducer while the ultrasonic transducer is activated. The mist delivery device 200 calculates an effectiveness value or quality index which is indicative of how effective the ultrasonic transducer is operating to atomise a liquid within the device. The device uses the effectiveness value to calculate the actual amount of mist that was generated over the duration of activation of the ultrasonic transducer.

Once the actual amount of mist has been calculated, the device is configured to calculate the actual amount of a therapeutic which was present in the mist and hence the actual amount of a therapeutic which was inhaled by a user based on the concentration of the therapeutic in the liquid. Knowing the exact amount of a therapeutic which is delivered to a user is particularly important when the mist delivery device is being used as part of a therapeutic treatment program. Knowing the exact amount of a therapeutic which is delivered to a user during each inhalation or puff allows for the therapeutic treatment program to operate more accurately and effectively compared with using a conventional device which simply counts the number of inhalations or puffs, with each inhalation or puff assumed to deliver the same quantity of therapeutic to a user.

In practice, as described above, there are many different factors which affect the operation of an ultrasonic transducer and which have an impact on the amount of mist which is generated by the ultrasonic transducer and hence the actual amount of a therapeutic which is delivered to a user.

For instance, if an ultrasonic transducer within a mist inhaler is not operating in an optimal manner due to a low charge in the battery reducing the current flowing through the ultrasonic transducer, a lower amount of mist will be generated and a lower amount of a therapeutic will be delivered to a user compared with if the device was operating optimally. The device may thus allow a greater number of puffs for a user in order to deliver a set amount of therapeutic to the user over a period of time compared with the number of puffs that would be permitted if the ultrasonic transducer was operating optimally. This enables the therapeutic treatment program to operate more effectively and precisely compared with a conventional program which relies on using a device which simply counts and restricts the number of puffs taken by a user.

The configuration of the mist delivery device and a method of generating mist using the mist delivery device of some examples will now be described in detail below.

In this example, the mist delivery device incorporates the components of the mist delivery device 200 described above, but the memory of the driver 202 further stores instructions which, when executed by the processor, cause the processor to activate the mist generator 200 for a first predetermined length of time. As described above, the mist generator is activated by driving the ultrasonic transducer 215 in the mist generator 200 with the AC drive signal so that the ultrasonic transducer 215 atomises liquid carried by the capillary element 222.

The executed instructions cause the processor to sense, using a current sensor, periodically during the first predetermined length of time the current of the AC drive signal flowing through the ultrasonic transducer 215 and storing periodically measured current values in the memory.

The executed instructions cause the processor to calculate an effectiveness value using the current values stored in the memory. The effectiveness value is indicative of the effectiveness of the operation of the ultrasonic transducer at atomising the liquid.

In one example, the executed instructions cause the processor to calculate the effectiveness value using this equation:

$$Q_I = \frac{\sum_{t=0}^{t=D} \dfrac{\sqrt{Q_{A(t)}^2 + Q_{F(t)}^2}}{\sqrt{2}}}{N}$$

where:

$Q_I$ is the effectiveness value,
$Q_F$ is a frequency sub-effectiveness value which is based on the monitored frequency value (the frequency at which the ultrasonic transducer 215 is being driven),
$Q_A$ is an analogue to digital converter sub-effectiveness value which is based on the measured current value (the rms current flowing through the ultrasonic transducer 215),
t=0 is the start of the first predetermined length of time,

t=D is the end of the first predetermined length of time,

N is the number of periodic measurements (samples) during the first predetermined length of time, and

$\sqrt{2}$ is a normalization factor.

In one example, the memory stores instructions which, when executed by the processor, cause the processor to measure periodically during the first predetermined length of time the duty cycle of the AC drive signal driving the ultrasonic transducer and storing periodically measured duty cycle values in the memory. The mist delivery device then modifies the analogue to digital converter sub-effectiveness value $Q_A$ based on the current values stored in the memory. Consequently, the mist delivery device of this example takes into account variations in the duty cycle which may occur throughout the activation of the ultrasonic transducer 215 when the device calculates the effectiveness value. The mist delivery device can therefore calculate the actual amount of mist which is generated accurately by taking into account variations in the duty cycle of the AC drive signal which may occur while the ultrasonic transducer is activated.

In one example, the memory stores instructions which, when executed by the processor, cause the processor to measure periodically during the first predetermined length of time a voltage of a battery which is powering the mist generator and storing periodically measured battery voltage values in the memory. The mist delivery device then modifies the analogue to digital converter sub-effectiveness value $Q_A$ based on the battery voltage values stored in the memory. Consequently, the mist delivery device of this example takes into account variations in the battery voltage which may occur throughout the activation of the ultrasonic transducer 215 when the device calculates the effectiveness value. The mist delivery device can therefore calculate the actual amount of mist which is generated accurately by taking into account variations in the battery voltage which may occur while the ultrasonic transducer is activated.

The effectiveness value is used by the mist delivery device as a weighting to calculate the actual amount of mist generated by the mist delivery device by proportionally reducing a value of a maximum amount of mist that would be generated if the device was operating optimally.

In one example, the memory stores instructions which, when executed by the processor, cause the processor to measure periodically during the first predetermined length of time the frequency of the AC drive signal driving the ultrasonic transducer 215 and storing periodically measured frequency values in the memory. The device then calculates the effectiveness value using the using the frequency values stored in the memory, in addition to the current values as described above.

In one example, the memory stores instructions which, when executed by the processor, cause the processor to calculate a maximum mist amount value that would be generated if the ultrasonic transducer 215 was operating optimally over the duration of the first predetermined length of time. In one example, the maximum mist amount value is calculate based on modelling which determines the maximum amount of mist which would be generated when the ultrasonic transducer was operating optimally.

Once the maximum mist amount value has been calculated, the mist delivery device can calculate an actual mist amount value by reducing the maximum mist amount value proportionally based on the effectiveness value to determine the actual mist amount that was generated over the duration of the first predetermined length of time.

Once the actual mist amount has been calculated, the mist delivery device can calculate a therapeutic amount value which is indicative of the amount of therapeutic in the actual mist amount that was generated over the duration of the first predetermined length of time. The mist delivery device then stores a record of the therapeutic amount value in the memory. In this way, the mist delivery device can record accurately the actual amount of therapeutic which has been delivered to a user in each inhalation or puff.

In one example, the memory stores instructions which, when executed by the processor, cause the processor to selecting a second predetermined length of time in response to the effectiveness value. In this case, the second predetermined length of time is a length of time over which the ultrasonic transducer 215 is activated during a second inhalation or puff by a user. In one example, the second predetermined length of time is equal to the first predetermined length of time but with the time reduced or increased proportionally according to the effectiveness value. For instance, if the effectiveness value indicates that the ultrasonic transducer 215 is not operating effectively, the second predetermined length of time is made longer by the effectiveness value such that a desired amount of mist is generated during the second predetermined length of time.

When it comes to the next inhalation, the mist delivery device activates the mist generator for the second predetermined length of time so that the mist generator generates a predetermined amount of mist during the second predetermined length of time. The mist delivery device thus controls the amount of mist generated during the second predetermined length of time accurately, taking into account the various parameters which are reflected by the effectiveness value which affect the operation of the mist delivery device.

In one example, the memory stores instructions which, when executed by the processor, cause the processor to activate the mist generator for a plurality of predetermined lengths of time. For instance, the mist generator is activated during a plurality of successive inhalations or puffs by a user.

The mist delivery device stores a plurality of therapeutic amount values in the memory, each therapeutic amount value being indicative of the amount of therapeutic in the mist that was generated over the duration of a respective one of the predetermined lengths of time. In one example, the mist delivery device prevents further activation of the mist generator for a predetermined duration if the total amount of the therapeutic in the mist that was generated over the duration of the predetermined lengths of time is equal to or greater than a predetermined threshold. In one example, the predetermined duration is a duration in the range of 1 to 24 hours. In other examples, the predetermined duration is 24 hours or 12 hours.

The mist delivery device of some examples of this disclosure is configured to transmit data indicative of the therapeutic amount values from the mist generator to a computing device (e.g. via Bluetooth™ Low Energy communication) for storage in a memory of the computing device (e.g. a smartphone). An executable application running on the computing device can thus log the amount of therapeutic which has been delivered to a user. The executable application can also control the operation of the mist delivery device to limit the activation of the mist delivery device to restrict the amount of therapeutic being delivered to a user over a period of time.

The mist delivery device of some examples of this disclosure is therefore configured to prevent further activation once a user has consumed a set amount of therapeutic during a set timeframe, such as the amount of therapeutic consumed during a day.

All of the above applications involving ultrasonic technology can benefit from the optimisation achieved by the frequency controller which optimises the frequency of sonication for optimal performance.

It is to be appreciated that the disclosures herein are not limited to use for nicotine delivery. Indeed, in some examples, the mist inhaler device contains a liquid comprising a therapeutic which does not comprise nicotine. Some examples are configured for use for various medical purposes (e.g. the delivery of CBD for pain relief, supplements for performance enhancement, albuterol/salbutamol for asthma patients, etc.)

The devices disclosed herein are for use with any therapeutics, drugs or other compounds, with the drug or compound being provided in a liquid within the liquid chamber of the device for aerosolisation by the device. In some examples, the devices disclosed herein are for use with therapeutics, drugs and compounds including, but not limited to, the following:

*Respiratory*

*Brochodilators*

Olodaterol
Levalbuterol
Berodual (Ipratropium bromide / Fenoterol)
Combivent (Ipratropium bromide / Salbutamol)

*Anti-inflammatory*

Betamethasone
Dexamethasone
Methylprednisolone
Hydrocortisone
Mucolytics
N-Acetylcysteine

*Pulmonary Hypertension*

Sildenafil
Tadalafil
Epoprostenol
Treprostenil
Iloprost

*Infectious Disease*

*Antimicrobials*
Aminoglycosides (Gentamicin, Tobramycin, Amikacin, Colomycin, Neomycin, Liposomal Amikacin,)
Quinolones (Ciprofloxacin,Levofloxacin, Moxifloxacin Ofloxacin)
Macrolides (Azithromycin)
Minocycline
Betalactams (Piperacillin-Tazobactam, Ceftazidime Ticarcillin )
Cephalosporins (Cefotaxime, Cefepime, Ceftriaxone, Cefotaxime)
Glycopeptides (Vancomycin)
Meropenem
Polymixin ( Colistin, Polymixin B)

*Antifungals*

Amphotericin
Fluconazole
Caspofungen

*Antivirals*

Valganciclovir
Favipiravir
Remdisivir
Acyclovir
*Anti TB*
Isoniazid
Pyrazinamide
Rifampin
Ethambutol

*Oncology*

*Biologics*
Gilotrif
Afatinib
Caplacizumab
Dupilumab
Isarilumab
Alirucomab
Volasertib
Nintedanib
Imatinib
Sirolimus

*Chemotherapy*

Azacitidine

Decitabine
Docetaxel
Gemcitabine
Cisplatinum

*CNS & PSYCH*

Sodium valproate
Teriflunomide
Zomitriptan

*METABOLIC/HORMONAL*

Insulin
Estrogen

*IMMUNOLOGY*

Vaccine
Monoclonal Antibodies
Stem Cells

*Vitamins*

Zinc
Ascorbic Acid

*Miscellaneous*

Niclosamide
Hydroxychloroquine
Ivermectin

The mist generator of some examples is a more powerful version of current portable medical nebulizers.

Other examples of the ultrasonic mist inhaler devices are easily envisioned, including medicinal delivery devices which do not have the appearance of a cigarette.

The driver 202 is configured to drive the mist generator 500 of the examples described above which includes a plurality of ultrasonic transducers 505, 506, 578. In these examples, the driver 202 achieves the same technical benefits that are described above in relation to the example mist generator 400 that comprises a single ultrasonic transducer.

Aerosol tests confirm that a liquid particle size of less than $4\mu m$ (4 microns) or in the range of $0.03\mu m$ to $0.3\mu m$ or $1\mu m$ to $3\mu m$ is preferred for efficient delivery of the mist (aerosol) to the lower parts of the lungs (tracheobronchial and alveolar regions) for maximised absorption into the bloodstream. This particle size is preferred for delivering substances into the bloodstream through the alveoli and increases the efficacy of pharmaceutical drugs.

The only parts of the liquid that are required to be absorbed in the alveolar regions of the respiratory tract are the therapeutic-containing aerosol particles. The remainder of the components of the liquid are not necessarily required to be absorbed in the lower lungs.

In some examples, one of the components of the mist is a second therapeutic which is to be absorbed in upper respiratory tract (nasopharyngeal region) of a user. However, if the second therapeutic particles are small, then they will be carried to the lower lungs.

To enhance the therapeutic effect of the second therapeutic, the particle size of the second therapeutic needs to be increased to a range of 4μm to 10μm. Preferably, the particle size of a liquid containing at least one second therapeutic is 4μm or greater.

In this example, as described below, the driver 202 is configured to drive the plurality of ultrasonic transducers 505, 506, 578 to vibrate at different frequencies to generate a polydisperse mist including different liquid particle sizes. In some examples, one of the ultrasonic transducers 505, 506, 578 is driven at a frequency in the range of 2.9 MHz and 3.1 MHz to produce a mist of a liquid including therapeutic with a liquid particle size of less than 4μm or in the range 0.01μm to 0.3μm or 1μm to 3μm. The driver 202 preferably drives the other ultrasonic transducer(s) 505, 506 at a frequency in the range of 0.5 MHz & 1.5 MHz to produce a mist of another liquid, which may include a further therapeutic, with the mist having a liquid particle size of 4μm or greater for improved absorption in the nasopharyngeal region of the respiratory tract.

The configuration of a mist delivery device for generating a mist for inhalation by a user will now be described. In some examples, the driver 202 is configured for driving a mist generator 500 for generating a polydisperse mist including different liquid particle sizes.

In other examples, the driver 202 is configured for driving the mist generator 500 for generating two portions of a mist having the same or similar sized particles as one another, with the two portions of the mist being combined for inhalation by a user. In these examples, the first particle size is the same or is substantially the same as the second particle size.

The driver 202 is configured to drive a mist generator which may be the mist generator of examples described herein. In other examples, the driver 202 is configured to drive any mist generator having two or more ultrasonic transducers. In some examples, the driver 202 is configured to drive a mist generator which comprises either all or only some of the components of the mist generator 500 described above and the same reference numbers will be used in the description below for components that are equivalent to the components of the mist generator 500 described above. The driver 202 is, in some examples, configured to drive a mist generator 500 which comprises an air inlet port and a mouthpiece 401 including a mist outlet port 208. The mist generator 500 comprises an air flow path from the air inlet port to the mist outlet port 208. The mist generator 500 further comprises a first liquid chamber 546 containing a first liquid including a therapeutic and a second liquid chamber 547 containing a further therapeutic.

In some examples, the second liquid includes at least one further therapeutic.

In this example, the mist generator 500 comprises a first ultrasonic transducer 505 including a first atomisation surface and a first capillary 509 in contact with at least a portion of the first atomisation surface, the first capillary 509 being absorbent and extending from the first liquid chamber 546 to the first ultrasonic transducer 505. The mist generator 500 further comprises a second ultrasonic transducer 506 including a second atomisation surface and a second capillary 510 in contact with at least a portion of the second atomisation surface, the second capillary 510 being absorbent and extending from the second liquid chamber 547 to the second ultrasonic transducer 506.

In some examples, the second ultrasonic transducer 506 has the same configuration as the first ultrasonic transducer 505.

In this example, the driver 202 is configured to generate a first AC drive signal and a second AC drive signal concurrently. The driver 202 is coupled electrically to the first ultrasonic transducer 505 and the second ultrasonic transducer 506.

The first AC drive signal drives the first ultrasonic transducer 505 to generate and transmit ultrasonic waves. The driver 202 selects a frequency of the first AC drive signal in a first frequency range to generate a first portion of the mist from the first liquid at the interface between the first capillary 509 and the first ultrasonic transducer 505, the first portion of the mist having a first liquid particle size.

The second AC drive signal drives the second ultrasonic transducer 506 to generate and transmit ultrasonic waves. The driver 202 selects a frequency of the second AC drive signal in a second frequency range to generate a second portion of the mist from the second liquid at the interface between the second capillary 510 and the second ultrasonic transducer 506, the second portion of the mist having a second liquid particle size.

In some examples, the first frequency range and the second frequency range are different. In some examples, the first particle size and the second particle size are different.

In some examples, the first frequency range is 2.9 MHz to 3.1 MHz. In some examples, the second frequency range is 0.5 MHz to 1.5 MHz. In other examples, the first and second frequency ranges are 2.9 MHz to 3.1 MHz. In other examples, the first and second frequency ranges are 0.5 MHz to 1.5 MHz.

In some examples, the first liquid particle size is less than 4 microns. In some examples, the second liquid particle size is greater than 4 microns. In other examples, the first liquid particle size is the same or substantially the same as the second liquid particle size.

The first portion of the mist and the second portion of the mist combine to form a mist or a polydisperse mist including different liquid particle sizes. The mist flows through the mist outlet port to the user when the user draws on the mouthpiece 401.

In some examples, the mist generator 500 includes at least one further liquid chamber each containing a respective further liquid. The mist generator includes at least one further ultrasonic transducer 578 each including a respective further atomisation surface. The mist generator 500 also includes at least one further capillary 510' each in contact with a portion of a respective further atomisation surface, each further capillary 510' being absorbent and each further capillary 510' extending from a respective further liquid chamber to a respective further ultrasonic transducer 578.

In some examples, the driver 202 is configured to generate at least one further AC drive signal concurrently with the first AC drive signal and the second AC drive signal, the driver 202 being coupled electrically to each further ultrasonic transducer 578.

Each further AC drive signal drives the respective further ultrasonic transducer 578 to generate and transmit ultrasonic waves. The driver 202 selects a frequency of each further AC drive signal in at least one further frequency range to generate at least one further portion of the mist from each further liquid at the interface between each further capillary and a respective further ultrasonic transducer 578. In some examples, each further portion of the mist has a respective further liquid particle size. Each further portion of the mist combines with the first portion of the mist and the second portion of the mist to form the polydisperse mist.

In some examples, each further liquid includes at least one further therapeutic.

In some examples, the driver 202 comprises a plurality of H-bridge circuits which are each connected to a respective one of the ultrasonic transducers. Each H-bridge circuit may be an H-bridge circuit of the same configuration as the H-bridge circuit 334 described herein. Each H-bridge circuit is configured to generate the respective AC drive signal to drive the respective ultrasonic transducer. In some examples, the plurality of H-bridge circuits are each subsystems of a bridge integrated circuit (bridge IC), such as the bridge IC 301 described herein. The bridge integrated circuit (bridge IC) may be a multi-channel output integrated circuit having a plurality of outputs for driving a plurality of ultrasonic transducers 505, 506. In other examples, each H-bridge circuit is implemented in a respective discrete integrated circuit, such as the bridge IC 301 described herein.

In some examples, the driver 202 comprises a plurality of power management subsystems each configured to output a first phase output signal and a second phase output signal to a respective one of the H-bridge circuits. In some examples, the plurality of power management subsystems are each subsystems of a power management integrated circuit (PMIC), such as the PMIC 300 described herein. The power management integrated circuit (PMIC) may be a multi-channel output integrated circuit having a plurality of outputs for driving a plurality of H-bridge circuits. In other examples, each power management subsystem is implemented in a separate integrated circuit, such as the PMIC 300 described herein. In further examples, the PMIC 300 is modified to provide a plurality of outputs for driving a plurality of H-bridge circuits.

Each power management subsystem includes a first feedback input terminal which is configured to receive a feedback signal from the respective H-bridge circuit. The feedback input terminal is preferably one or more of the analogue inputs 319 of the ADC 318 of the PMIC 300 described herein. The feedback signal is indicative of a parameter of the operation of the respective H-bridge circuit when the respective H-bridge circuit is driving the respective ultrasonic transducer 505, 506 with the respective AC drive signal. The power management subsystem is configured to generate a respective voltage for modulation by the respective H-bridge circuit to drive the respective ultrasonic transducer 505, 506 in response to the feedback signal which is indicative of the operation of the respective ultrasonic transducer 505, 506 to optimise the generation of the respective portion of the mist.

In some examples, each power management subsystem includes an oscillator 315 which is configured to generate a main clock signal, a first phase clock signal which is high for a first time during the positive half-period of the main clock signal and low during the negative half-period of the main clock signal. The oscillator 315 is configured to generate a second phase clock signal which is high for a second time during the negative half-period of the main clock signal and low during the positive half-period of the main clock signal. The phases of the first phase clock signal and the second phase clock signal are preferably centre aligned.

In some examples, each power management subsystem includes a pulse width modulation (PWM) signal generator subsystem 329 comprising a delay locked loop 332 which is configured to generate a double frequency clock signal using the first phase clock signal and the second phase clock signal. The double frequency clock signal is double the frequency of the main clock signal. The delay locked loop 332 is configured to control the rising edge of the first phase clock signal and the second phase clock signal to be synchronous with the rising edge of the double frequency clock signal.

The delay locked loop 332 is configured to adjust the frequency and the duty cycle of the first phase clock signal and the second phase clock signal in response to a driver control signal to produce the first phase output signal and the second phase output signal. The first phase output signal and the second phase output signal are configured to drive the H-bridge circuit to generate an AC drive signal.

In some examples, the driver 202 comprises a first mist density sensor which is configured to sense a density of the first portion of the mist. In some examples, the first mist density sensor is an optical sensor that is placed along a portion of the gas flow path carrying the first portion of the mist. The first mist density sensor preferably measures the optical attenuation of a light source due to the first portion of the mist, wherein the optical attenuation corresponds with the density of the first portion of the mist. The density of the mist correlates with concentration of liquid and/or compounds in the mist. The density of the first portion of the mist is therefore indicative of a dosage of therapeutic that is being delivered to a user.

The first mist density sensor generates a first mist density signal which is indicative of the density of the first portion of the mist. The first mist density sensor provides the first mist density signal to a second feedback input terminal 319 of a respective power management subsystem of the plurality of power management subsystems. The respective power management subsystem generates the respective voltage for modulation by the respective H-bridge circuit in response to the first mist density signal to optimise the generation of the first portion of the mist. For example, if the mist density signal indicates that the density of the first portion of the mist is lower than expected (lower than a minimum threshold) then the power management subsystem reacts in response to the mist density signal to modify the frequency and/or intensity of the AC drive signal being output to the ultrasonic transducer 505, 506 to control the ultrasonic transducer 505, 506 to optimise the generation of the first portion of the mist in a feedback loop.

In some examples, the driver 202 comprises at least one further mist density sensor which is configured to sense a density of at least one further portion of the mist. The at least one further mist density sensor is preferably of a similar configuration to the first mist density sensor described above. The density of the mist correlates with concentration of liquid and/or compounds in the mist.

The at least one further mist density sensor generates at least one further mist density signal which is indicative of the density of the at least one further portion of the mist. The at least one further mist density sensor provides the at least one further mist density signal to a further feedback input terminal 319 of a respective power management subsystem of the plurality of power management subsystems. The respective power management subsystem generates the respective voltage for modulation by the respective H-bridge circuit in response to the at least one further mist density signal to optimise the generation of the at least one further portion of the mist. For example, if the at least one further mist density signal indicates that the density of the at least one further portion of the mist is lower than expected (lower than a minimum threshold) then the power management subsystem reacts in response to the at least one further mist density signal to modify the frequency and/or intensity of the AC drive signal being output to the ultrasonic transducer to control the ultrasonic transducer to optimise the generation of the at least one further portion of the mist in a feedback loop.

In some examples, the driver 202 comprises a main controller coupled electrically to each power management subsystem. The main controller is preferably the microcontroller 303 as described herein which is electrically coupled to each power management subsystem via the communication bus 302. The main controller is configured to coordinate the operation of the power management subsystems in response to a configuration signal to control the generation of mist.

In some examples, the driver 202 comprises an input device for receiving an input from a user. In these examples, the driver 202 is configured to generate the configuration signal in response to the input from the user.

In some examples, the input device includes a wireless communication device which is configured to communicate wirelessly with a computing device to enable a user to provide the input using the computing device. For example, the input device is configured to transmit and receive data between the driver device and a computing device, such as a smartphone, to enable a user to configure the device to generate mist as the user wishes and/or in line with a prescription.

The foregoing outlines features of several examples or embodiments so that those of ordinary skill in the art may better understand various aspects of the present disclosure. Those of ordinary skill in the art should appreciate that they may readily use the present disclosure as a basis for designing or modifying other processes and structures for carrying out the same purposes and/or achieving the same advantages of various examples or embodiments introduced herein. Those of ordinary skill in the art should also realise that such equivalent constructions do not depart from the scope of the present disclosure, and that they may make various changes, substitutions, and alterations herein without departing from the scope of the present disclosure.

Although the subject matter has been described in language specific to structural features or methodological acts, it is to be understood that the subject matter of the appended claims is not necessarily limited to the specific features or acts described above. Rather, the specific features and acts described above are disclosed as example forms of implementing at least some of the claims.

Various operations of examples or embodiments are provided herein. The order in which some or all of the operations are described should not be construed to imply that these operations are necessarily order dependent. Alternative ordering will be appreciated having the benefit of this description. Further, it will be understood that not all operations are necessarily present in each embodiment provided herein. Also, it will be understood that not all operations are necessary in some examples or embodiments.

Moreover, "exemplary" is used herein to mean serving as an example, instance, illustration, etc., and not necessarily as advantageous. As used in this application, "or" is intended to mean an inclusive "or" rather than an exclusive "or". In addition, "a" and "an" as used in this application and the appended claims are generally be construed to mean "one or more" unless specified otherwise or clear from context to be directed to a singular form. Also, at least one of A and B and/or the like generally means A or B or both A and B. Furthermore, to the extent that "includes", "having", "has", "with", or variants thereof are used, such terms are intended to be inclusive in a manner similar to the term "comprising". Also, unless specified otherwise, "first," "second," or the like are not intended to imply a temporal aspect, a spatial aspect, an ordering, etc. Rather, such terms are merely used as identifiers, names, etc. for features, elements, items, etc. For example, a first element and a second element generally correspond to element A and element B or two different or two identical elements or the same element.

Also, although the disclosure has been shown and described with respect to one or more implementations, equivalent alterations and modifications will occur to others of ordinary skill in the art based upon a reading and understanding of this specification and the annexed drawings. The disclosure comprises all such modifications and alterations and is limited only by the scope of the following claims. In particular regard to the various functions performed by the above described features (e.g., elements, resources, etc.), the terms used to describe such features are intended to correspond, unless otherwise indicated, to any features which performs the specified function of the described features (e.g., that is functionally equivalent), even though not structurally equivalent to the disclosed structure. In addition, while a particular feature of the disclosure may have been disclosed with respect to only one of several implementations, such feature may be combined with one or more other features of the other implementations as may be desired and advantageous for any given or particular application.

Examples or embodiments of the subject matter and the functional operations described herein can be implemented in digital electronic circuitry, or in computer software, firmware, or hardware, including the structures disclosed in this specification and their structural equivalents, or in combinations of one or more of them.

Some examples or embodiments are implemented using one or more modules of computer program instructions encoded on a computer-readable medium for execution by, or to control the operation of, a data processing apparatus. The computer-readable medium can be a manufactured product, such as hard drive in a computer system or an embedded system. The computer-readable medium can be acquired separately and later encoded with the one or more modules of computer program instructions, such as by delivery of the one or more modules of computer program instructions over a wired or wireless network. The computer-readable medium can be a machine-readable storage device, a machine-readable storage substrate, a memory device, or a combination of one or more of them.

The terms "computing device" and "data processing apparatus" encompass all apparatus, devices, and machines for processing data, including by way of example a programmable processor, a computer, or multiple processors or computers. The apparatus can include, in addition to hardware, code that creates an execution environment for the computer program in question, e.g., code that constitutes processor firmware, a protocol stack, a database management system, an operating system, a runtime environment, or a combination of one or more of them. In addition, the apparatus can employ various different computing model infrastructures, such as web services, distributed computing and grid computing infrastructures.

The processes and logic flows described in this specification can be performed by one or more programmable processors executing one or more computer programs to perform functions by operating on input data and generating output.

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Devices suitable for storing computer program instructions and data include all forms of non-volatile memory, media and memory devices.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

It is appreciated that it is within the scope of the present invention that the various embodiments and features described and shown herein can be combined in whole or in part with one another.

The detailed description set forth below is intended as a description of various configurations of the present invention and is not intended to represent the only configurations in which the present invention may be practiced. It will be apparent, however, to those of ordinary skill in the art that the present invention is not limited to the specific details set forth herein and may be practiced without these specific details. When used in this specification and claims, the terms "comprises" and "comprising" and variations thereof mean that the specified features, steps or integers are included. The terms are not to be interpreted to exclude the presence of other features, steps or components.

One or more features in any of the embodiments described herein may be combined with one or more features from any other embodiment(s) described herein.

Although certain example embodiments of the invention have been described, the scope of the appended claims is not intended to be limited solely to these embodiments. The claims are to be construed literally and purposively.

**Claims**

1. A mist generator for generating a mist for inhalation by a user, the mist generator comprising:

an air inlet port (569);
a mouthpiece (401) including a mist outlet port (208);
an air flow path from the air inlet port (569) to the mist outlet port (208);
a first liquid chamber (546) containing a first liquid including a therapeutic;
a second liquid chamber (547) containing a second liquid;
a first ultrasonic transducer (505) including a first atomisation surface;
a second ultrasonic transducer (506) including a second atomisation surface;
a transducer holder (514) including a holder surface that is provided with a first recess (520) and a second recess (521), wherein the first recess (520) retains the first ultrasonic transducer (505) with the first atomisation surface generally in a plane with the holder surface and the second recess (521) retains the second ultrasonic transducer (506) with the second atomisation surface generally in the plane with the holder surface;
a first capillary (509) which is absorbent, at least part of the first capillary (509) being parallel with the plane and extending along the holder surface from the first liquid chamber (546) to the first ultrasonic transducer (505), one part of the first capillary (509) being superimposed on the holder surface and another part of the first capillary (509) being in contact with at least a portion of the first atomisation surface, wherein the first capillary (509) transports the first liquid by capillary action from the first liquid chamber (546) to the first atomisation surface; and
a second capillary (510) which is absorbent, at least part of the second capillary (510) being parallel with the plane and extending along the holder surface from the second liquid chamber to the second ultrasonic transducer, one part of the second capillary (510) being superimposed on the holder surface and another part of the second capillary (510) being in contact with at least a portion of the second atomisation surface, wherein the second capillary (510) transports the second liquid by capillary action from the second liquid chamber (547) to the second atomisation surface,
the first ultrasonic transducer (505) being configured to generate and transmit ultrasonic waves to generate a first portion of the mist from the first liquid at the interface between the first capillary (509) and the first ultrasonic transducer (505), and
the second ultrasonic transducer (510) being configured to generate and transmit ultrasonic waves to generate a second portion of the mist from the second liquid at the interface between the second capillary (510) and the second ultrasonic transducer (506), wherein the first portion of the mist and the second portion of the mist combine and flow out through the mist outlet port (208) to the user when the user draws on the mouthpiece (401).

2. The mist generator of claim 1, wherein the transducer holder (514) comprises:

a first electrical contact channel (535) which receives at least part of a first electrical contact (518), the first electrical contact (518) extending from a first end of the transducer holder (514) to the first transducer (505); and
a second electrical contact channel (536) which receives at least part of a second electrical contact (519), the second electrical contact (519) extending from the first end of the transducer holder (514) to the second transducer (506).

3. The mist generator of claim 1 or claim 2, wherein the first recess (520) of the transducer holder (514) is provided with a first through hole (522) and the second recess (521) of the transducer holder (514) is provided with a second through hole (523), where a side of the first ultrasonic transducer (505) is contactable through the first through hole (522) and a side of the second ultrasonic transducer (506) is contactable through the second through hole (523).

4. The mist generator of claim 3, wherein the mist generator comprises:

a third electrical contact (515), part of the third electrical contact (515) extending through the first through hole (522) to contact the side of the first ultrasonic transducer (505); and
a fourth electrical contact (516), part of the fourth electrical contact (516) extending through the second through hole (523) to contact the side of the second ultrasonic transducer (506).

5. The mist generator of any one of the preceding claims, wherein the mist generator comprises:
a plurality of assembly portions which are coupled to one another to form an air inlet port (569) and an air flow path from the air inlet port (569) to the mist outlet port (208), one assembly portion of the plurality of assembly portions comprising a mist outlet channel (560) and at least a part of a mist outlet conduit.

6. The mist generator of claim 5, wherein the plurality of assembly portions include a first assembly portion (511), the first assembly portion (511) including a first surface (563), a second surface (564), and two air inlet channels (557, 558) which extend along the first surface (563) and to the second surface (564).

7. The mist generator of claim 6, wherein the first assembly portion (511) includes the mist outlet channel (560) and the at least a part of the mist outlet conduit.

8. The mist generator of claim 6 or claim 7, wherein the plurality of assembly portions include a second assembly portion (512), the second assembly portion (512) including:

an upper section (566);
a lower section (565) spaced from the upper section (566); and
a middle section (567) extending from the upper section (566) to the lower section (565),
the upper section (566) including two sonication chamber recesses (541, 542), two sonication chamber air inlets (552, 553), two sonication chamber mist outlets (554, 555); and
the lower section (565) including two channels (550, 551),
such that then the first assembly portion (511) is coupled to the second assembly portion (512):
the two air inlet channels (557, 558) of the first assembly portion (511) and the two channels (550, 551) of the second assembly portion (512) form an air flow path from the inlet port (569) to the sonication chamber air inlets (552, 553); and
a mist flow path from the sonication chamber mist outlets (554, 555) through the mist outlet conduit.

9. The mist generator of claim 6 or claim 7, wherein the plurality of assembly portions includes a second assembly portion (512), the second assembly portion including:

an upper section (566);
a lower section (565) spaced from the upper section (566); and
a middle section (567) extending from the upper section (566) to the lower section (565),
the upper section (566) including two sonication chamber recesses (541, 542), two sonication chamber air inlets (552, 553), two sonication chamber mist outlets (554, 555), and a groove (556); and
the lower section (565) including two channels (550, 551),
wherein the two air inlet channels (557, 558) of the first assembly portion (511) and the two channels (550, 551) of the second assembly portion (512) form an air flow path from the inlet port (569) to the sonication chamber air inlets (552, 553); and
the at least a part of the mist outlet conduit of the first assembly portion (511) and the groove (556) of the upper section (566) of the second assembly portion (512) form a mist flow path from the sonication chamber mist outlets (554, 555) through the mist outlet conduit.

10. The mist generator of claim 8 or claim 9, wherein the upper section (566) includes at least one biasing member (466) within each recess (541, 542) for biasing the respective capillary (509, 510) against the portion of the respective atomisation surface.

11. The mist generator of any one of claims 8 to 10, wherein the middle section (567) of the second assembly portion (512) includes two extrusions (582, 583).

12. The mist generator of any one of claims 8 to 11, wherein the plurality of assembly portions includes a third assembly portion (513), the third assembly portion (513) including two through holes (539, 540), the first liquid chamber (546), and second liquid chamber (547).

13. The mist generator of any one of the preceding claims, further including two generally annular electrical connections (507, 508), each of the two generally annular electrical connections (507, 508) coupled to a respective one of the first ultrasonic transducer (505) and the second ultrasonic transducer (506).

14. The mist generator of any one of claims 8 to 12, further including a fourth assembly portion (517) which is at least partly superimposed on a portion of the transducer holder (514).

15. The mist generator of claim 12, wherein the third assembly portion (513) includes two depressions (537, 538), each depression (537, 538) overlapping a respective one of the two through holes (539, 540) in the third assembly portion (513), and optionally wherein the second liquid includes at least one further therapeutic, and optionally wherein the mist generator comprises:

at least one further liquid chamber each containing a respective further liquid;
at least one further ultrasonic transducer (578) each including a respective further atomisation surface;
at least one further capillary (510'), at least part of the further capillary (510') being generally parallel with the plane and extending along the holder surface from the respective further liquid chamber to the respective further ultrasonic transducer (578), one part of the respective further capillary (510') being superimposed on the holder surface and another part of the respective further capillary (510') being in contact with at least a portion of the respective further atomisation surface, wherein the respective further capillary (510') transports the respective further liquid by capillary action from the respective further liquid chamber to the respective further atomisation surface,
each further ultrasonic transducer (578) being configured to generate and transmit ultrasonic waves to generate a respective further portion of the mist from the respective further liquid at the interface between the respective further capillary (510') and the respective further ultrasonic transducer (578), wherein the respective further portion of the mist combines with the first portion of the mist and the second portion to form the mist which flows out through the mist outlet port (208) to the user when the user draws on the mouthpiece (401), and optionally wherein each further liquid includes at least one further therapeutic.

**Patentansprüche**

1.  Nebelgenerator zum Generieren eines Nebels für eine Inhalation durch einen Benutzer, der Nebelgenerator umfassend:

einen Lufteinlassanschluss (569);
ein Mundstück (401), das einen Nebelauslassanschluss (208) einschließt;
einen Luftstromweg von dem Lufteinlassanschluss (569) zu dem Nebelauslassanschluss (208);
eine erste Flüssigkeitskammer (546), die eine erste Flüssigkeit enthält, die ein Therapeutikum einschließt;
eine zweite Flüssigkeitskammer (547), die eine zweite Flüssigkeit enthält;
einen ersten Ultraschallwandler (505), der eine erste Zerstäubungsoberfläche einschließt;
einen zweiten Ultraschallwandler (506), der eine zweite Zerstäubungsoberfläche einschließt;
einen Wandlerhalter (514), der eine Halteroberfläche einschließt, die mit einer ersten Aussparung (520) und einer zweiten Aussparung (521) bereitgestellt wird, wobei die erste Aussparung (520) den ersten Ultraschallwandler (505) mit der ersten Zerstäubungsoberfläche im Allgemeinen in einer Ebene mit der Halteroberfläche hält und die zweite Aussparung (521) den zweiten Ultraschallwandler (506) mit der zweiten Zerstäubungsoberfläche im Allgemeinen in der Ebene mit der Halteroberfläche hält;
eine erste Kapillare (509), die absorbierend ist, wobei mindestens ein Teil der ersten Kapillare (509) parallel zu der Ebene verläuft und sich entlang der Halteroberfläche von der ersten Flüssigkeitskammer (546) zu dem ersten Ultraschallwandler (505) erstreckt, wobei ein Teil der ersten Kapillare (509) der Halteroberfläche überlagert ist und ein anderer Teil der ersten Kapillare (509) in Kontakt mit mindestens einem Abschnitt der ersten Zerstäubungsoberfläche steht, wobei die erste Kapillare (509) die erste Flüssigkeit durch Kapillarwirkung von der ersten Flüssigkeitskammer (546) zu der ersten Zerstäubungsoberfläche transportiert; und
eine zweite Kapillare (510), die absorbierend ist, wobei mindestens ein Teil der zweiten Kapillare (510) parallel zu der Ebene verläuft und sich entlang der Halteroberfläche von der zweiten Flüssigkeitskammer zu dem zweiten Ultraschallwandler erstreckt, wobei ein Teil der zweiten Kapillare (510) der Halteroberfläche überlagert ist und ein anderer Teil der zweiten Kapillare (510) in Kontakt mit mindestens einem Abschnitt der zweiten Zerstäubungsoberfläche steht, wobei die zweite Kapillare (510) die zweite Flüssigkeit durch Kapillarwirkung von der zweiten Flüssigkeitskammer (547) zu der zweiten Zerstäubungsoberfläche transportiert,
der erste Ultraschallwandler (505) konfiguriert ist, um Ultraschallwellen zu generieren und zu übertragen, um einen ersten Abschnitt des Nebels aus der ersten Flüssigkeit an der Grenzfläche zwischen der ersten Kapillare (509) und dem ersten Ultraschallwandler (505) zu generieren, und
wobei der zweite Ultraschallwandler (510) konfiguriert ist, um Ultraschallwellen zu generieren und zu übertragen, um einen zweiten Abschnitt des Nebels aus der zweiten Flüssigkeit an der Grenzfläche zwischen der zweiten Kapillare (510) und dem zweiten Ultraschallwandler (506) zu generieren, wobei der erste Abschnitt des Nebels und der zweite Abschnitt des Nebels sich bündeln und durch den Nebelauslassanschluss (208) hindurch zu dem Benutzer herausströmen, wenn der Benutzer an dem Mundstück (401) zieht.

2. Nebelgenerator nach Anspruch 1, wobei der Wandlerhalter (514) umfasst:

   einen Kanal (535) eines ersten elektrischen Kontakts, der mindestens einen Teil eines ersten elektrischen Kontakts (518) aufnimmt, wobei sich der erste elektrische Kontakt (518) von einem ersten Ende des Wandlerhalters (514) zu dem ersten Wandler (505) erstreckt; und
   einen Kanal (536) eines zweiten elektrischen Kontakts, der mindestens einen Teil eines zweiten elektrischen Kontakts (519) aufnimmt, wobei sich der zweite elektrische Kontakt (519) von dem ersten Ende des Wandlerhalters (514) zu dem zweiten Wandler (506) erstreckt.

3. Nebelgenerator nach Anspruch 1 oder 2, wobei die erste Aussparung (520) des Wandlerhalters (514) mit einem ersten Durchgangsloch (522) bereitgestellt wird und die zweite Aussparung (521) des Wandlerhalters (514) mit einem zweiten Durchgangsloch (523) bereitgestellt wird, wobei eine Seite des ersten Ultraschallwandlers (505) durch das erste Durchgangsloch (522) hindurch kontaktierbar ist und eine Seite des zweiten Ultraschallwandlers (506) durch das zweite Durchgangsloch (523) hindurch kontaktierbar ist.

4. Nebelgenerator nach Anspruch 3, wobei der Nebelgenerator umfasst:

   einen dritten elektrischen Kontakt (515), wobei sich ein Teil des dritten elektrischen Kontakts (515) durch das erste Durchgangsloch (522) hindurch erstreckt, um die Seite des ersten Ultraschallwandlers (505) zu kontaktieren; und
   einen vierten elektrischen Kontakt (516), wobei sich ein Teil des vierten elektrischen Kontakts (516) durch das zweite Durchgangsloch (523) hindurch erstreckt, um die Seite des zweiten Ultraschallwandlers (506) zu kontaktieren.

5. Nebelgenerator nach einem der vorstehenden Ansprüche, wobei der Nebelgenerator ferner umfasst:
   eine Vielzahl von Baugruppenabschnitten, die miteinander gekoppelt sind, um einen Lufteinlassanschluss (569) und einen Luftstromweg von dem Lufteinlassanschluss (569) zu dem Nebelauslassanschluss (208) auszubilden, ein Baugruppenabschnitt der Vielzahl von Baugruppenabschnitten umfassend einen Nebelauslasskanal (560) und mindestens einen Teil einer Nebelauslassleitung.

6. Nebelgenerator nach Anspruch 5, wobei die Vielzahl von Baugruppenabschnitten einen ersten Baugruppenabschnitt (511) einschließt, wobei der erste Baugruppenabschnitt (511) eine erste Oberfläche (563), eine zweite Oberfläche (564) und zwei Lufteinlasskanäle (557, 558) einschließt, die sich entlang der ersten Oberfläche (563) und zu der zweiten Oberfläche (564) erstrecken.

7. Nebelgenerator nach Anspruch 6, wobei der erste Baugruppenabschnitt (511) den Nebelauslasskanal (560) und den mindestens einen Teil der Nebelauslassleitung einschließt.

8. Nebelgenerator nach Anspruch 6 oder 7, wobei die Vielzahl von Baugruppenabschnitten einen zweiten Baugruppenabschnitt (512) einschließt, wobei der zweite Baugruppenabschnitt (512) einschließt:

   einen oberen Bereich (566);
   einen unteren Bereich (565), der von dem oberen Bereich (566) beabstandet ist; und
   einen mittleren Bereich (567), der sich von dem oberen Bereich (566) zu dem unteren Bereich (565) erstreckt, der obere Bereich (566) zwei Beschallungskammeraussparungen (541, 542), zwei Beschallungskammerlufteinlässe (552, 553), zwei Beschallungskammernebelauslässe (554, 555) einschließt; und
   der untere Bereich (565), der zwei Kanäle (550, 551) derart einschließt, dass dann der erste Baugruppenabschnitt (511) mit dem zweiten Baugruppenabschnitt (512) gekoppelt ist:

      die zwei Lufteinlasskanäle (557, 558) des ersten Baugruppenabschnitts (511) und die zwei Kanäle (550, 551) des zweiten Baugruppenabschnitts (512) einen Luftstromweg von dem Einlassanschluss (569) zu den Beschallungskammerlufteinlässen (552, 553) ausbilden; und
      einen Nebelstromweg von den Beschallungskammernebelauslässen (554, 555) durch die Nebelauslassleitung hindurch.

9. Nebelgenerator nach Anspruch 6 oder 7, wobei die Vielzahl von Baugruppenabschnitten einen zweiten Baugruppenabschnitt (512) einschließt, wobei der zweite Baugruppenabschnitt einschließt:

einen oberen Bereich (566);

einen unteren Bereich (565), der von dem oberen Bereich (566) beabstandet ist; und

einen mittleren Bereich (567), der sich von dem oberen Bereich (566) zu dem unteren Bereich (565) erstreckt, der obere Bereich (566), der zwei Beschallungskammeraussparungen (541, 542), zwei Beschallungskammerlufteinlässe (552, 553), zwei Beschallungskammernebelauslässe (554, 555) und eine Nut (556) einschließt; und

der untere Bereich (565), der zwei Kanäle (550, 551) einschließt,

wobei die zwei Lufteinlasskanäle (557, 558) des ersten Baugruppenabschnitts (511) und die zwei Kanäle (550, 551) des zweiten Baugruppenabschnitts (512) einen Luftstromweg von dem Einlassanschluss (569) zu den Beschallungskammerlufteinlässen (552, 553) ausbilden; und

der mindestens eine Teil der Nebelauslassleitung des ersten Baugruppenabschnitts (511) und die Nut (556) des oberen Bereichs (566) des zweiten Baugruppenabschnitts (512) einen Nebelstromweg von den Beschallungskammernebelauslässen (554, 555) durch die Nebelauslassleitung hindurch ausbilden.

10. Nebelgenerator nach Anspruch 8 oder 9, wobei der obere Bereich (566) mindestens ein Vorspannelement (466) innerhalb jeder Aussparung (541, 542) zum Vorspannen der jeweiligen Kapillare (509, 510) gegen den Abschnitt der jeweiligen Zerstäubungsoberfläche einschließt.

11. Nebelgenerator nach einem der Ansprüche 8 bis 10, wobei der mittlere Bereich (567) des zweiten Baugruppenabschnitts (512) zwei Extrusionen (582, 583) einschließt.

12. Nebelgenerator nach einem der Ansprüche 8 bis 11, wobei die Vielzahl von Baugruppenabschnitten einen dritten Baugruppenabschnitt (513) einschließt, wobei der dritte Baugruppenabschnitt (513) zwei Durchgangslöcher (539, 540), die erste Flüssigkeitskammer (546) und die zweite Flüssigkeitskammer (547) einschließt.

13. Nebelgenerator nach einem der vorstehenden Ansprüche, der ferner zwei im Allgemeinen ringförmige elektrische Verbindungen (507, 508) einschließt, wobei jede der zwei im Allgemeinen ringförmigen elektrischen Verbindungen (507, 508) mit einem jeweiligen des ersten Ultraschallwandlers (505) und des zweiten Ultraschallwandlers (506) gekoppelt ist.

14. Nebelgenerator nach einem der Ansprüche 8 bis 12, der ferner einen vierten Baugruppenabschnitt (517) einschließt, der einem Abschnitt des Wandlerhalters (514) mindestens teilweise überlagert ist.

15. Nebelgenerator nach Anspruch 12, wobei der dritte Baugruppenabschnitt (513) zwei Vertiefungen (537, 538) einschließt, wobei jede Vertiefung (537, 538) ein jeweiliges der zwei Durchgangslöcher (539, 540) in dem dritten Baugruppenabschnitt (513) überlappt, und optional

wobei die zweite Flüssigkeit mindestens ein weiteres Therapeutikum einschließt, und optional wobei der Nebelgenerator umfasst:

mindestens eine weitere Flüssigkeitskammer, die jeweils eine jeweilige weitere Flüssigkeit enthält;

mindestens einen weiteren Ultraschallwandler (578), der jeweils eine entsprechende weitere Zerstäubungsoberfläche einschließt;

mindestens eine weitere Kapillare (510'), wobei mindestens ein Teil der weiteren Kapillare (510') im Allgemeinen parallel zu der Ebene verläuft und sich entlang der Halteroberfläche von der jeweiligen weiteren Flüssigkeitskammer zu dem jeweiligen weiteren Ultraschallwandler (578) erstreckt, wobei ein Teil der jeweiligen weiteren Kapillare (510') der Halteroberfläche überlagert ist und ein anderer Teil der jeweiligen weiteren Kapillare (510') in Kontakt mit mindestens einem Abschnitt der jeweiligen weiteren Zerstäubungsoberfläche steht, wobei die jeweilige weitere Kapillare (510') die jeweilige weitere Flüssigkeit durch Kapillarwirkung von der jeweiligen weiteren Flüssigkeitskammer zu der jeweiligen weiteren Zerstäubungsoberfläche transportiert,

wobei jeder weitere Ultraschallwandler (578) konfiguriert ist, um Ultraschallwellen zu generieren und zu übertragen, um einen jeweiligen weiteren Abschnitt des Nebels aus der jeweiligen weiteren Flüssigkeit an der Grenzfläche zwischen der jeweiligen weiteren Kapillare (510') und dem jeweiligen weiteren Ultraschallwandler (578) zu generieren, wobei sich der jeweilige weitere Abschnitt des Nebels mit dem ersten Abschnitt des Nebels und dem zweiten Abschnitt verbindet, um den Nebel auszubilden, der durch den Nebelauslassanschluss (208) hindurch zu dem Benutzer herausströmt, wenn der Benutzer an dem Mundstück (401) zieht, und optional wobei jede weitere Flüssigkeit mindestens ein weiteres Therapeutikum einschließt.

**Revendications**

1. Générateur de vapeur permettant de générer une vapeur pour inhalation par un utilisateur, le générateur de vapeur comprenant :

   un orifice d'entrée d'air (569) ;
   un embout buccal (401) comportant un orifice de sortie de vapeur (208) ;
   une trajectoire d'écoulement d'air allant de l'orifice d'entrée d'air (569) à l'orifice de sortie de vapeur (208) ;
   une première chambre à liquide (546) contenant un premier liquide comportant une substance thérapeutique ;
   une seconde chambre à liquide (547) contenant un second liquide ;
   un premier transducteur à ultrasons (505) comportant une première surface d'atomisation ;
   un second transducteur à ultrasons (506) comportant une seconde surface d'atomisation ;
   un support de transducteur (514) comportant une surface de support qui est pourvue d'un premier évidement (520) et d'un second évidement (521), dans lequel le premier évidement (520) retient le premier transducteur à ultrasons (505) avec la première surface d'atomisation généralement dans un plan avec la surface de support et le second évidement (521) retient le second transducteur à ultrasons (506) avec la seconde surface d'atomisation généralement dans le plan avec la surface de support ;
   un premier capillaire (509) qui est absorbant, au moins une partie du premier capillaire (509) étant parallèle au plan et s'étendant le long de la surface de support, de la première chambre à liquide (546) au premier transducteur à ultrasons (505), une partie du premier capillaire (509) étant superposée sur la surface de support et une autre partie du premier capillaire (509) étant en contact avec au moins une portion de la première surface d'atomisation, dans lequel le premier capillaire (509) transporte le premier liquide par action capillaire, de la première chambre à liquide (546) à la première surface d'atomisation ; et
   un second capillaire (510) qui est absorbant, au moins une partie du second capillaire (510) étant parallèle au plan et s'étendant le long de la surface de support, de la seconde chambre à liquide au second transducteur à ultrasons, une partie du second capillaire (510) étant superposée sur la surface de support et une autre partie du second capillaire (510) étant en contact avec au moins une portion de la seconde surface d'atomisation, dans lequel le second capillaire (510) transporte le second liquide par action capillaire, de la seconde chambre à liquide (547) à la seconde surface d'atomisation,
   le premier transducteur à ultrasons (505) étant configuré pour générer et transmettre des ondes ultrasonores pour générer une première portion de la vapeur provenant du premier liquide au niveau de l'interface entre le premier capillaire (509) et le premier transducteur à ultrasons (505), et
   le second transducteur à ultrasons (510) étant configuré pour générer et transmettre des ondes ultrasonores pour générer une seconde portion de la vapeur provenant du second liquide au niveau de l'interface entre le second capillaire (510) et le second transducteur à ultrasons (506), dans lequel la première portion de la vapeur et la seconde portion de la vapeur se combinent et s'écoulent à l'extérieur à travers l'orifice de sortie de vapeur (208) vers l'utilisateur lorsque l'utilisateur aspire sur l'embout buccal (401).

2. Générateur de vapeur selon la revendication 1, dans lequel le support de transducteur (514) comprend :

   un canal de premier contact électrique (535) qui reçoit au moins une partie d'un premier contact électrique (518), le premier contact électrique (518) s'étendant d'une première extrémité du support de transducteur (514) jusqu'au premier transducteur (505) ; et
   un canal de deuxième contact électrique (536) qui reçoit au moins une partie d'un deuxième contact électrique (519), le deuxième contact électrique (519) s'étendant de la première extrémité du support de transducteur (514) jusqu'au second transducteur (506).

3. Générateur de vapeur selon la revendication 1 ou la revendication 2, dans lequel le premier évidement (520) du support de transducteur (514) est pourvu d'un premier trou traversant (522) et le second évidement (521) du support de transducteur (514) est pourvu d'un second trou traversant (523), où un côté du premier transducteur à ultrasons (505) peut être mis en contact à travers le premier trou traversant (522) et un côté du second transducteur à ultrasons (506) peut être mis en contact à travers le second trou traversant (523).

4. Générateur de vapeur selon la revendication 3, dans lequel le générateur de vapeur comprend :

   un troisième contact électrique (515), une partie du troisième contact électrique (515) s'étendant à travers le premier trou traversant (522) pour venir en contact avec le côté du premier transducteur à ultrasons (505) ; et
   un quatrième contact électrique (516), une partie du quatrième contact électrique (516) s'étendant à travers le second trou traversant (523) pour venir en contact avec le côté du second transducteur à ultrasons (506).

5. Générateur de vapeur selon l'une quelconque des revendications précédentes, dans lequel le générateur de vapeur comprend :

une pluralité de portions d'assemblage qui sont accouplées les unes aux autres pour former un orifice d'entrée d'air (569) et une trajectoire d'écoulement d'air allant de l'orifice d'entrée d'air (569) à l'orifice de sortie de vapeur (208), une portion d'assemblage de la pluralité de portions d'assemblage comprenant un canal de sortie de vapeur (560) et au moins une partie d'un conduit de sortie de vapeur.

6. Générateur de vapeur selon la revendication 5, dans lequel la pluralité de portions d'assemblage comportent une première portion d'assemblage (511), la première portion d'assemblage (511) comportant une première surface (563), une seconde surface (564), et deux canaux d'entrée d'air (557, 558) qui s'étendent le long de la première surface (563) et jusqu'à la seconde surface (564).

7. Générateur de vapeur selon la revendication 6, dans lequel la première portion d'assemblage (511) comporte le canal de sortie de vapeur (560) et l'au moins une partie du conduit de sortie de vapeur.

8. Générateur de vapeur selon la revendication 6 ou la revendication 7, dans lequel la pluralité de portions d'assemblage comportent une deuxième portion d'assemblage (512), la deuxième portion d'assemblage (512) comportant :

une section supérieure (566) ;
une section inférieure (565) espacée de la section supérieure (566) ; et
une section médiane (567) s'étendant de la section supérieure (566) à la section inférieure (565),
la section supérieure (566) comportant deux évidements de chambre de sonication (541, 542), deux entrées d'air de chambre de sonication (552, 553), deux sorties de vapeur de chambre de sonication (554, 555) ; et
la section inférieure (565) comportant deux canaux (550, 551), de telle sorte que lorsque la première portion d'assemblage (511) est accouplée à la deuxième portion d'assemblage (512) :

les deux canaux d'entrée d'air (557, 558) de la première portion d'assemblage (511) et les deux canaux (550, 551) de la deuxième portion d'assemblage (512) forment une trajectoire d'écoulement d'air allant de l'orifice d'entrée (569) aux entrées d'air de chambre de sonication (552, 553) ; et
une trajectoire d'écoulement de vapeur partant des sorties de vapeur de chambre de sonication (554, 555) à travers le conduit de sortie de vapeur.

9. Générateur de vapeur selon la revendication 6 ou la revendication 7, dans lequel la pluralité de portions d'assemblage comportent une deuxième portion d'assemblage (512), la deuxième portion d'assemblage comportant :

une section supérieure (566) ;
une section inférieure (565) espacée de la section supérieure (566) ; et
une section médiane (567) s'étendant de la section supérieure (566) à la section inférieure (565),
la section supérieure (566) comportant deux évidements de chambre de sonication (541, 542), deux entrées d'air de chambre de sonication (552, 553), deux sorties de vapeur de chambre de sonication (554, 555), et une rainure (556) ; et
la section inférieure (565) comportant deux canaux (550, 551),
dans lequel les deux canaux d'entrée d'air (557, 558) de la première portion d'assemblage (511) et les deux canaux (550, 551) de la deuxième portion d'assemblage (512) forment une trajectoire d'écoulement d'air allant de l'orifice d'entrée (569) aux entrées d'air de chambre de sonication (552, 553) ; et
l'au moins une partie du conduit de sortie de vapeur de la première portion d'assemblage (511) et la rainure (556) de la section supérieure (566) de la deuxième portion d'assemblage (512) forment une trajectoire d'écoulement de vapeur partant des sorties de vapeur de chambre de sonication (554, 555) à travers le conduit de sortie de vapeur.

10. Générateur de vapeur selon la revendication 8 ou la revendication 9, dans lequel la section supérieure (566) comporte au moins un élément de sollicitation (466) au sein de chaque évidement (541, 542) permettant de solliciter le capillaire (509, 510) respectif contre la portion de la surface d'atomisation respective.

11. Générateur de vapeur selon l'une quelconque des revendications 8 à 10, dans lequel la section médiane (567) de la deuxième portion d'assemblage (512) comporte deux extrusions (582, 583).

**12.** Générateur de vapeur selon l'une quelconque des revendications 8 à 11, dans lequel la pluralité de portions d'assemblage comporte une troisième portion d'assemblage (513), la troisième portion d'assemblage (513) comportant deux trous traversants (539, 540), la première chambre à liquide (546), et la seconde chambre à liquide (547).

**13.** Générateur de vapeur selon l'une quelconque des revendications précédentes, comportant en outre deux connexions électriques généralement annulaires (507, 508), chacune des deux connexions électriques généralement annulaires (507, 508) étant couplée à l'un respectif parmi le premier transducteur à ultrasons (505) et le second transducteur à ultrasons (506).

**14.** Générateur de vapeur selon l'une quelconque des revendications 8 à 12, comportant en outre une quatrième portion d'assemblage (517) qui est au moins partiellement superposée sur une portion du support de transducteur (514).

**15.** Générateur de vapeur selon la revendication 12, dans lequel la troisième portion d'assemblage (513) comporte deux dépressions (537, 538), chaque dépression (537, 538) chevauchant un respectif des deux trous traversants (539, 540) dans la troisième portion d'assemblage (513), et facultativement

dans lequel le second liquide comporte au moins une substance thérapeutique supplémentaire, et facultativement dans lequel le générateur de vapeur comprend :

au moins une chambre à liquide supplémentaire contenant chacune un liquide supplémentaire respectif ;

au moins un transducteur à ultrasons supplémentaire (578) comportant chacun une surface d'atomisation supplémentaire respective ;

au moins un capillaire supplémentaire (510'), au moins une partie du capillaire supplémentaire (510') étant généralement parallèle au plan et s'étendant le long de la surface de support, de la chambre à liquide supplémentaire respective au transducteur à ultrasons supplémentaire (578) respectif, une partie du capillaire supplémentaire (510') respectif étant superposée sur la surface de support et une autre partie du capillaire supplémentaire (510') respectif étant en contact avec au moins une portion de la surface d'atomisation supplémentaire respective, dans lequel le capillaire supplémentaire (510') respectif transporte le liquide supplémentaire respectif par action capillaire, de la chambre à liquide supplémentaire respective à la surface d'atomisation supplémentaire respective,

chaque transducteur à ultrasons supplémentaire (578) étant configuré pour générer et transmettre des ondes ultrasonores pour générer une portion supplémentaire respective de la vapeur provenant du liquide supplémentaire respectif au niveau de l'interface entre le capillaire supplémentaire (510') respectif et le transducteur à ultrasons supplémentaire (578) respectif, dans lequel la portion supplémentaire respective de la vapeur se combine avec la première portion de la vapeur et la seconde portion pour former la vapeur qui s'écoule à l'extérieur à travers l'orifice de sortie de vapeur (208) vers l'utilisateur lorsque l'utilisateur aspire sur l'embout buccal (401), et facultativement dans lequel chaque liquide supplémentaire comporte au moins une substance thérapeutique supplémentaire.

200

FIG. 1

FIG. 2

*FIG. 3*

*FIG. 4*

FIG. 5

EP 4 608 482 B1

214  212  210
220
221
213
211

**FIG. 6**

215
210

**FIG. 7**

*FIG. 8*

*FIG. 9*

*FIG. 10*

*FIG. 11*

FIG. 12

FIG. 13

206

234

208

## FIG. 14

205

FIG. 15

236

FIG. 16

FIG. 17

FIG. 18

FIG. 19

*241* *241* *239*

*240*

## FIG. 20

*243*

*240*

*242*

## FIG. 21

*239*

FIG. 22

FIG. 23

FIG. 24

*FIG. 25*

*FIG. 26*

FIG. 27

208

406

401

400

402

204

FIG. 28

204

241

241

240

FIG. 29

EP 4 608 482 B1

FIG. 30

FIG. 31

**FIG. 32**

FIG. 33

FIG. 34

FIG. 35

FIG. 36

FIG. 37

FIG. 38

*425*

*FIG. 39*

*470* *471*

*222*

*204*

*425*

*FIG. 40*

*204*

FIG. 41

FIG. 42

FIG. 43

**FIG. 44**

**FIG. 45**

FIG. 46

479 478 477

480

472

FIG. 47

204

479 478 477

FIG. 48

204

208

401

481

*FIG. 49*

477

204

208

401

*FIG. 50*

204

FIG. 51

FIG. 52

FIG. 53

FIG. 54

FIG. 55

*FIG. 56*

FIG. 57

FIG. 58

FIG. 59

500

401

402

FIG. 60

204

485

500

569

240

241

570

FIG. 61

FIG. 62

FIG. 63

FIG. 64

FIG. 65

FIG. 66

FIG. 67

FIG. 68

FIG. 70

FIG. 69

FIG. 71

FIG. 72

FIG. 73

FIG. 74

FIG. 75

FIG. 76

252

FIG. 77

252

257

255

256

252

254

253

251

FIG. 78

259

FIG. 79

259

260

264

263

FIG. 80

**FIG. 81**

EP 4 608 482 B1

FIG. 82

FIG. 83

FIG. 84

FIG. 85

Oscillator _315_

Clk_m

Frequency divider _330_

/2    /4    /8    /16

Divided Frequency

Multiplexer _331_

Phase 1

Phase 2

Delay Locked Loop _332_

Phase A

Phase B

_329_

EP 4 608 482 B1

clk_m

Phase 1

Phase 2

*FIG. 86*

clk_m

ph A

ph B

R    F    R

*FIG. 87*

# FIG. 88

| Port # | Primary Function | Secondary Function |
|--------|------------------|--------------------|
| GPIO.0 | digital GPIO | used for chip test |
| GPIO.1 | digital GPIO | ADC channel 0 |
| GPIO.2 | digital GPIO | ADC channel 1 |
| GPIO.3 | rms current | digital GPIO |
| GPIO.4 | digital GPIO | used for chip test |
| GPIO.5 | digital GPIO | I2C address select ADR0 |
| GPIO.6 | digital GPIO | I2C address select ADR1 |
| GPIO.7 | digital GPIO | I2C address select ADR2 |

FIG. 89

EP 4 608 482 B1

*FIG. 90*

FIG. 91

EP 4 608 482 B1

External shunts for
current sensing →

A

H Bridge
*334*

C

B

VOUT

D

Piezo
Model

*215*

## FIG. 92

A ON
B OFF
C OFF
D ON

A OFF
B OFF
C OFF
D ON

A OFF
B OFF
C ON
D ON

A OFF
B OFF
C ON
D OFF

A OFF
B ON
C ON
D OFF

A OFF
B OFF
C ON
D OFF

VOUT+

VOUT-

## FIG. 93

FIG. 94

FIG. 95

*FIG. 96*

EP 4 608 482 B1

FIG. 97

**FIG. 98**

FIG. 99

FIG. 100

FIG. 101

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020227717 A